# EUROPÄISCHE PATENTSCHRIFT

(11) **EP 2 436 365 B1**
(45) Veröffentlichungstag und Bekanntmachung des Hinweises auf die Patenterteilung: **08.03.2017**
(21) Anmeldenummer: 11183328.1
(22) Anmeldetag: 29.09.2011
(51) Int. Cl.: A61K 6/027, A61L 27/44, A61K 6/083, A61K 6/00

(54) **Kompositmaterial umfassend ein Monomer mit einem polyalicyclischen Strukturelement**
Composite material comprising a monomer with a polyalicyclic structure element
Matériau composite comprenant un monomère doté d'un élément structurel polyalicyclique

(30) Priorität: 30.09.2010 DE 102010041783
(43) Veröffentlichungstag der Anmeldung: 04.04.2012
(73) Patentinhaber: VOCO GmbH, 27472 Cuxhaven (DE)
(72) Erfinder: Blömker, Dr. Tobias, 22527 Hamburg (DE); Stepputtis, Dr. Manfred, 27449 Kutenholz (DE); Maletz, Dr. Reinhard, 27472 Cuxhaven (DE); Plaumann, Manfred Thomas, 27472 Cuxhaven (DE)
(74) Vertreter: Eisenführ Speiser

(56) Entgegenhaltungen:
- EP-A2- 2 016 931
- EP-B1- 0 969 789
- DE-A1- 19 617 931

## Beschreibung

Die vorliegende Erfindung betrifft ein Kompositmaterial, insbesondere ein dentales (und vorzugsweise lichthärtbares) Kompositmaterial, sowie die Verwendung eines erfindungsgemäßen Kompositmaterials als Dentalmaterial und ein Verfahren zur Herstellung eines erfindungsgemäßen Kompositmaterials. Die Erfindung betrifft außerdem neue radikalisch polymerisierbare Monomere umfassend mindestens ein polyalicyclisches Strukturelement und bestimmte ethylenische Strukturelemente, die für den Einsatz in einem erfindungsgemäßen Kompositmaterial, insbesondere einem erfindungsgemäßen dentalen Kompositmaterial, besonders geeignet sind, und deren Verwendung in einem Kompositmaterial.

Innerhalb der letzten 50 Jahre wurde Amalgam, das über 100 Jahre die zahnärztliche Füllungstherapie prägte, in zunehmendem Maße durch kunststoffbasierende Kompositmaterialien abgelöst. Gründe für diesen Wandel waren zum einen der Wunsch der Patienten nach einer ästhetischen Zahnversorgung auch im Seitenzahnbereich und zum anderen Fortschritte in der Entwicklung zahnärztlicher Restaurationskunststoffe. Insbesondere wurden mit 2,2-Bis[4-(2-hydroxy-3-methacryloyl-oxypropoxy)phenyl)propan (Bis-GMA) als Grundsubstanz Füllungskunststoffe entwickelt.

Bis-GMA, synthetisiert aus 2 Mol Glycidylmethacrylat und einem Mol Bisphenol A, enthält an seinen beiden Enden je eine terminale Vinylgruppe, die in einer radikalischen Reaktion schnell polymerisiert und ein vernetztes Polymerisat ergibt. Die chemische Struktur von Bis-GMA weist einige Besonderheiten auf:
- Die beiden aromatischen Ringe des Bisphenol A-Strukturelements, die an dem Propanrest anbinden, sind in ihrer Rotation sterisch gehindert. Quantenchemische Rechnungen sowie experimentelle Untersuchungen zeigen, dass die Wasserstoffatome an den Positionen 2 und 6 jedes aromatischen Rings mit den Wasserstoffatomen der beiden Methylgruppen des Propanrests überlappen, so dass selbst bei höheren Temperaturen keine Rotation der aromatischen Ringe möglich ist. Das Molekül besitzt somit eine weitestgehend starre Konformation, die einen vergleichsweise hohen Elastizitätsmodul des Harzes bewirkt.
- Das Molekül verfügt sowohl über freie Hydroxylgruppen als auch über Carbonylsauerstoffatome. Die Gegenwart dieser Strukturelemente ermöglicht Wechselwirkungen und den Aufbau von Wasserstoffbrückenbindungen zwischen benachbarten Bis-GMA Molekülen.

Diese Strukturmerkmale des Bis-GMA ermöglichen den Aufbau einer sogenannten Überstruktur basierend auf Wasserstoffbrückenbindungen im Polymerisat, wobei die Überstrukturen aufgrund der starren Molekülkonformation extrem dicht gepackt sind. Dies führt zu besonders guten mechanischen Eigenschaften des Werkstoffs. Zusätzlich sorgen die Überstrukturen und die außergewöhnlich dichte Packung der Molekülketten dafür, dass die Aufnahme von Wasser aus der Mundhöhle in den dentalen Werkstoff hinein erheblich erschwert wird. Absorption von Wasser führt zu einem Erweichen des Werkstoffes und kann einen hydrolytischen Abbau an den Esterfunktionen des Polymerisats einleiten.

Aufgrund seiner Größe und ausgesprochen starren Konformation ist es außerdem unwahrscheinlich, dass nicht umgesetztes Bis-GMA, sollte es aus dem vernetzten Polymerisat entweichen, über die dentinalen Tubuli zur Pulpa gelangt. Es ist ebenfalls nicht zu erwarten, dass Bis-GMA leicht biologische Membranen passieren und bioakkumulieren könnte. Aus diesen Gründen wird dem Bis-GMA eine geringe Toxizität und eine im Allgemeinen gute Bioverträglichkeit zugeschrieben. Jedoch sind die toxikologischen Eigenschaften von Bisphenol A, dem Ausgangsprodukt der Synthese des Bis-GMA, das in Spuren stets in Bis-GMA enthalten ist, noch nicht abschließend geklärt.

Die Moleküle des Bis-GMA sind auf Grund ihrer Größe sehr raumerfüllend. Bei der Vernetzung ist daher die Polymerisationsschrumpfung typischerweise gering, denn bei der radikalischen Polymerisation gleicher Substanzmengen Bis-GMA und beispielsweise Methylacrylat werden weniger Bindungen gebildet als bei Methylmethacrylat, und somit wird weniger Bindungsenergie frei.

Die Polymerisationsschrumpfung entspricht phänomenologisch einer während und nach der Vernetzung auftretenden Änderung der Dichte. Dies hat im Wesentlichen zwei Ursachen. Einerseits kommt es im Verlauf der Polymerisation zu einer Annäherung der Monomerbausteine von einem Van-der-Waals-Abstand zum Abstand einer kovalenten Bindung, und andererseits ist die Packungsdichte der Polymerketten höher als die Packungsdichte der Monomeren. Der Schrumpf (die Volumenschwindung) der Reaktionsharzmasse hängt in erster Linie von der Anzahl der abreagierten funktionellen Gruppen ab. Der Schrumpf tritt sowohl im flüssigen Zustand, also ganz zu Beginn der Polymerisation, als auch während und nach der Gelierung auf. Der Gesamtschrumpf umfasst einen physikalischen und einen chemischen Anteil. Während der physikalische Schrumpf richtungsbestimmt ist und räumlich von den Außenbereichen des Polymerisats mit dem Temperaturgefälle nach innen zum Mittelpunkt des aushärtenden Formstoffs verläuft, ist der chemische Anteil nicht richtungsbestimmt und resultiert einzig aus der Polymerbildung. Zu Beginn der Polymerisation kann die Volumenschwindung durch Nachfließen des Materials noch kompensiert werden. Doch in kurzer Zeit ist das Polymernetzwerk so weit aufgebaut, dass der Gelpunkt erreicht ist, die Mobilität der Monomeren eingeschränkt ist und ein Nachfließen des Materials unmöglich wird. In diesem Zustand entstehen nachteiligerweise innere Spannungen im Material, die bei seiner Anwendung als dentaler Füllungswerkstoff entweder zum Ablösen von den Kavitätenwänden und so zur Randspaltbildung führen, oder sie schwächen das Material durch die Bildung von Volumendefekten.

Um dem Schrumpf entgegenzuwirken, werden - neben der Verwendung voluminöser Monomere wie dem vorstehend diskutierten Bis-GMA - der dentalen Zusammensetzung oft volumenstabile Füllstoffe zugegeben. Dadurch wird erreicht, dass die Anzahl vernetzbarer Gruppen herabgesetzt und somit der Schrumpf sowie die bei der Bindungsbildung freiwerdende Wärmeenergie reduziert wird. Sowohl die Verwendung besonders raumerfüllender Monomerer als auch die Zugabe von Füllstoffen führt aber nachteiligerweise zu einer Erhöhung der Viskosität und damit zu einer verminderten Verarbeitbarkeit. Daher enthalten herkömmliche dentale Kompositmaterialien sogenannte niedermolekulare Reaktivverdünner, die die Viskosität herabsetzen und so die Verarbeitbarkeit des Kompositmaterials sicherstellen. Konventionelle dentale Kompositmaterialien bestehen somit aus einem voluminösen Monomer wie insbesondere Bis-GMA und niedermolekularen Monomeren (Reaktivverdünnern) wie beispielsweise Triethylenglykoldimethacrylat (TEDMA) sowie üblichen Füllstoffen, Polymerisationsinitiatoren sowie Additiven.

Durch niedermolekulare Monomere (Reaktivverdünner) wie z.B. Triethylenglykoldimethacrylat werden bestimmte mechanische Eigenschaften des Materials verbessert, da diese Moleküle selbst nach Erreichen des Gelpunkts noch so beweglich sind, dass sie Reaktionspartner finden und durch die gebildeten Bindungen die Netzwerkdichte des Polymerisats erhöhen. TEDMA und andere niedermolekulare Monomere ähnlicher Struktur sind in der Regel aber auch hochflexibel, da diese Moleküle um ihre Etherbindungen herum ungehindert rotieren können. Durch ihren flexiblen Charakter stören die niedermolekularen Monomeren die Homogenität der starren und eng gepackten Bis-GMA Strukturen, so dass neben teilweise verbesserten mechanischen Eigenschaften andere mechanische Eigenschaften treten, die beeinträchtigt sind. Daher ist es bisher nicht gelungen, Bis-GMA-Kompositmaterialien mit minimaler Polymerisationsschrumpfung und insgesamt guten weiteren mechanischen Eigenschaften, insbesondere einer guten Abrasionsbeständigkeit und Oberflächenhärte des ausgehärteten Kompositmaterials anzugeben.

Es wurden in der Vergangenheit Monomersysteme vorgeschlagen, bei denen über Ringöffnungsreaktionen die Volumenkontraktion ausgeglichen werden sollte. Andere Entwicklungen nutzen eine kationische Ringöffnungspolymerisation anstelle einer radikalischen Additionspolymerisation. Außerdem wurden flüssigkristalline Monomere, dendritische Monomere oder organisch-anorganische Hybridmaterialien, wie die Ormoceren (Organically modified ceramics) getestet. Konzeptionell wird so versucht, die Volumenschwindung durch eine Verschiebung der Bilanz zwischen aufgebrochenen und neu geknüpften kovalenten Bindungen oder durch Unterschiede in der Packungsdichte zwischen flüssiger und fester Phase zu überwinden.

Ebenfalls bekannt ist bereits der Einsatz radikalisch polymerisierbarer Methacrylsäure- oder Acrylsäureester mit einem Tricyclo[5.2.1.0^{2,6}]-decan (TCD) - Strukturelement zur Herstellung schrumpfungsarmer Dentalwerkstoffe. Aufgrund der starren Dreiringkonformation bei völliger sterischer Einschränkung der Beweglichkeit weist diese Substanzgruppe Ähnlichkeit zu Bis-GMA auf. Zudem bewirkt das zentrale aliphatische Kohlenwasserstoffelement die Ausbildung einer beachtlichen Hydrophobie, die eine hohe Wasserfestigkeit des Polymerisats zur Folge haben sollte. Radikalisch polymerisierbare Methacrylsäure- oder Acrylsäureester mit einem Tricyclo[5.2.1.0^{2,6}]-decan-Strukturelement weisen eine sehr niedrige Viskosität auf und sind somit leicht verarbeitbar, und ihr Brechungsindex passt überdies zu der bei dentalen Materialien üblicherweise verwendeten Glaskeramik.

Dentale Kompositmaterialien enthaltend radikalisch polymerisierbare Methacrylsäure- oder Acrylsäureester mit einem Tricyclo[5.2.1.0^{2,6}]-decan-Strukturelement werden u.a. in den folgenden Druckschriften genannt: DE 28 16 823 A1, DE 24 19 887 A1, DE 24 06 557 A1, DE 29 31 926 A1, DE 35 22 005 A1, DE 35 22 006 A1, DE 37 03 080 A1, DE 37 03 130 A1, DE 37 07 908 A1, DE 38 19 777 A1, DE 197 01 599 A1, DE 699 35 794 T2.

Die Dokumente DE 22 00 021 A1, EP 0 023 686 A2, EP 0 049 631 A1, JP 7-206740 A, JP 7-206741 A, JP 11-21370 A offenbaren ebenfalls radikalisch polymerisierbare Methacrylsäure- oder Acrylsäureester mit einem Tricyclo[5.2.1.0^{2,6}]-decan-Strukturelement.

Die DE 10 2005 021 332 B4 beschreibt Kompositmaterialien, die eine geringe Schrumpfkraft aufweisen sollen. Das offenbarte Kompositmaterial mit einem Gesamtfüllstoffgehalt von 80 bis 95 Gew.-% enthält
- in der Füllstoffkomponente
   - 0,5 bis 10 Gew.-% nicht-agglomerierte Nanofüller mit Partikelgrößen von 1 nm bis 50 nm,
      und
   - mindestens 60 Gew.-% eines Füllstoffgemischs aus 50 bis 90 Gew.-% grob- und 10 bis 50 Gew.-% feinteiligen Dentalgläsern, welche ein Größenverhältnis, bezogen auf die mittlere Partikelgröße von feinteilig zu grobteilig von 1:4 bis 1:30 aufweisen, wobei der Anteil an feinteiligen Dentalgläsern maximal 40 Gew.-% an der Füllstoffmischung betragen soll,
- sowie als Monomerkomponente eine Mischung aus
   - 60-80 Gew.-% Bis-GMA oder TCD-di-HEMA (Bis(methacryloyloxymethyl-)-tricyclo[5.2.1.0^{2,6}]decan) oder TCD-di-HEA (Bis(acryloyloxymethyl)tricyclo [5.2.1.0^{2,6}]decan)
   - 10-18 Gew.-% UDMA (Urethandimethacrylat)
   - Rest TEDMA und/oder multifunktionelle Vernetzer
- bis 1 Gew.-% Initiator(en).

Durch den Einsatz nicht agglomerierter Nanofüllstoffe und eines Füllstoffgemisches aus grob- und feinteiligen Dentalgläsern, durch weitgehende Substitution von TEDMA durch UDMA und die optionale Verwendung von radikalisch polymerisierbare Methacrylsäure- oder Acrylsäureester mit einem Tricyclo[5.2.1.0^{2,6}]-decan-Strukturelement sowie die optionale Reduktion der Initiatormenge soll gemäß DE 10 2005 021 332 B4 eine Verringerung der Polymerisationsschrumpfung erreicht werden. Belegt wurde dies allerdings nur für TCD-freie Zusammensetzungen.

In der DE 10 2005 053 775 A1 wird ein selbsthärtendes oder dualhärtendes, dünnfließendes Kompositmaterial zur Herstellung eines dentalen Liners mit Polymerisation in zwei Stufen mit zwei Abbindezeiten und verzögerter Polymerisationscharakteristik, der zum Einsatz im Bereich der Kavitätenwand in einer dünnen Schicht vorgesehen ist, offenbart. Bevorzugte Monomerkomponenten sind u.a. TCD-di-HEMA und TCD-di-HEA.

Die EP 1 935 393 A2 und DE 10 2006 060 983 A1 betreffen Dentalkomposite umfassend radikalisch polymerisierbare Acrylsäureester mit einem Tricyclo[5.2.1.0^{2,6}]-decan-Strukturelement. Deren höherer Polymerisationsgrad soll vorteilhaft sein für die mechanischen Eigenschaften der Komposite. Allerdings gelten Acrylatmonomere für dentale Anwendungen als ungeeignet aufgrund ihrer nachteiligen toxikologischen Eigenschaften. Nach der Aushärtung sollen die vernetzten TCD-Acrylatmonomere dennoch eine sehr günstige biologische Verträglichkeit aufweisen. In diesem Dokument werden auch Zusammensetzungen beschrieben, die im Wesentlichen frei von Bis-GMA sind.

Auch in der EP 2 016 931 A2 und DE 10 2007 034 457 A1 werden Dentalkomposite umfassend radikalisch polymerisierbare Methacrylsäure- oder Acrylsäureester mit einem Tricyclo[5.2.1.0^{2,6}]-decan-Strukturelement beschrieben, wobei die Monomerkomponente sowohl Bis-GMA als auch TCD-di-HEMA oder TCD-di-HEA enthalten muss.

WO 03/035013 A1 und DE 602 16 951 T2 betreffen Dentalkleberzusammensetzungen zum Binden von Dental-Restaurierungsmitteln an Dentin und/oder Zahnschmelz. In diesen Dokumenten wird unter anderem die Herstellung von 3,(4),8,(9)-Bis(2-propenamidomethyl)tricyclo[5.2.1.0]^{2,6}-decan beschrieben.

Aus dem Stand der Technik sind somit Zusammensetzungen auf der Basis radikalisch polymerisierbarer Methacrylsäure- oder Acrylsäureester mit einem Tricyclo[5.2.1.0^{2,6}]-decan-Strukturelement bekannt, die zu schrumpfungsarmen dentalen Kompositmaterialien führen. Jedoch sind mit diesen Zusammensetzungen keine Kompositmaterialien erhältlich, die gleichzeitig schrumpfungsarm sind und hohe mechanische Anforderungen erfüllen, insbesondere hinsichtlich der Abrasionsbeständigkeit und Oberflächenhärte der Polymerisate. Es war daher die primäre Aufgabe der vorliegenden Erfindung, ein dentales Kompositmaterial zur Verfügung zu stellen, das sowohl schrumpfungsarm ist als auch eine hohe Abrasionsbeständigkeit und Oberflächenhärte aufweist.

Weitere Aufgaben ergeben sich aus der nachfolgenden Beschreibung und den beigefügten Patentansprüchen.

Die primäre Aufgabe wird gelöst durch ein erfindungsgemäßes Kompositmaterial, insbesondere ein erfindungsgemäßes dentales Kompositmaterial bestehend aus oder umfassend
(a) > 75 (vorzugsweise 80) bis 95 Gew.-%, bezogen auf die Gesamtmasse des Kompositmaterials, einer Mischung von Füllstoffen umfassend
   (a1) > 12) bis 30 Gew.-% nicht agglomerierte, organisch oberflächenmodifizierte Nanopartikel mit einer mittleren Partikelgröße kleiner 200 nm (vorzugsweise kleiner 100 nm, besonders bevorzugt kleiner 60 nm) und
   (a2) 45 bis < 83 Gew.-%) Mikropartikel mit einer mittleren Partikelgröße von 0,4 µm bis 10 µm sowie
   (a3) gegebenenfalls weitere Füllstoffe,
   wobei die Gewichtsprozentangaben für die Komponenten (a1) und (a2) auf die Gesamtmasse des Kompositmaterials bezogen sind,
(b) 3 bis < 25 (vorzugsweise bis 20) Gew.-%, bezogen auf die Gesamtmasse des Kompositmaterials, einer Monomerenmischung umfassend
   (b1) ein, zwei oder mehr Monomere ausgewählt aus der Gruppe bestehend aus Verbindungen (Monomere) der Struktur Q(YZₑ)_{b}, wobei gilt:
      - Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten YZₑ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind;
      - b ist eine natürliche Zahl ausgewählt aus der Gruppe der natürlichen Zahlen 1, 2, 3 und 4;
      - jedes Z bedeutet ein Strukturelement, das unabhängig von etwaigen weiteren Strukturelementen Z ausgewählt ist aus der Gruppe bestehend aus
         -O-(C=O)-CH=CH₂, -O-(C=O)-C(CH₃)=CH₂,
         -(C=O)-CH=CH₂, -(C=O)-C(CH₃)₌CH₂,
         -CH=CH₂, -C(CH₃)=CH₂ und -O-CH=CH₂,
      - jeder Index e ist eine natürliche Zahl, die unabhängig von etwaigen weiteren Indizes e ausgewählt ist aus der Gruppe der natürlichen Zahlen 1, 2, 3 und 4;
      - jedes Y bedeutet ein Strukturelement, welches in der Struktur Q(YZₑ)_{b} das polyalicyclische Strukturelement Q mit e Strukturelementen Z verbindet, wobei jedes Y unabhängig von etwaigen weiteren Strukturelementen Y gewählt ist oder entfällt,
   (b2) ein, zwei oder mehr weitere radikalisch polymerisierbare Monomere aus der Gruppe bestehend aus Acrylaten und Methacrylaten, vorzugsweise der Gruppe der Methacrylate,
   wobei das Verhältnis der Masse der Komponente (b1) zur Masse der Komponente (b2) im Bereich von 1 : 20 bis 4 : 1 (vorzugsweise 1 : 3 bis 3 : 1) liegt,
(c) ein oder mehrere Initiatoren und/oder Katalysatoren, sowie
(d) gegebenenfalls ein oder mehrere Additive.

Mit anderen Worten betrifft die vorliegende Erfindung ein Kompositmaterial, insbesondere ein dentales Kompositmaterial bestehend aus oder umfassend:
(a) eine Gesamtmenge an Füllstoffen im Bereich von > 75 bis 95 Gew.-%, bezogen auf die Gesamtmasse des Kompositmaterials, wobei die Gesamtmenge an Füllstoffen eine Mischung von Füllstoffen ist umfassend
   (a1) eine Gesamtmenge im Bereich von > 12 bis 30 Gew.-% an nicht agglomerierten, organisch oberflächenmodifizierten Nanopartikeln mit einer mittleren Partikelgröße kleiner 200 nm
      und
   (a2) eine Gesamtmenge im Bereich von 45 bis < 83 Gew.-% an Mikropartikeln mit einer mittleren Partikelgröße im Bereich von 0,4 µm bis 10 µm sowie
   (a3) gegebenenfalls weitere Füllstoffe,
   wobei die Gewichtsprozentangaben für die Komponenten (a1) und (a2) auf die Gesamtmasse des Kompositmaterials bezogen sind,
(b) eine Gesamtmenge an polymerisierbaren Monomeren im Bereich von 3 bis < 25 Gew.-%, bezogen auf die Gesamtmasse des Kompositmaterials, wobei die Gesamtmenge an polymerisierbaren Monomeren eine Monomerenmischung ist umfassend
   (b1) ein, zwei oder mehr Monomere ausgewählt aus der Gruppe bestehend aus Verbindungen (Monomere) der Struktur Q(YₓZₑ)_{b}, wobei gilt:
      - Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten YₓZₑ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,
      - b ist eine natürliche Zahl ausgewählt aus der Gruppe der natürlichen Zahlen 1, 2, 3 und 4,
      - jedes Z bedeutet ein Strukturelement, das unabhängig von etwaigen weiteren Strukturelementen Z ausgewählt ist aus der Gruppe bestehend aus
         -O-(C=O)-CH=CH₂, -O-(C=O)-C(CH₃)=CH₂,
         -(C=O)-CH=CH₂, -(C=O)-C(CH₃)₌CH₂,
         -CH=CH₂, -C(CH₃)=CH₂ und -O-CH=CH₂,
      - jeder Index e ist eine natürliche Zahl, die unabhängig von etwaigen weiteren Indizes e ausgewählt ist aus der Gruppe der natürlichen Zahlen 1, 2, 3 und 4,
      - jeder Index x bedeutet unabhängig von etwaigen weiteren Indizes x 0 oder 1,
      - jedes Y bedeutet in der Struktur Q(YₓZₑ)_{b} bei x = 1 ein Strukturelement, welches das polyalicyclische Strukturelement Q mit e Strukturelementen Z verbindet, wobei jedes Y unabhängig von etwaigen weiteren Strukturelementen Y gewählt ist,
   (b2) ein, zwei oder mehr weitere radikalisch polymerisierbare Monomere aus der Gruppe bestehend aus Acrylaten und Methacrylaten, vorzugsweise der Gruppe der Methacrylate,
   wobei das oder die weiteren radikalisch polymerisierbaren Monomere keine Verbindungen (Monomere) der vorstehend definierten Struktur Q(YₓZₑ)_{b} sind und wobei das Verhältnis der Masse der Komponente (b1) zur Masse der Komponente (b2) im Bereich von 1 : 20 bis 4 : 1 liegt,
(c) ein oder mehrere Initiatoren und/oder Katalysatoren, sowie
(d) gegebenenfalls ein oder mehrere sonstige Additive.

Sämtliche nachfolgenden Ausführungen betreffend die Verbindungen der Struktur Q(YZₑ)_{b} (wobei jedes Y unabhängig von etwaigen weiteren Strukturelementen Y gewählt ist oder entfällt) und die im Zusammenhang mit diesen Verbindungen angegebenen bevorzugten bzw. besonders bevorzugten Ausgestaltungen der vorliegenden Erfindung gelten entsprechend für die Verbindungen der Struktur Q(YₓZₑ)_{b} (wobei jeder Index x unabhängig von etwaigen weiteren Indizes x 0 oder 1 bedeutet), und umgekehrt.

Eine erfindungsgemäße bzw. erfindungsgemäß einzusetzende Verbindung der Struktur Q(YₓZₑ)_{b} umfasst ein polyalicyclisches Strukturelement Q, das von einem entsprechenden polyalicyclischen Kohlenwasserstoff abgeleitet ist. Dies bedeutet im Rahmen des vorliegenden Textes, dass b Wasserstoffatome des Kohlenwasserstoffs durch Substituenten YₓZₑ ersetzt sind (wie oben beschrieben), und optional ein, zwei oder mehr der nicht durch Substituenten YₓZₑ substituierten Wasserstoffatome durch Alkylgruppen, Alkoxygruppen, Halogenatome oder Trifluormethylgruppen substituiert sind. Das polyalicyclische Strukturelement Q wird konstituiert durch Kohlenstoff-Ring-Atome. Kohlenstoffatome außerhalb der Ringe sind Bestandteil von Substituenten.

Bei dem "polyalicyclischen" Strukturelement Q handelt es sich um einen bicyclischen, tricyclischen, tetracyclischen, pentacyclischen oder hexacyclischen Kohlenwasserstoffrest, wie oben definiert. Die Bezeichnungen "bicyclisch", "tricyclisch", tetracyclisch", "pentacyclisch" und "hexacyclisch" entsprechen dabei der IUPAC-Nomenklatur.

Q bedeutet mit anderen Worten ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten YₓZₑ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert ist bzw. sind.

Vorzugsweise bedeutet jedes Y ein Strukturelement, welches in der Struktur Q(YₓZₑ)_{b} mit x = 1 das polyalicyclische Strukturelement Q mit e Strukturelementen Z verbindet, wobei jedes Y unabhängig von etwaigen weiteren Strukturelementen Y gewählt ist.

Ein erfindungsgemäßes Kompositmaterial ist vorzugsweise lichthärtbar.

Die Summe der Zahlenwerte des Index b und des Index e beträgt vorzugsweise 3, 4, 5, 6, 7 oder 8.

Überraschend hat sich in eigenen Untersuchungen gezeigt, dass sich ein erfindungsgemäßes Kompositmaterial, insbesondere ein erfindungsgemäßes dentales Kompositmaterial, welches
- einen relativ hohen Anteil (mehr als 10 Gew.-%) oberflächenmodifizierter nichtagglomerierter Nanopartikel (Komponente (a1)) umfasst
   und
- ein, zwei oder mehr Monomere der Struktur Q(YZₑ)_{b} (wie oben definiert und wobei jedes Y unabhängig von etwaigen weiteren Strukturelementen Y gewählt ist oder entfällt) der Komponente (b1),
sowie
- ein, zwei oder mehrere weitere radikalisch polymerisierbare Monomere aus der Gruppe bestehend aus Acrylaten und Methacrylaten (b2) in einem Massenverhältnis der Komponente (b1) zur Komponente (b2) im Bereich von 1 : 20 bis 4 : 1 umfasst,
im ausgehärteten Zustand nicht nur durch eine geringe Polymerisationsschrumpfung (vorzugsweise weniger als 1,7 Vol-%, besonders bevorzugt weniger als 1,6 Vol.-%, gemessen nach der Bonded-Disc-Methode (Dental Materials 2004, 20, 88-95)), sondern auch durch eine geringe Abrasion (vorzugsweise weniger als 30 µm, bestimmt nach der ACTA-Methode) und eine hohe Vickers-Mikrohärte (vorzugsweise 140 oder mehr) auszeichnet.

Bevorzugt ist ein erfindungsgemäßes Kompositmaterial bestehend aus oder umfassend
(a) 80 bis 95 Gew.-%, bezogen auf die Gesamtmasse des Kompositmaterials, einer Mischung von Füllstoffpartikeln umfassend
   (a1) > 12 bis 30 Gew.-% nicht agglomerierte, organisch oberflächenmodifizierte Nanopartikel mit einer mittleren Partikelgröße kleiner 100 nm (besonders bevorzugt kleiner 60 nm) und
   (a2) 45 bis < 84 Gew.-%, bevorzugt 45 bis < 83 Gew.-%, Mikropartikel mit einer mittleren Partikelgröße von 0,4 µm bis 10 µm
      sowie
   (a3) gegebenenfalls weitere Füllstoffe
   wobei die Gewichtsprozentangaben für die Komponenten (a1) und (a2) auf die Gesamtmasse des Kompositmaterials bezogen sind,
(b) 3 bis 20 Gew.-%, bezogen auf die Gesamtmasse des Kompositmaterials, einer Monomerenmischung umfassend
   (b1) ein, zwei oder mehr Monomere der Struktur Q(YZₑ)_{b} (wie oben definiert und wobei jedes Y unabhängig von etwaigen weiteren Strukturelementen Y gewählt ist oder entfällt) der Komponente (b1), wobei vorzugsweise Z -O-(C=O)-CH=CH₂, -O-(C=O)-C(CH₃)=CH₂, -(C=O)-CH=CH₂ oder -(C=O)-C(CH₃)=CH₂ bedeutet,
   (b2) ein, zwei oder mehr weitere radikalisch polymerisierbare Monomere aus der Gruppe bestehend aus Acrylaten und Methacrylaten, vorzugsweise der Gruppe der Methacrylate,
   wobei das Verhältnis der Masse der Komponente (b1) zur Masse der Komponente (b2) im Bereich von 1 : 3 bis 3 : 1 liegt
(c) ein oder mehrere Initiatoren und/oder Katalysatoren, sowie
(d) gegebenenfalls ein oder mehrere Additive.

Besonders bevorzugt ist ein erfindungsgemäßes Kompositmaterial mit einem Verhältnis der Vickers-Mikrohärte zum Produkt aus der Mikroabrasion (gemessen mit der ACTA-Methode) und der Polymerisationsschrumpfung von größer als 2, vorzugsweise größer als 2,5 [100/µm].

Erfindungsgemäße dentale Kompositmaterialien sind vorzugsweise so ausgestaltet, dass sie als restauratives Kompositmaterial eingesetzt werden können, insbesondere als Füllungs-, Unterfüllungs-, Befestigungs- und/oder Stumpfaufbaumaterial, provisorisches Kronen- und/oder Brückenmaterial, als Prothesen- und/oder Unterfütterungsmaterial sowie als Flow-Material. Entsprechende Verwendungen eines erfindungsgemäßen Kompositmaterials sind bevorzugt.

Das erfindungsgemäße Kompositmaterial umfasst oder besteht aus verschiedenen Bestandteilen, für die das Folgende gilt:

### Bestandteil (a): Füllstoffpartikel

Ein erfindungsgemäßes Kompositmaterial enthält einen Anteil an Füllstoffpartikeln von mehr als 75 Gew.-% (d.h. > 75 Gew.-%), vorzugsweise 80 Gew.-% bis 95 Gew.-%, bezogen auf die Gesamtmasse des erfindungsgemäßen Kompositmaterials. Der Füllstoffanteil umfasst eine Mischung eines ersten Füllstoffs (a1) in Form nicht agglomerierter, organisch oberflächenmodifizierter Nanopartikel mit einer mittleren Partikelgröße kleiner 200 nm und eines zweiten Füllstoffs (a2) in Form von Mikropartikeln mit einer mittleren Partikelgröße im Bereich von 0,4 µm bis 10 µm. Durch die Kombination von (a1) Nanopartikeln und (a2) Mikropartikeln im erfindungsgemäßen Kompositmaterial wird eine vollständige und gleichmäßige Volumenfüllung des Kompositmaterials erzielt. Dadurch wird sowohl die Schrumpfung des Kompositmaterials beim Aushärten der Polymermatrix als auch die Empfindlichkeit des Kompositmaterials gegen Abrasion vermindert.

Die mittlere Partikelgröße d₅₀ der erfindungsgemäß einzusetzenden Füllstoffpartikel der Füllstoffkomponente (a) eines erfindungsgemäßen Kompositmaterials wird mittels Lichtstreuung (Laserbeugung) bestimmt, vorzugsweise mit einem Partikelgrößenmessgerät Beckman Coulter LS 13320.

### Komponente (a1): nicht agglomerierte, organisch oberflächenmodifizierte Nanopartikel

Innerhalb eines erfindungsgemäßen Kompositmaterials besteht die Funktion der Nano-partikel u.a. darin, die Zwischenräume zwischen den Mikropartikeln aufzufüllen, um so eine gleichmäßige Füllung des Kompositmaterials zu bewirken, und die Härte und Abrasionsbeständigkeit zu erhöhen. Unter Nanopartikeln werden in Zusammenhang mit der vorliegenden Erfindung Partikel mit einer mittleren Partikelgröße von weniger als 200 nm verstanden. Vorzugsweise ist die mittlere Partikelgröße kleiner als 100 nm und besonders bevorzugt kleiner 60 nm. Je kleiner die Nanopartikel sind, desto besser können sie ihrer Funktion gerecht werden, die Hohlräume zwischen den Mikropartikeln auszufüllen.

Der Anteil organisch oberflächenmodifizierter Nanopartikel mit einer mittleren Partikelgröße kleiner 200 nm ist größer als 12 Gew.-%. In eigenen Untersuchungen hat sich gezeigt, dass bei einem Gehalt von 10 Gew.- % oder weniger an nicht agglomerierten, organisch oberflächenmodifizierten Nanopartikeln mit einer mittleren Partikelgröße kleiner 200 nm das Kompositmaterial im Einzelfall nicht mehr ausreichend abriebfest ist. Dies ist wahrscheinlich u.a. darauf zurückzuführen, dass bei einem Gehalt von 10 Gew.- % oder weniger an den besagten Nanopartikeln die Hohlräume zwischen den Mikropartikeln mit einer mittleren Partikelgröße von 0,4 µm bis 10 µm nicht mehr ausreichend gefüllt sind. Andererseits hat sich gezeigt, dass bei einem Gehalt von mehr als 30 Gew.- % an nicht agglomerierten, organisch oberflächenmodifizierten Nanopartikein mit einer mittleren Partikelgröße kleiner 200 nm die Verarbeitbarkeit des Kompositmaterials nicht mehr ausreichend ist; wegen des hohen Feststoffgehalts wird dann seine Viskosität zu hoch.

Die Materialien für die erfindungsgemäß einzusetzenden Nanopartikel sind vorzugsweise Oxide oder Mischoxide und bevorzugt ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Wismut, Molybdän, Zinn, Zink, Ytterbium, Lanthan, Cer, Aluminium und deren Mischungen. Die bevorzugten oxidischen Nanopartikel sind dabei wie dargelegt nicht agglomeriert.

In einer bevorzugten Ausgestaltung liegen die nanoskaligen Teilchen in nicht agglomerierter Form vor, beispielsweise dispergiert in einem Medium, vorzugsweise in monodisperser Form.

Um eine gute Einbindung der Nanopartikel in die Polymermatrix eines erfindungsgemäßen Kompositmaterials zu ermöglichen, sind die Oberflächen der Nanopartikel (vorzugsweise der bevorzugten oxidischen Nanopartikel) organisch modifiziert, d.h. ihre Oberflächen weisen organische Strukturelemente auf. Beispielhaft genannt sei die Oberflächenbehandlung der Füllstoffe mit einem Silan. Als Haftvermittler eignet sich besonders das Methacryloxypropyltrimethoxysilan.

### Komponente (a2): Mikropartikel mit einer mittleren Partikelgröße im Bereich von 0,4 µm bis 10 µm

Innerhalb eines erfindungsgemäßen Kompositmaterials bewirken die Mikropartikel eine weitgehende gleichmäßige Füllung des Volumens, wobei die verbleibenden Hohlräume zwischen den Mikropartikeln durch die oben beschriebenen Nanopartikel (Komponente (a2)) zumindest teilweise gefüllt werden. Unter Mikropartikeln werden in Zusammenhang mit der vorliegenden Erfindung Partikel mit einer mittleren Partikelgröße von 400 nm bis 10 µm verstanden. Vorzugsweise ist die mittlere Partikelgröße kleiner als 5 µm. In eigenen Untersuchungen hat sich gezeigt, dass die mit den Mikropartikeln bereits erreichbare Volumenfüllung des Kompositmaterials umso vollständiger und gleichmäßiger ist, je kleiner die Mikropartikel sind.

Die Mikropartikel der Komponente (a2) können eine monomodale oder polymodale, beispielsweise eine bimodale Partikelgrößenverteilung aufweisen. Mikropartikel mit einer bimodalen oder multimodalen Partikelgrößenverteilung sind erfindungsgemäß bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Hohlräume zwischen den Partikeln der Fraktionen mit der größeren Partikelgröße ausfüllen werden. Die noch verbleibenden Hohlräume werden wie oben beschrieben durch Nanopartikel gefüllt.

Bevorzugt wird somit in einem erfindungsgemäßen Kompositmaterial eine Komponente (a2) eingesetzt, welche zwei oder mehr Fraktionen von Mikropartikeln enthält, wobei sich die mittleren Partikelgrößen der Fraktionen unterscheiden.

Vorzugsweise enthält Komponente (a2) zumindest zwei Mikropartikel-Fraktionen, wobei deren mittlere Partikelgrößen um mindestens 0,5 µm, bevorzugt um mindestens 0,7 µm, voneinander abweichen. In manchen Ausgestaltungen beträgt die Differenz der mittleren Partikelgrößen der Mikropartikel-Fraktionen mindestens 1,0 µm.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Bevorzugt umfasst ein erfindungsgemäßes Kompositmaterial eine Komponente (a2), welche eine oder mehrere erste Mikropartikelfraktionen, die jeweils eine mittlere Partikelgröße im Bereich von 1 µm bis 10 µm, vorzugsweise 1 µm bis 5 µm, besitzen, und eine oder mehrere zweite Mikropartikelfraktionen, die jeweils eine mittlere Partikelgröße im Bereich von > 0,4 µm bis < 1 µm (d.h. größer als 0,4 µm, aber kleiner als 1 µm), vorzugsweise 0,5 µm bis 0,8 µm, besitzen.

Bevorzugt liegt das Verhältnis der Gesamtmasse der ersten Mikropartikelfraktionen zur Gesamtmasse der zweiten Mikropartikelfraktionen im Bereich von 1 : 1 bis 10 : 1, vorzugsweise im Bereich von 1,5 : 1 bis 5 : 1.

Bevorzugt liegt das Verhältnis der mittleren Korngröße der oder einer ersten Mikropartikelfraktion zur mittleren Korngröße der oder einer zweiten Mikropartikelfraktion der Komponente (a2) im Bereich von 1,5 : 1 bis 10 : 1, vorzugsweise im Bereich von 2 : 1 bis 5 : 1.

In einem besonders bevorzugten erfindungsgemäßen Kompositmaterial umfasst die Komponente (a2) eine oder mehrere erste Mikropartikelfraktionen, die jeweils eine mittlere Partikelgröße im Bereich von 1 µm bis 10 µm, vorzugsweise 1 µm bis 5 µm, besitzen, und eine oder mehrere zweite Mikropartikelfraktionen, die jeweils eine mittlere Partikelgröße im Bereich von > 0,4 µm bis < 1 µm, vorzugsweise 0,5 µm bis 0,8 µm, besitzen; wobei das Verhältnis der Gesamtmasse der ersten Mikropartikelfraktionen zur Gesamtmasse der zweiten Mikropartikelfraktionen im Bereich von 1 : 1 bis 10 : 1, vorzugsweise 1,5 : 1 bis 5 : 1 und/oder das Verhältnis der mittleren Korngröße der oder einer ersten Mikropartikelfraktion zur mittleren Korngröße der oder einer zweiten Mikropartikelfraktion der Komponente (a2) im Bereich von 1,5 : 1 bis 10 : 1, vorzugsweise 2 : 1 bis 5 : 1 liegt.

Die Basismaterialien für die erfindungsgemäß in oberflächenmodifizierter Form einzusetzenden Mikropartikel sind bevorzugt ausgewählt aus der Gruppe bestehend aus amorphen Materialien auf der Basis von SiO₂, ZrO₂ und/oder TiO₂, sowie Mischoxiden, pyrogener Kieselsäure oder Fällungskieselsäure, wie Quarz-Glaskeramik oder Glaspulver (insbesondere Dentalglaspulver), Barium- oder Strontiumgläser, Fluoridionen abgebende Gläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zur besseren Einbindung in die Polymermatrix eines erfindungsgemäßen Kompositmaterials sind die Mikropartikel vorzugsweise organisch oberflächenmodifiziert. Beispielhaft genannt sei die Oberflächenbehandlung der Füllstoffe mit einem Silan, die zu silanisierten Mikropartikeln führt. Zur Oberflächenbehandlung (als Haftvermittler) eignet sich besonders Methacryloxypropyl-trimethoxysilan.

In einem besonders bevorzugten erfindungsgemäßen Kompositmaterial wird zumindest ein Teil der Mikropartikel der Komponente (a2) durch organisch oberflächenmodifizierte Partikel, vorzugsweise silanisierte Partikel gebildet und/oder es wird zumindest ein Teil der Mikropartikel der Komponente (a2) durch Dentalglas-Partikel gebildet; vorzugsweise sind zumindest ein Teil der Mikropartikel der Komponente (a2) organisch oberflächenmodifizierte Dentalglas-Partikel, vorzugsweise silanisierte Dentalglas-Partikel.

Bevorzugt zeichnet sich in diesen Fällen Komponente (a2) durch eine bi- oder multimodale Partikelgrößenverteilung aus, insbesondere eine bi- oder multimodale Partikelgrößenverteilung mit den oben beschriebenen bevorzugten Merkmalen.

### Komponente (a3) - Weitere Füllstoffe

Neben den Komponenten (a1) und (a2) kann die Mischung von Füllstoffpartikeln zusätzlich weitere Füllstoffe als Komponente (a3) umfassen.

So können z.B. verstärkend wirkende Füllstoff-Materialien, wie Glasfasern, Polyamidoder Kohlenstofffasern eingesetzt werden. Ein erfindungsgemäßes Kompositmaterial kann auch feinteilige Splitter oder Perlpolymerisate enthalten, wobei die Perlpolymerisate Homo- oder Copolymere organischer härtbarer Monomerer sein können.

In einer bevorzugten Ausgestaltung betrifft die vorliegende Erfindung ein erfindungsgemäßes Kompositmaterial, vorzugsweise ein erfindungsgemäßes Kompositmaterial wie es vorstehend und nachfolgend als bevorzugt bezeichnet ist, umfassend:
(a) eine Gesamtmenge an Füllstoffen im Bereich von > 75 bis 95 Gew.-%, bezogen auf die Gesamtmasse des Kompositmaterials, wobei die Gesamtmenge an Füllstoffen eine Mischung von Füllstoffen ist bestehend aus
   (a1) einer Gesamtmenge im Bereich von >12 bis 30 Gew.-% an nicht agglomerierten, organisch oberflächenmodifizierten Nanopartikeln mit einer mittleren Partikelgröße kleiner 200 nm, wobei diese Nanopartikel keine Dentalglas-Partikel sind
      und
   (a2) einer Gesamtmenge im Bereich von 45 bis < 83 Gew.-% an Mikropartikeln mit einer mittleren Partikelgröße im Bereich von 0,4 µm bis 10 µm, wobei diese Mikropartikel Dentalglas-Partikel sind
      sowie
   (a3) gegebenenfalls weiteren Füllstoffen, wobei die weiteren Füllstoffe weder nicht agglomerierte, organisch oberflächenmodifizierte Partikel noch Dentalglas-Partikel sind,
   wobei die Gewichtsprozentangaben für die Komponenten (a1) und (a2) auf die Gesamtmasse des Kompositmaterials bezogen sind.

### Bestandteil (b): Monomerenmischung

Innerhalb eines erfindungsgemäßen Kompositmaterials besteht die Funktion der Monomerenmischung (b) darin, eine Matrix zu bilden, in welche die oben genannten Füllstoffe (a) eingebunden sind. Diese Matrix wird gebildet durch jeweilige radikalische Polymerisation von (b1) ein, zwei oder mehr Monomere der Struktur Q(YZₑ)_{b} (wobei jedes Y unabhängig von etwaigen weiteren Strukturelementen Y gewählt ist oder entfällt), und von (b2) einem, zwei oder mehr weiteren radikalisch polymerisierbaren Monomeren aus der Gruppe bestehend aus Acrylaten und Methacrylaten, wobei das Verhältnis der Masse der Komponente (b1) zur Masse der Komponente (b2) im Bereich von 1 : 20 bis 4 : 1 (vorzugsweise 1 : 3 bis 3 : 1) liegt.

Das polyalicyclische Strukturelement Q der Komponente (b1) sorgt dabei für ein sterisch starres und hydrophobes Rückgrat, und die weiteren Monomere der Komponente (b2) sichern eine ausreichende Vernetzung, insbesondere zu den oberflächenmodifizierten Füllstoffen. Eigene Untersuchungen haben gezeigt, dass Pasten mit einem geringeren Anteil an Komponente (b2) bzw. ohne Komponente (b2) eine erhöhte Abrasion aufweisen, wohingegen Pasten mit einem geringeren Anteil an Komponente (b1) bzw. ohne Komponente (b1) eine erhöhte Wasseraufnahme und ungünstigere mechanische Eigenschaften (insbesondere ein verringertes E-Modul) zeigen.

### Komponente (b1): ein, zwei oder mehr Monomere der Struktur Q(YZₑ)_{b} mit mindestens einem polyalicyclischen Strukturelement

Die erste Komponente (b1) der matrixbildenden Monomerenmischung bilden ein, zwei oder mehr Monomere der oben definierten Struktur Q(YZₑ)_{b} (wobei jedes Y unabhängig von etwaigen weiteren Strukturelementen Y gewählt ist oder entfällt), wobei Z vorzugsweise ein Strukturelement bedeutet, das unabhängig von etwaigen weiteren Strukturelementen Z ausgewählt ist aus der Gruppe bestehend aus -O-(C=O)-CH=CH₂, -O-(C=O)-C(CH₃)=CH₂, -(C=O)-CH=CH₂ oder -(C=O)-C(CH₃)=CH₂. Bevorzugt sind Verbindungen der Struktur Q(YZₑ)_{b} (wobei jedes Y unabhängig von etwaigen weiteren Strukturelementen Y gewählt ist oder entfällt), wobei Z ausgewählt ist aus der Gruppe bestehend aus -O-(C=O)-CH=CH₂, -O-(C=O)-C(CH₃)=CH₂, d.h. solche Verbindungen der Struktur Q(YZₑ)_{b}, die ein, zwei oder mehr Acrylat- und/oder Methacrylatgruppen aufweisen, vorzugsweise zwei oder mehr Acrylat- und/oder Methacrylatgruppen.

Die mit den erfindungsgemäßen bzw. erfindungsgemäß zu verwendenden Monomeren der Komponente (b1) erhältlichen Polymere und Kompositmaterialien weisen eine ausgeprägte Hydrophobie auf, die sich u.a. in einer sehr geringen Wasseraufnahme der Polymere und Kompositmaterialien zeigt. Daneben zeichnen sich die unter Verwendung der erfindungsgemäßen bzw. erfindungsgemäß zu verwendenden Monomere der Komponente (b1) erhältlichen Polymere durch eine hohe mechanische Stabilität aus, die sich u.a. in einer hohen Biegefestigkeit der Polymere zeigt. Die erfindungsgemäßen bzw. erfindungsgemäß zu verwendenden Monomere der Komponente (b1), insbesondere gemäß den besonders bevorzugten Ausgestaltungen und Ausführungsformen, lassen sich zu Polymeren verarbeiten, die sowohl eine geringe Wasseraufnahme als auch eine hohe Biegefestigkeit aufweisen.

Die Monomere der Komponente (b1) sind mit den weiteren Monomeren der Komponente (b2) copolymerisierbar, wobei die ausgehärteten Polymere bzw. Formstoffe einen geringen Schrumpf, eine hohe Hydrolysebeständigkeit, eine geringe Wasseraufnahme und eine hohe mechanische Festigkeit aufweisen. Die genannten Eigenschaften sind insbesondere im Bereich der Dentaltechnik wichtig.

Insbesondere die bevorzugten und besonders bevorzugten erfindungsgemäßen bzw. erfindungsgemäß zu verwendenden Verbindungen der Komponente (b1) ermöglichen einen hohen Vernetzungsgrad und sind ferner vorzugsweise radikalisch vernetzbar. Wegen ihrer hochfunktionalisierten Struktur weisen sie eine hohe Vernetzungs- und Polymerisationswahrscheinlichkeit auf.

Bevorzugte erfindungsgemäße oder erfindungsgemäß einzusetzende Verbindungen sind solche, wobei Q ein polyalicyclisches Strukturelement bedeutet, vorzugsweise ein gesättigtes polyalicyclisches Strukturelement, das ausgewählt ist aus der Gruppe bestehend aus bicyclischen und tricyclischen Kohlenwasserstoffresten, wobei vorzugsweise keines der nicht durch Substituenten YZₑ substituierten Wasserstoffatome dieses polyalicyclischen Strukturelements Q substituiert ist.

Sofern eine erfindungsgemäße Verbindung zwei oder mehrere polyalicyclische Strukturelemente umfasst, können diese identisch oder verschieden sein.

Besonders bevorzugt sind erfindungsgemäße oder erfindungsgemäß einzusetzende Monomere Q(YZₑ)_{b} (wobei jedes Y unabhängig von etwaigen weiteren Strukturelementen Y gewählt ist oder entfällt), deren polyalicyclisches Strukturelement Q sich von einem der folgenden tricyclischen Kohlenwasserstoffe ableitet: Tricyclo[5.2.1.0^{2,6}]decan (TCD), Tricyclo[5.2.1.0^{2,6}]dec-3-en oder Tricyclo[3.3.1.1^{3,7}]decan (Adamantan), d.h. bevorzugt sind erfindungsgemäße Verbindungen, die ein TCD - Gerüst, ein Tricyclo[5.2.1.0^{2,6}]dec-3-en - Gerüst oder ein Adamantan-Gerüst aufweisen.

Bei den genannten besonders bevorzugten erfindungsgemäßen oder erfindungsgemäß einzusetzenden Verbindungen, in denen das Strukturelement Q einen Tricyclo[5.2.1.0^{2,6}]decan-Rest, einen Tricyclo[5.2.1.0^{2,6}]dec-3-en-Rest, einen Tricyclo[3.3.1.1^{3,7}]decan-Rest oder einen Bicyclo[2.2.1]heptan-Rest bedeutet, handelt es sich vorzugsweise um solche mit einem Tricyclo[5.2.1.0^{2,6}]decan-Gerüst, einem Tricyclo[5.2.1.0^{2,6}]dec-3-en-Gerüst, einem Tricyclo[3.3.1.1^{3,7}]decan-Gerüst bzw. einem Bicyclo[2.2.1]heptan-Gerüst, bei dem jeweils keines der nicht durch Substituenten YZₑ (wobei jedes Y unabhängig von etwaigen weiteren Strukturelementen Y gewählt ist oder entfällt) substituierten Wasserstoffatome dieses polyalicyclischen Strukturelements Q substituiert ist.

Besonders bevorzugte erfindungsgemäße oder erfindungsgemäß einzusetzende Verbindungen sind solche, wobei das Strukturelement Q einen Tricyclo[5.2.1.0^{2,6}]decan-Rest, einen Tricyclo[5.2.1.0^{2,6}]dec-3-en-Rest, einen Tricyclo[3.3.1.1^{3,7}]decan-Rest oder einen Bicyclo[2.2.1]heptan-Rest bedeutet, ganz besonders bevorzugt bedeutet das Strukturelement Q einen Tricyclo[5.2.1.0^{2,6}]decan-Rest, einen Tricyclo[5.2.1.0^{2,6}]dec-3-en-Rest.

Bevorzugt eingesetzt werden Methacrylsäure- oder Acrylsäureester mit einem Tricyclo[5.2.1.0^{2,6}]-decan- oder Tricyclo[5.2.1.0^{2,6}]-decen-Strukturelement ausgewählt aus der Gruppe bestehend aus
- 8,9-Bis(acryloxymethyl)tricyclo[5.2.1.0^{2,6}]decan
- 8,9-Bis(methacryloyloxymethyl)tricyclo[5.2.1.0^{2,6}]decan
- 8,9-[Bis(2-vinyloxyethyl)oxymethyl]tricyclo[5.2.1.0^{2,6}]dec-3-en
- 8,9-[Bis(2-vinyloxyethyl)oxymethyl]tricyclo[5.2.1.0^{2,6}]decan
- 8-Hydroxymethyl-9-(2-vinyloxyethyl)oxymethyl]tricyclo[5.2.1.0^{2,6}]dec-3-en
- 9-Hydroxymethyl-8-(2-vinyloxyethyl)oxymethyl]tricyclo[5.2.1.0^{2,6}]dec-3-en
- 8,9-Bis(acryloyloxymethyl)tricyclo[5.2.1.0^{2,6}]dec-3-en
- 8,9-Bis(methacryloyloxymethyl)tricyclo[5.2.1.0^{2,6}]dec-3-en
- Diacrylsäure- oder Dimethacrylsäureester von Verbindungen ausgewählt aus der Gruppe bestehend aus:
   - 3,8-Dihydroxymethyl- tricyclo[5.2.1.0^{2,6}]decan
   - 3,9-Dihydroxymethyltricyclo-[5.2.1.0^{2,6}]decan
   - 4,8-Dihydroxymethyltricyclo[5.2.1.0^{2,6}]decan
   - 3,8-Dihydroxytricyclo[5.2.1.0^{2,6}]decan
   - 3,9-Dihydroxytricyclo-[5.2.1.0^{2,6}]decan
   - 4,8-Dihydroxytricyclo[5.2.1.0^{2,6}]decan
- Methacrylsäure- oder Acrylsäureester von Verbindungen aus der Gruppe bestehend aus:
   - Poly(hydroxymethyl-tricyclo[5.2.1.0^{2,6}]decanyl-siloxanen
   - oxyalkyliertes Bishydroxymethyltricyclo[5.2.1.0^{2,6}]decan
   - oxyalkyliertes Bishydroxytricyclo[5.2.1.0^{2,6}]decan
- Urethan- oder Harnstoffgruppen enthaltende Methacrylsäure- oder Acrylsäureester von Verbindungen ausgewählt aus der Gruppe bestehend aus:
   - 3,8-Dihydroxymethyl-tricyclo[5.2.1.0^{2,6}]decan
   - 4,8-Dihydroxymethyl-tricyclo[5.2.1.0^{2,6}]decan
   - 3,9-Dihydroxymethyl-tricyclo[5.2.1.0^{2,6}]decan
   - 4,9-Dihydroxymethyl-tricyclo[5.2.1.0^{2,6}]decan

Hierbei kann in den genannten Verbindungen Wasserstoff am Tricyclo[5.2.1.0^{2,6}]-decan- oder Tricyclo[5.2.1.0^{2,6}]-decen-Rest durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sein.

Ein erfindungsgemäßes Kompositmaterial umfasst vorzugsweise ein, zwei oder mehr Verbindungen der Struktur Q(YZₑ)_{b} (wobei jedes Y unabhängig von etwaigen weiteren Strukturelementen Y gewählt ist oder entfällt) mit einem Tricyclo[5.2.1.0^{2,6}]-decan- oder Tricyclo[5.2.1.0^{2,6}]-decen-Strukturelement, wobei Z vorzugsweise ausgewählt ist aus der Gruppe bestehend aus -O-(C=O)-CH=CH₂ und -O-(C=O)-C(CH₃)=CH₂, besonders bevorzugt bedeutet Z die Gruppe -O-(C=O)-C(CH₃)=CH₂.

Viele der vorstehend aufgelisteten radikalisch polymerisierbaren Methacrylsäure- oder Acrylsäureester mit einem TCD-Strukturelement sind aus dem Stand der Technik bekannt.

In eigenen Untersuchungen hat sich gezeigt, dass mit den oben genannten Monomeren mit mindestens einer radikalisch polymerisierbaren Doppelbindung und einem Tricyclo[5.2.1.0^{2,6}]-decan-Strukturelement der Komponente (b1) Kompositmaterialien mit geringer Polymerisationsschrumpfung (vorzugsweise weniger als 1,7 Vol-%, besonders bevorzugt weniger als 1,6 Vol.-%, gemessen nach der Bonded-Disc-Methode), geringer Abrasion (vorzugsweise weniger als 30 µm, bestimmt nach der ACTA-Methode) und einer hohen Vickers-Mikrohärte (vorzugsweise 140 oder mehr) erhältlich sind.

Y ist vorzugsweise ein Strukturelement, welches in der Struktur Q(YₓZₑ)_{b} das polyalicyclische Strukturelement Q mit e Strukturelementen Z verknüpft und ein Strukturelement enthält oder aus diesem besteht, das ausgewählt ist aus der Gruppe bestehend aus 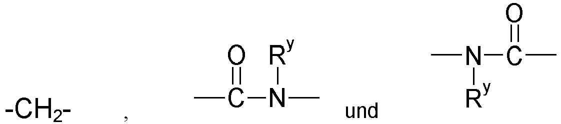
wobei R^{y} einen sonstigen Rest der Verbindung bedeutet und
wobei die jeweils im Formelbild links angeordnete Bindung dem Strukturelement Q und die rechts angeordnete Bindung dem Strukturelement Z näher ist.

Der sonstige Rest R^{y} einer erfindungsgemäßen bzw. erfindungsgemäß einzusetzenden Verbindung der Struktur Q(YₓZₑ)_{b} ist vorzugsweise ausgewählt aus der Gruppe bestehend aus Wasserstoff, linearen, verzweigten oder Ringe umfassenden Strukturelementen mit 1 bis 50 C-Atomen und 0 bis 12 Heteroatomen, wobei die gegebenenfalls vorhandenen Heteroatome vorzugsweise gewählt sind aus der Gruppe bestehend aus N und O.

Der sonstige Rest R^{y} ist dabei bevorzugt ausgewählt aus der Gruppe bestehend aus Wasserstoff, linearen, verzweigten oder Ringe umfassenden Strukturelementen mit 1 bis 40 C-Atomen und 0 bis 10 Heteroatomen, wobei die gegebenenfalls vorhandenen Heteroatome vorzugsweise gewählt sind aus der Gruppe bestehend aus N und O.

Der sonstige Rest R^{y} ist dabei besonders bevorzugt ausgewählt aus der Gruppe bestehend aus Wasserstoff, linearen, verzweigten oder Ringe umfassenden Strukturelementen mit 1 bis 35 C-Atomen und 1 bis 10 Heteroatomen, wobei die gegebenenfalls vorhandenen Heteroatome vorzugsweise gewählt sind aus der Gruppe bestehend aus N und O.

Y ist dabei bevorzugt ein Strukturelement, welches ein Strukturelement enthält oder aus diesem besteht, ausgewählt aus der Gruppe bestehend aus 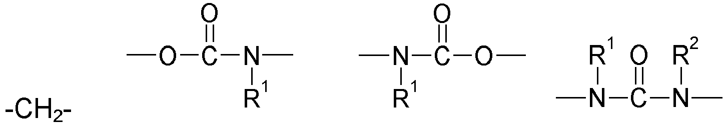 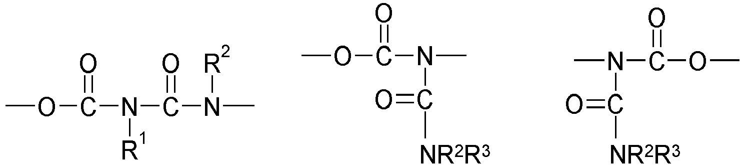 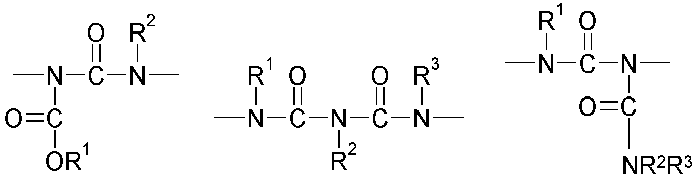 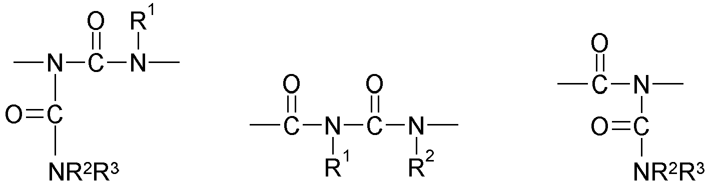 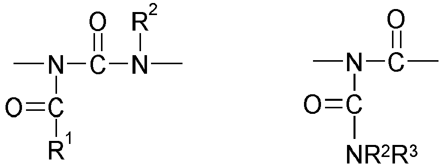 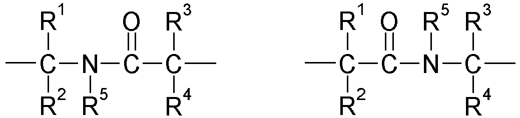 wobei R¹, R², R³, R⁴ und R⁵ sonstige Reste der Verbindung bedeuten, wobei die jeweils im Formelbild links angeordnete Bindung dem Strukturelement Q und die rechts angeordnete Bindung dem Strukturelement Z näher ist.

Die oben genannten sonstigen Reste R¹, R², R³, R⁴ bzw. R⁵ einer erfindungsgemäßen bzw. erfindungsgemäß einzusetzenden Verbindung der Struktur Q(YₓZₑ)_{b} sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste R¹, R², R³, R⁴ bzw. R⁵ sind dabei, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste R¹, R², R³, R⁴ bzw. R⁵ sind dabei, jeweils unabhängig voneinander, besonders bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

In mit vergleichsweise geringem Aufwand synthetisierbaren erfindungsgemäßen bzw. erfindungsgemäß einzusetzenden Verbindungen der Struktur Q(YₓZₑ)_{b} ist Y ein Strukturelement, welches ein Strukturelement enthält oder aus diesem besteht, das ausgewählt ist aus der Gruppe bestehend aus 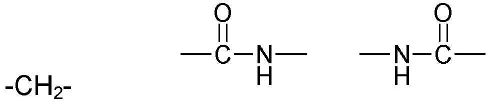 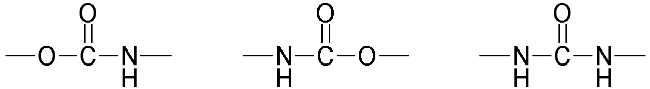 wobei die jeweils im Formelbild links angeordnete Bindung dem Strukturelement Q und die rechts angeordnete Bindung dem Strukturelement Z näher ist.

Die erfindungsgemäßen bzw. erfindungsgemäß einzusetzenden Verbindungen der Struktur Q(YₓZₑ)_{b} können gemäß dem Fachmann bekannten Herstellungsverfahren erhalten werden.

Erfindungsgemäße bzw. erfindungsgemäß einzusetzende Verbindungen der Struktur Q(YₓZₑ)_{b} mit einem Amid-Strukturelement können beispielsweise durch Umsetzung von (i) einer Edukt-Verbindung mit einer Isocyanatgruppe und (ii) einer Edukt-Verbindung mit einer Carbonsäuregruppe erhalten werden.

Erfindungsgemäße bzw. erfindungsgemäß einzusetzende Verbindungen der Struktur Q(YₓZₑ)_{b} mit einem Urethan-Strukturelement können beispielsweise durch Umsetzung von (i) einer Edukt-Verbindung mit einer Isocyanatgruppe und einer Edukt-Verbindung mit einer Alkoholgruppe erhalten werden.

Erfindungsgemäße bzw. erfindungsgemäß einzusetzende Verbindungen der Struktur Q(YₓZₑ)_{b} mit einem Harnstoff-Strukturelement können beispielsweise durch Umsetzung von (i) einer Edukt-Verbindung mit einer Isocyanatgruppe und (ii) einer Edukt-Verbindung mit einer Aminogruppe erhalten werden.

Erfindungsgemäße bzw. erfindungsgemäß einzusetzende Verbindungen der Struktur Q(YₓZₑ)_{b} mit einem Allophanat-Strukturelement können beispielsweise durch Umsetzung von (i) einer Edukt-Verbindung mit einer Urethangruppe und (ii) einer Edukt-Verbindung mit einer Isocyanatgruppe erhalten werden.

Erfindungsgemäße bzw. erfindungsgemäß einzusetzende Verbindungen der Struktur Q(YₓZₑ)_{b} mit einem Biuret-Strukturelement können beispielsweise durch Umsetzung von (i) einer Edukt-Verbindung mit einer Harnstoffgruppe und (ii) einer Edukt-Verbindung mit einer Isocyanatgruppe erhalten werden.

Erfindungsgemäße bzw. erfindungsgemäß einzusetzende Verbindungen der Struktur Q(YₓZₑ)_{b} mit einem *N*-Acylharnstoff-Strukturelement können beispielsweise durch Umsetzung von (i) einer Edukt- Verbindung mit einer Amidgruppe und (ii) einer Edukt-Verbindung mit einer Isocyanatgruppe erhalten werden.

In einer bevorzugten Ausgestaltung eines erfindungsgemäßen Kompositmaterials ist Komponente (b1) so gewählt, dass diese umfasst oder besteht aus Bis(methacryloyloxymethyl)tricyclo[5.2.1.0^{2,6}]decan und/oder Bis(acryloyloxymethyl)tricyclo [5.2.1.0^{2,6}]decan.

In erfindungsgemäßem Kompositmaterial sind die Methacrylsäureester wegen ihrer höheren biologischen Verträglichkeit gegenüber den entsprechenden Acrylsäureestern bevorzugt, d.h. dass Z in Verbindungen der Struktur Q(YZₑ)_{b} (wobei jedes Y unabhängig von etwaigen weiteren Strukturelementen Y gewählt ist oder entfällt) bevorzugt -O-(C=O)-C(CH₃)=CH₂ bedeutet.

Weiterhin bevorzugte Verbindungen (Monomere) der Struktur Q(YZₑ)_{b}, (wobei jedes Y unabhängig von etwaigen weiteren Strukturelementen Y gewählt ist oder entfällt) der Komponente (b1) sind solche mit ein, zwei, drei, vier oder mehr funktionellen Gruppen ausgewählt aus der Gruppe bestehend aus Urethan, Harnstoff, *N*-Acylharnstoff, Allophanat, Biuret und Amid, wobei die Amidfunktion nicht unmittelbar mit einem N-Atom, einem O-Atom oder einer Carbonylgruppe verknüpft ist.

EP 1 238 993 beschreibt ein Verfahren zur Herstellung von Acylharnstoff-Gruppen enthaltenden Polyisocyanaten sowie Gemische hiervon sowie deren Verwendung als Ausgangskomponente bei der Herstellung von Polyurethan-Kunststoffen.

EP 0 209 700 A2 und DE 35 22 005 beschreiben (Meth)acrylsäure-Derivate bestimmter Tricyclodecane mit zweibindigen Brückengliedern aus der Gruppe der Urethane bzw. Harnstoffe, welche im Dentalbereich eingesetzt werden können.

EP 0 000 194 A1 (entsprechend US 4,160,080) beschreibt Polyisocyanate, die Allophanatgruppen enthalten. Diese Allophanatpolyisocyanate können zur Herstellung von Polyurethan-Schaumstoffen, Elastomeren, Duromeren, Beschichtungen, Verklebungen und Lackierungen eingesetzt werden.

EP 0 682 012 B1 betrifft ein Verfahren zur Herstellung von hellfarbigen lichtechten Allophanatgruppen aufweisenden (cyclo)aliphatischen Polyisocyanaten, durch Umsetzung von Urethangruppen aufweisenden organischen Verbindungen mit organischen Polyisocyanaten mit (cyclo)aliphatisch gebundenen Isocyanatgruppen in Gegenwart von Zinn(II)-salzen. Die in EP 0 682 012 B1 beschriebenen Polyisocyanate können als Aufbaukomponente bei der Herstellung von Polyurethankunststoffen verwendet werden.

EP 1 727 846 B1 offenbart ein Verfahren zur Herstellung von Allophanatgruppen enthaltenden Bindemitteln, die unter Einwirkung aktinischer Strahlung mit ethylenisch ungesättigten Verbindungen unter Polymerisation reagierende Gruppen und gegebenenfalls NCO-reaktive Gruppen aufweisen.

EP 0 712 840 B1 betrifft ein Verfahren zur Herstellung von bestimmten Allophanatgruppen aufweisenden Polyisocyanaten durch Umsetzung unter Allophanatbildung von Urethangruppen aufweisenden Verbindungen. Die Verbindungen gemäß EP 0 712 840 B1 können als Bindemittel oder Bindemittelkomponente in Beschichtungsmitteln verwendet werden.

EP 0 867 457 B1 offenbart ein ethylenisch ungesättigtes Polyurethan, das im Wesentlichen frei von Isocyanatgruppen ist, welches das Reaktionsprodukt eines ethylenisch ungesättigten Polyisocyanats, das Allophanatgruppen und β,γ-ethylenisch ungesättigte Ethergruppen enthält, mit einer hydroxyfunktionellen, ethylenisch ungesättigten Verbindung ist, wobei das ethylenisch ungesättigte Polyisocyanat durch Allophanatierung des Urethangruppen-enthaltenden Reaktionsprodukts eines organischen Diisocyanats mit einem β,γ-ethylenisch ungesättigten Etheralkohol hergestellt wird, wobei der β,γ-ethylenisch ungesättigte Etheralkohol eine Verbindung umfasst, die aus der aus Glycerindiallylether, Trimethylolpropandiallylether und Pentaerythtrittriallylether bestehenden Gruppe ausgewählt ist. Die in EP 0 867 457 B1 offenbarten ethylenisch ungesättigten Polyurethane mit Allophanatgruppen können als Bindemittel in Einkomponenten-Beschichtungszusammensetzungen verwendet werden.

DE 10 2007 040 240 A1 und EP 1 645 582 A1 beschreiben jeweils Verfahren zur Herstellung von strahlenhärtenden Allophanaten durch Umsetzung von isocyanatgruppenhaltigen Verbindungen und hydroxyfunktionellen Verbindungen, wobei das Verhältnis von NCO-Gruppen zu den OH-Gruppen 1,45 : 1,0 bis 1,1 : 1,0 beträgt. Gemäß DE 10 2007 040 239 A1 werden unter Einsatz von bestimmten Mischungen enthaltend Hydroxyethylacrylat und Hydroxypropylacrylat als hydroxyfunktionellen Verbindungen entsprechende strahlenhärtenden Allophanate erhalten. Die strahlenhärtenden Allophanate gemäß dieser drei Dokumente können zur Herstellung von Beschichtungen und Lacken sowie Klebstoffen, Druckfarben, Gießharzen, Dentalmassen, Schlichten, Photoresisten, Stereolithographiesystemen, Harzen für Verbundwerkstoffe und Dichtungsmassen verwendet werden.

DE 10 2004 060 285 A1 betrifft strahlungshärtbare Zusammensetzungen auf Basis eines Dicidolgemisches (enthaltend zwei oder drei isomere 3,8-, 4,8- und/oder 5,8-Bis(hydroxymethyl)tricyclo[5.2.1.0^{2,6}]decane) mit mindestens einer Verbindung, welche mindestens eine ethylenisch ungesättigte Gruppierung mit gleichzeitig mindestens einer gegenüber Dicidol reaktive Gruppierung aufweist, wobei diese Verbindung ein Umsetzungsprodukt aus Hydroxyalkyl(meth)acrylat und Diisocyanat sein kann. Die Zusammensetzungen gemäß DE 10 2004 060 285 A1 können verwendet werden als strahlungsinduziert härtende Beschichtungsstoffe, Klebstoffe, Laminierungen, Druckfarben und Tinten, Polituren, Lasuren, Pigmentpasten, Spachtelmassen, Kosmetikartikel, Verpackungsmaterialien und/oder Dicht- und/oder Dämmstoffe.

WO 2006/063891 A1 offenbart radikalisch polymerisierbare Verbindungen, im Wesentlichen enthaltend das Umsetzungsprodukt eines Dicidolgemisches und mindestens einer Verbindung, welche mindestens eine ethylenisch ungesättigte Gruppierung mit gleichzeitig mindestens einer gegenüber Dicidol reaktive Gruppierung aufweist. Die Anwendungsgebiete entsprechen den in DE 10 2004 060 285 A1 genannten.

US 6,670,499 B1 beschreibt vom Adamantan abgeleitet Diurethane. Die in US 6,670,499 beschriebenen Verbindungen eignen sich als Intermediate für den Einsatz im dentalen Bereich oder zur Herstellung von optischen Materialien (wie beispielsweise Linsen).

Auf dem Gebiet der Dentaltechnik besteht ein ständiger Bedarf an weiteren schrumpfungsarmen radikalisch polymerisierbaren Monomeren. Daher war es eine weitere Aufgabe der vorliegenden Erfindung, neue radikalisch polymerisierbare Monomere bereitzustellen, die in einem erfindungsgemäßen Kompositmaterial, insbesondere in einem erfindungsgemäßen dentalen Kompositmaterial, als Bestandteil der Komponente (b1) eingesetzt werden können.

Unter (Meth)acryl ist im Rahmen des vorliegenden Textes sowohl Acryl als auch Methacryl zu verstehen.

Diese weitere Aufgabe wird gelöst durch eine Verbindung der Struktur Q(YZₑ)_{b} (wobei jedes Y unabhängig von etwaigen weiteren Strukturelementen Y gewählt ist) mit ein, zwei, drei, vier oder mehr funktionellen Gruppen ausgewählt aus der Gruppe bestehend aus *N*-Acylharnstoft, Allophanat, Biuret und Amid, wobei die Amidfunktion nicht unmittelbar mit einem N-Atom, einem O-Atom oder einer Carbonylgruppe verknüpft ist, wobei gilt:
- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten YZₑ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind,
- b ist eine natürliche Zahl ausgewählt aus der Gruppe der natürlichen Zahlen 2, 3, 4,
- jedes Z bedeutet ein Strukturelement, das unabhängig von etwaigen weiteren Strukturelementen Z ausgewählt ist aus der Gruppe bestehend aus
   -O-(C=O)-CH=CH₂, -O-(C=O)-C(CH₃)=CH₂,
   -(C=O)-CH=CH₂, -(C=O)-C(CH₃)=CH₂,
   -CH=CH₂, -C(CH₃)=CH₂ und -O-CH=CH₂,
- jeder Index e ist eine natürliche Zahl, die unabhängig von etwaigen weiteren Indizes e ausgewählt ist aus der Gruppe der natürlichen Zahlen 1, 2, 3 und 4,
- jedes Y bedeutet ein Strukturelement, welches in der Struktur Q(YZₑ)_{b} das polyalicyclische Strukturelement Q mit e Strukturelementen Z verbindet,
wobei die Verbindung ein erstes Umsetzungsprodukt ist aus einer ersten Reaktion von
A) einer Verbindung der Struktur QG_{b}, worin jedes G eine reaktive Gruppe bedeutet, die unabhängig von weiteren Gruppen G ausgewählt ist aus der Gruppe bestehend aus (-CH₂)ₙ-NH₂, (-CH₂)ₙ-(OCH₂-CHR)ₘ-OH, (-CH₂)ₙ-NCO und (-CH₂)ₙ-COOH mit
B) zwei oder mehr gleichen oder verschiedenen Verbindungen MZₑ, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, -NH₂, -OH, -NCO und -COOH,
   wobei gilt:
   - R bedeutet jeweils unabhängig von etwaigen weiteren R ein Wasserstoffatom oder einen Alkylrest; bevorzugt bedeutet R ein Wasserstoffatom oder einen linearen oder verzweigten Alkylrest mit 1 bis 6 C-Atomen; weiter bevorzugt bedeutet R ein Wasserstoffatom oder einen Methylrest,
   - m ist eine natürliche Zahl ausgewählt aus der Gruppe der natürlichen Zahlen von 0 bis 10,
   - jeder Index n ist eine natürliche Zahl, die unabhängig von etwaigen weiteren Indizes n ausgewählt ist aus der Gruppe bestehend aus 0 und 1,
   oder
   die Verbindung ein zweites Umsetzungsprodukt ist aus einer Reaktion von dem obigen ersten Umsetzungsprodukt mit
C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
   oder
   die Verbindung ein drittes Umsetzungsprodukt ist aus einer Reaktion von dem obigen zweiten Umsetzungsprodukt mit
D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Aus dem oben Gesagten ergibt sich, dass erfindungsgemäße Verbindungen, die eine Amidgruppe (wie definiert) enthalten, diese Amidgruppe nicht Bestandteil einer Urethangruppe ist.

Weiter bevorzugt sind erfindungsgemäße Verbindungen der Struktur Q(YZₑ)_{b} (wobei jedes Y unabhängig von etwaigen weiteren Strukturelementen Y gewählt ist) der Komponente (b1) mit ein, zwei, drei, vier oder mehr funktionellen Gruppen ausgewählt aus der Gruppe bestehend *N*-Acylharnstoff, Allophanat, Biuret und Amid, wobei die Amidfunktion nicht unmittelbar mit einem N-Atom, einem O-Atom oder einer Carbonylgruppe verknüpft ist, wobei das Amid wiederum vorzugsweise (Meth)acrylamid bedeutet.

In bevorzugten erfindungsgemäßen Verbindungen der Struktur Q(YZₑ)_{b} (wobei jedes Y unabhängig von etwaigen weiteren Strukturelementen Y gewählt ist) erfolgt die Verknüpfung zwischen Q und zumindest einem Strukturelement Z über eine Brücke, die ein zweibindiges Brückenglied enthält oder aus diesem besteht, ausgewählt aus der Gruppe bestehend aus 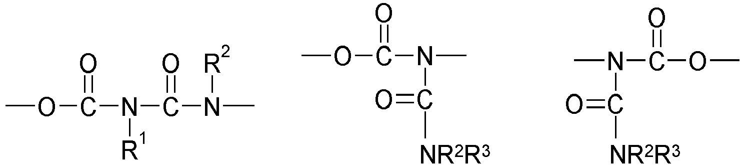 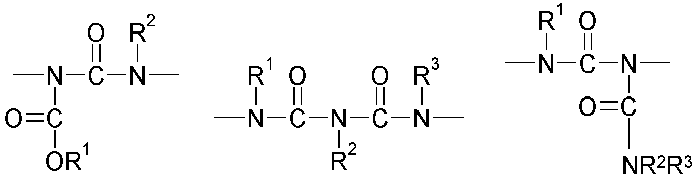 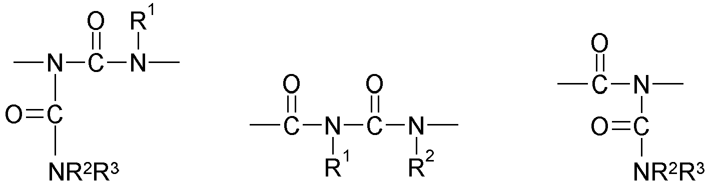 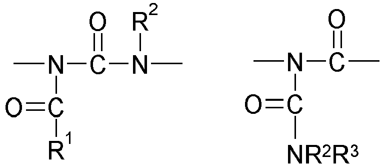 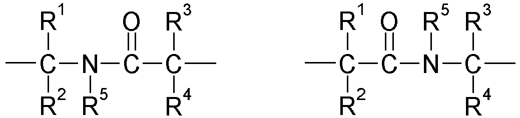 wobei R¹, R², R³ R⁴, R⁵ sonstige Reste der Verbindung bedeuten, wobei die jeweils im Formelbild links angeordnete Bindung dem Strukturelement Q und die rechts angeordnete Bindung dem Strukturelement Z näher ist.

Die weitere Aufgabe wird ebenfalls gelöst durch neue Verbindungen der Struktur Q(YₓZₑ)_{b} mit x = 1 mit ein, zwei, drei, vier oder mehr funktionellen Gruppen, welche ausgewählt sind aus der Gruppe bestehend aus *N*-Acylharnstoff, Allophanat und Biuret, wobei gilt:
- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten YZₑ substituierten Wasserstoffatome dieses polyalicyclischen Strukturelements Q durch Alkylgruppen, Alkoxygruppen, Halogenatome oder Trifluormethylgruppen substituiert ist bzw. sind;
- b ist eine natürliche Zahl ausgewählt aus der Gruppe der natürlichen Zahlen 2, 3, 4;
- jedes Z bedeutet ein Strukturelement, das unabhängig von etwaigen weiteren Strukturelementen Z ausgewählt ist aus der Gruppe bestehend aus
   -O-(C=O)-CH=CH₂, -O-(C=O)-C(CH₃)=CH₂,
   -(C=O)-CH=CH₂, -(C=O)-C(CH₃)=CH₂,
   -CH=CH₂, -C(CH₃)=CH₂ und -O-CH=CH₂,
- jeder Index e ist eine natürliche Zahl, die unabhängig von etwaigen weiteren Indizes e ausgewählt ist aus der Gruppe der natürlichen Zahlen 1, 2, 3 und 4;
- jedes Y bedeutet ein Strukturelement, welches in der Struktur Q(YₓZₑ)_{b} mit x = 1 das polyalicyclische Strukturelement Q mit e Strukturelementen Z verknüpft und ein Strukturelement enthält oder aus diesem besteht, ausgewählt aus der Gruppe bestehend aus 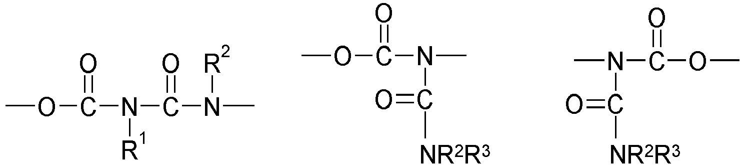 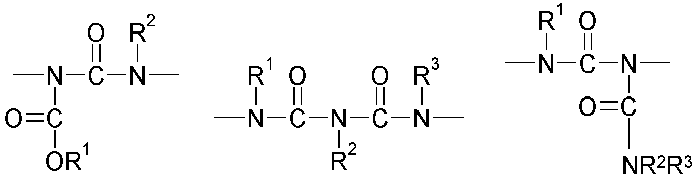 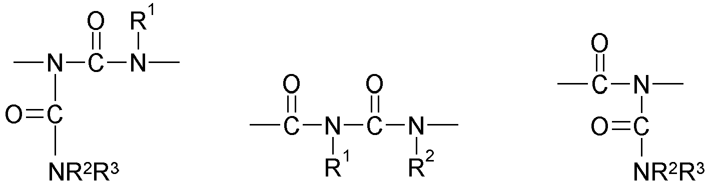 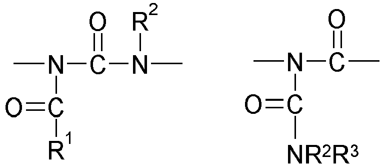 wobei R¹, R² und R³ sonstige Reste der Verbindung bedeuten, wobei die jeweils im Formelbild links angeordnete Bindung dem Strukturelement Q und die rechts angeordnete Bindung dem Strukturelement Z näher ist. Diese erfindungsgemäßen Verbindungen sind in besonderer Weise als Monomere für den Einsatz in erfindungsgemäßen Kompositmaterialien geeignet.

Vorzugsweise umfasst eine solche erfindungsgemäße Verbindung der Struktur Q(YₓZₑ)_{b} mit x = 1 zwei, drei, vier oder mehr funktionelle Gruppen ausgewählt aus der Gruppe bestehend aus *N*-Acylharnstoff, Allophanat und Biuret.

In einer bevorzugten Ausgestaltung bedeutet jeder Index e eine natürliche Zahl, die unabhängig von etwaigen weiteren Indizes e ausgewählt ist aus der Gruppe der natürlichen Zahlen 2, 3 und 4.

Die oben genannten sonstigen Reste R¹, R² bzw. R³ einer erfindungsgemäßen neuen Verbindung sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste R¹, R² bzw. R³ einer erfindungsgemäßen neuen Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste R¹, R² bzw. R³ einer erfindungsgemäßen neuen Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

Eine erfindungsgemäße neue Verbindung Q(YₓZₑ)_{b} mit x = 1, vorzugsweise eine erfindungsgemäße Verbindung Q(YₓZₑ)_{b} mit x = 1 wie sie vorstehend oder nachfolgend als bevorzugt bezeichnet ist, ist vorzugsweise herstellbar durch Umsetzung eines ersten

Umsetzungsproduktes, welches das Umsetzungsprodukt ist aus einer ersten Reaktion von
A) einer Verbindung der Struktur QG_{b}, worin jedes G eine reaktive Gruppe bedeutet, die unabhängig von weiteren Gruppen G ausgewählt ist aus der Gruppe bestehend aus (-CH₂)ₙ-NH₂, (-CH₂)ₙ-(OCH₂-CHR)ₘ-OH, (-CH₂)ₙ-NCO und (-CH₂)ₙ-COOH
   mit
B) zwei oder mehr gleichen oder verschiedenen Verbindungen MZₑ, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, -NH₂, -OH, -NCO und -COOH,
   wobei gilt:
   - R bedeutet jeweils unabhängig von etwaigen weiteren R ein Wasserstoffatom oder einen Alkylrest;
   - m ist eine natürliche Zahl ausgewählt aus der Gruppe der natürlichen Zahlen von 0 bis 10,
   - jeder Index n ist eine natürliche Zahl, die unabhängig von etwaigen weiteren Indizes n ausgewählt ist aus der Gruppe bestehend aus 0 und 1,
   wobei die Verbindung ein zweites Umsetzungsprodukt ist aus einer Reaktion von dem obigen ersten Umsetzungsprodukt mit
C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
   oder
   die Verbindung ein drittes Umsetzungsprodukt ist aus einer Reaktion von dem obigen zweiten Umsetzungsprodukt mit
D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Eine erfindungsgemäße Verbindung gemäß einer bevorzugten Ausgestaltung ist ein zweites Umsetzungsprodukt aus einer Reaktion von dem obigen ersten Umsetzungsprodukt mit
C) einer weiteren Verbindung gemäß A) oder B), wobei jede weitere Verbindung gemäß A) bzw. B) dieselbe Bedeutung hat wie in der ersten Reaktion,
   und/oder
   wobei die Verbindung ein drittes Umsetzungsprodukt ist aus einer Reaktion von dem obigen zweiten Umsetzungsprodukt mit
D) einer weiteren Verbindung gemäß A) oder B), wobei jede weitere Verbindung gemäß A) bzw. B) dieselbe Bedeutung hat wie in der ersten und/oder der zweiten Reaktion, vorzugsweise wie in der ersten und der zweiten Reaktion.

In einer bevorzugten Ausführungsform ist m = 0. Dies gilt für sämtliche Aspekte der vorliegenden Erfindung.

In bevorzugten erfindungsgemäßen Verbindungen erfolgt die Verknüpfung zwischen Q und zumindest einem Strukturelement Z über eine Brücke, die ein zweibindiges Brückenglied enthält oder aus diesem besteht, ausgewählt aus der Gruppe bestehend aus 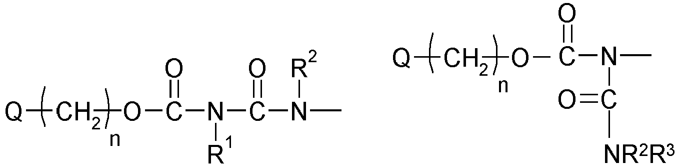 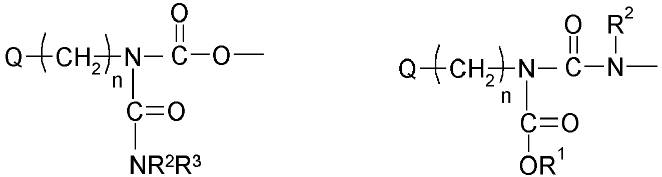 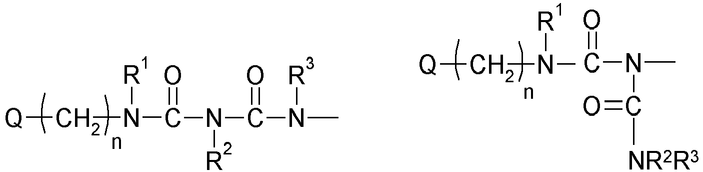 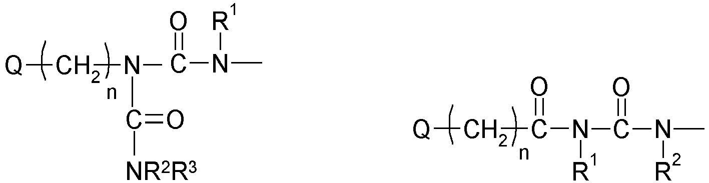 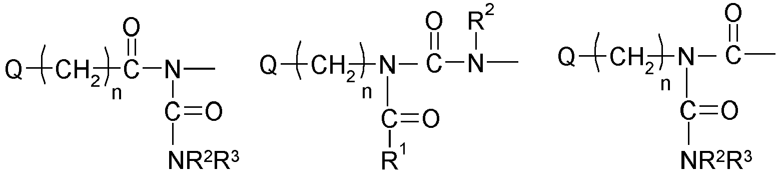 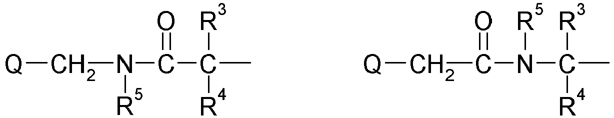 wobei R¹, R², R³ R⁴, R⁵ sonstige Reste der Verbindung bedeuten und Q sowie der Index n die oben angegebene Bedeutung haben.

Die jeweils im Formelbild rechts angeordnete Bindung ist dem Strukturelement Z näher.

In einer bevorzugten Ausgestaltung umfasst eine neue erfindungsgemäße Verbindung Q(YₓZₑ)_{b} mit x = 1, vorzugsweise eine erfindungsgemäße Verbindung Q(YₓZₑ)_{b} mit x = 1 wie sie vorstehend oder nachfolgend als bevorzugt bezeichnet ist, ein oder mehrere Strukturelemente ausgewählt aus der Gruppe bestehend aus 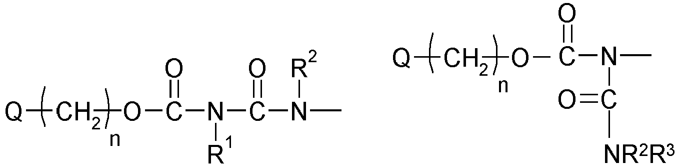 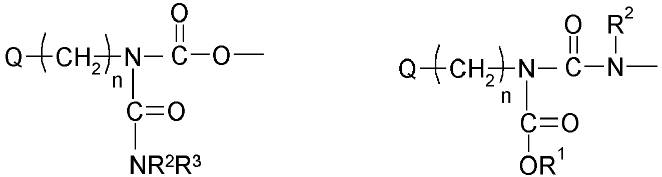 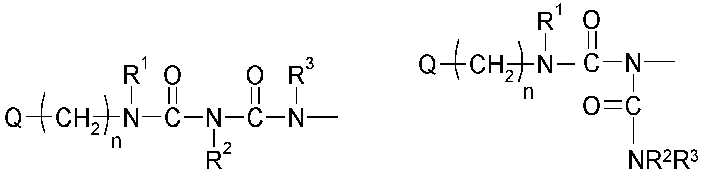 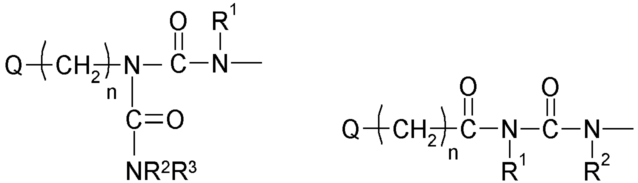 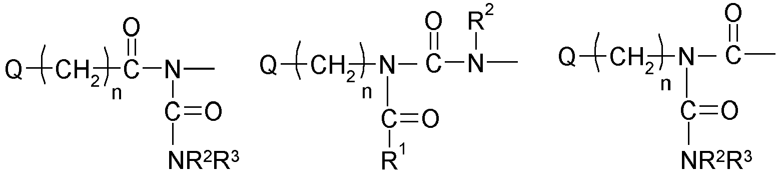 wobei R¹, R² und R³ sonstige Reste der Verbindung bedeuten (und vorzugsweise die oben genannte bevorzugte Bedeutung haben) und Q die oben genannte Bedeutung hat und der Index n ausgewählt ist aus der Gruppe bestehend aus 0 und 1.

Wie oben bereits erwähnt sind bevorzugte erfindungsgemäße neue Verbindungen solche, wobei Q ein gesättigtes polyalicyclisches Strukturelement bedeutet, das ausgewählt ist aus der Gruppe bestehend aus bicyclischen und tricyclischen Kohlenwasserstoffresten, wobei vorzugsweise keines der nicht durch Substituenten YZₑ substituierten Wasserstoffatome dieses polyalicyclischen Strukturelements Q substituiert ist.

Besonders bevorzugte erfindungsgemäße neue Verbindungen sind solche, wobei das Strukturelement Q einen Tricyclo[5.2.1.0^{2,6}]decan-Rest, einen Tricyclo[5.2.1.0^{2,6}]dec-3-en-Rest, einen Tricyclo[3.3.1.1^{3,7}]decan-Rest oder einen Bicyclo[2.2.1]heptan-Rest bedeutet.

Bevorzugte erfindungsgemäße oder erfindungsgemäß einzusetzende neue Verbindungen der Komponente (b1) sind solche, wobei Q einen tricyclischen Kohlenwasserstoffrest bedeutet, wobei vorzugsweise keines der nicht durch Substituenten YZₑ (wobei jedes Y unabhängig von etwaigen weiteren Strukturelementen Y gewählt ist oder entfällt) substituierten Wasserstoffatome dieses tricyclischen Kohlenwasserstoffrestes substituiert ist.

Besonders bevorzugte erfindungsgemäße neue Verbindungen sind solche, wobei das Strukturelement Q einen Tricyclo[5.2.1.0^{2,6}]decan-Rest, einen Tricyclo[5.2.1.0^{2,6}]dec-3-en-Rest oder einen Tricyclo[3.3.1.1^{3,7}]decan-Rest bedeutet, weiter bevorzugt einen Tricyclo[5.2.1.0^{2,6}]decan-Rest oder einen Tricyclo[3.3.1.1^{3,7}]decan-Rest.

Bevorzugt sind erfindungsgemäße neue Verbindungen, in denen
(i) das Strukturelement Z = -O-(C=O)-C(CH₃)=CH₂ bedeutet, wobei die funktionellen Gruppen Allophanat-, Biuret-oder Acylharnstoff-Gruppen sind, da mit diesen Verbindungen besonders gute Ergebnisse erzielt wurden,
   und/oder
(ii) das Strukturelement Q einen Tricyclo[5.2.1.0^{2,6}]decan-Rest bedeutet.

Weiter bevorzugt sind erfindungsgemäße neue Verbindungen, in denen das Strukturelement Z = -O-(C=O)-C(CH₃)=CH₂ bedeutet, wobei die funktionellen Gruppen Allophanat-, Biuret-oder Acylharnstoff-Gruppen sind und das Strukturelement Q einen Tricyclo[5.2.1.0^{2,6}]decan-Rest bedeutet.

Bevorzugt sind erfindungsgemäße neue Verbindungen, in denen sämtliche vorhandenen lichthärtbaren Gruppen dem Strukturelement Z entsprechen.

Bevorzugt sind erfindungsgemäße neue Verbindungen, in denen sämtliche vorhandenen endständigen polymerisierbaren Gruppen dem Strukturelement Z entsprechen.

Eine erfindungsgemäße neue Verbindung kann neben lichthärtbaren Gruppen des Strukturelements Z auch weitere polymerisierbare, vorzugsweise endständige polymerisierbare, Gruppen umfassen, die nicht lichthärtbar sind, insbesondere nicht unter den im Dentalbereich üblichen Bedingungen des Lichthärtens. Dies ist regelmäßig jedoch nicht bevorzugt, da solche Gruppen nicht zu den gewünschten Eigenschaften des nach der Polymerisation vorliegenden Produktes beitragen.

Weiter bevorzugte erfindungsgemäße Verbindungen sind solche, in denen mindestens ein Strukturelement YZₑ unabhängig von dem oder den weiteren Strukturelementen YZₑ ausgewählt ist, und vorzugsweise sämtliche Strukturelemente YZₑ ausgewählt sind, aus der Gruppe bestehend aus 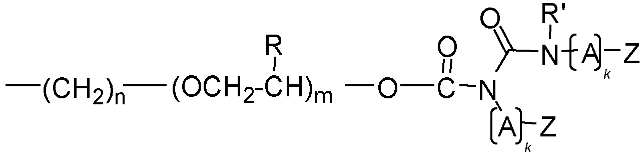 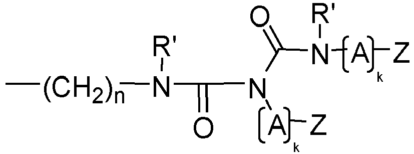 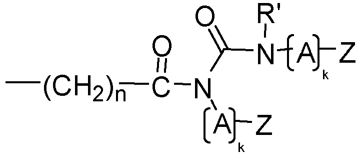 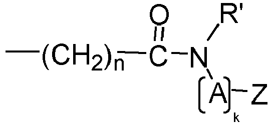 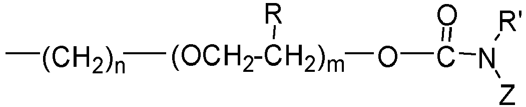 wobei Z, R, m sowie n die obige Bedeutung haben und worin ferner gilt:
- jedes A bedeutet ein zweibindiges organisches Brückenglied,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-(A)ₖ-Z, wobei A, Z und k wiederum die obigen Bedeutungen haben.

In einer bevorzugten Ausführungsform ist m = 0.

Ebenfalls bevorzugte erfindungsgemäße Verbindungen sind solche, bei denen mindestens ein Strukturelement YZₑ unabhängig von dem oder den weiteren Strukturelementen YZₑ ausgewählt ist, und vorzugsweise sämtliche Strukturelemente YZₑ ausgewählt sind, aus der Gruppe bestehend aus 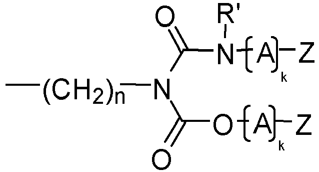 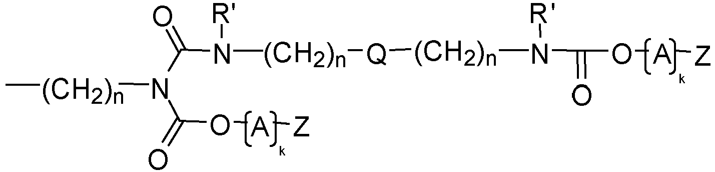 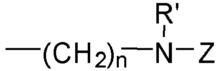 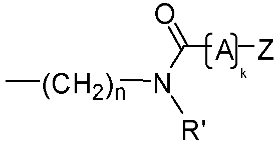 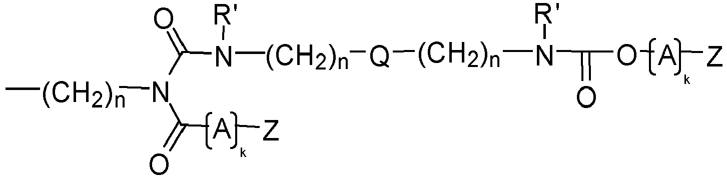 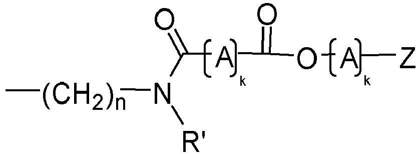 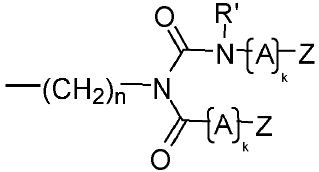 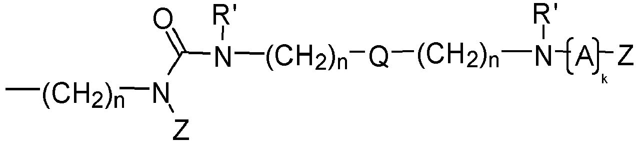 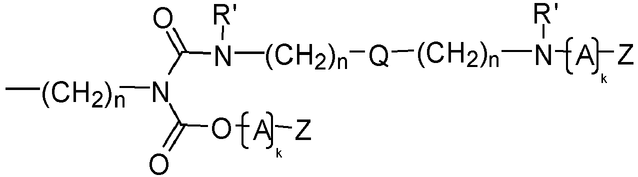 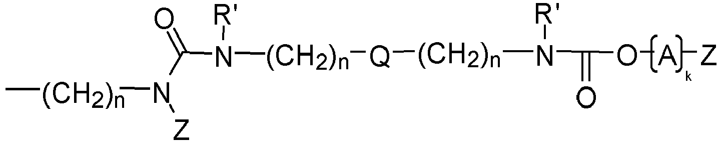 wobei jedes Q unabhängig von etwaigen weiteren Strukturelementen Q die obige Bedeutung hat und wobei Z, A, k und R' sowie n die oben genannte Bedeutung haben.

In einer bevorzugten Ausgestaltung betrifft die vorliegende Erfindung eine erfindungsgemäße Verbindung Q(YₓZₑ)_{b} mit x = 1, vorzugsweise eine erfindungsgemäße Verbindung Q(YₓZₑ)_{b} mit x = 1 wie sie vorstehend oder nachfolgend als bevorzugt bezeichnet ist, wobei
mindestens ein Strukturelement YZₑ unabhängig von dem oder den weiteren Strukturelementen YZₑ ausgewählt ist, und vorzugsweise sämtliche Strukturelemente YZₑ ausgewählt sind,
aus der Gruppe bestehend aus 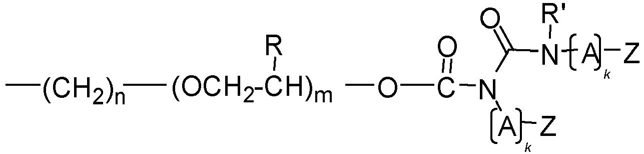 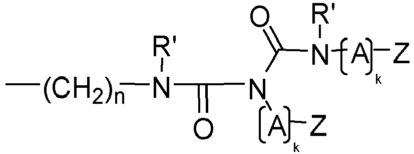 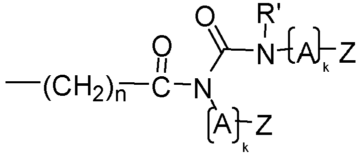 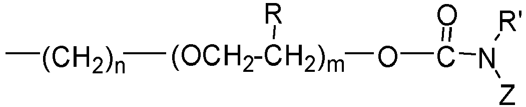 wobei Z, R, m sowie n die obige Bedeutung haben und worin ferner gilt:
- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-(A)ₖ-Z, wobei A, Z und k wiederum die obigen Bedeutungen haben.

In einer bevorzugten Ausgestaltung betrifft die vorliegende Erfindung eine erfindungsgemäße neue Verbindung Q(YₓZₑ)_{b} mit x = 1, vorzugsweise eine erfindungsgemäße Verbindung Q(YₓZₑ)_{b} mit x = 1 wie sie vorstehend oder nachfolgend als bevorzugt bezeichnet ist, wobei
mindestens ein Strukturelement YZₑ unabhängig von dem oder den weiteren Strukturelementen YZₑ ausgewählt ist, und vorzugsweise sämtliche Strukturelemente YZₑ ausgewählt sind,
aus der Gruppe bestehend aus 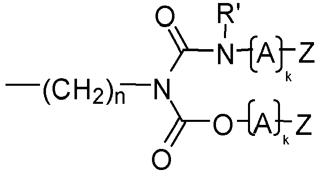 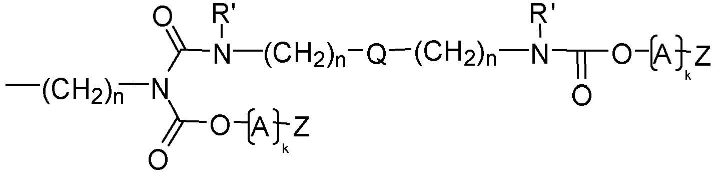 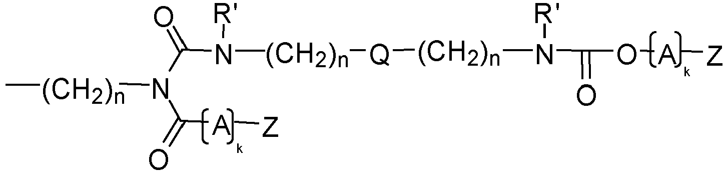 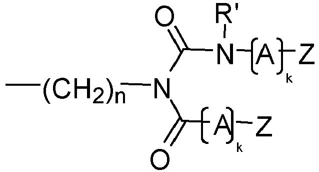 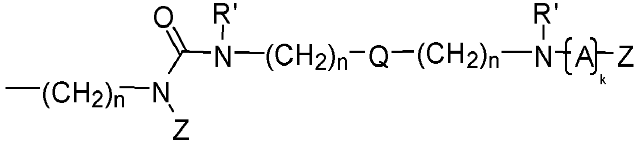 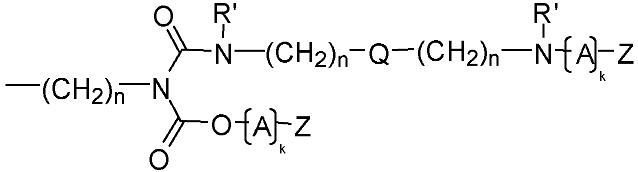 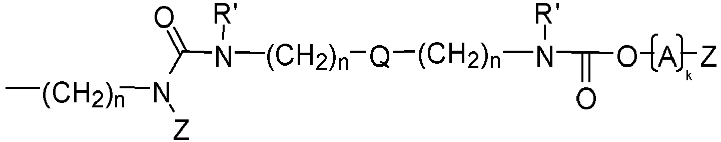 wobei jedes Q unabhängig von etwaigen weiteren Strukturelementen Q die obige Bedeutung hat, und
wobei Z und n die oben genannte Bedeutung haben und wobei ferner gilt:
- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-(A)ₖ-Z, wobei A, Z und k wiederum die obigen Bedeutungen haben.

Dabei wiederum bevorzugt sind erfindungsgemäße einzusetzende Verbindungen, in denen jedes Strukturelement A unabhängig von etwaigen weiteren Strukturelementen A ausgewählt ist aus der Gruppe bestehend aus linearen, verzweigten oder Ringe umfassenden zweibindigen organischen Brückengliedern mit 1 bis 25 C-Atomen und gegebenenfalls 1 bis 10, vorzugsweise 1 bis 5 Heteroatomen, wobei die gegebenenfalls vorhandenen Heteroatome vorzugsweise gewählt sind aus der Gruppe bestehend aus N und O.

Dabei wiederum bevorzugt ist eine erfindungsgemäße Verbindung, in der jedes Strukturelement A unabhängig von etwaigen weiteren Strukturelementen A ausgewählt ist aus der Gruppe bestehend aus linearen, verzweigten oder Ringe umfassenden Strukturelementen mit 1 bis 25 C-Atomen und 0 bis 10 Heteroatomen, vorzugsweise mit 1 bis 5 Heteroatomen, wobei die gegebenenfalls vorhandenen Heteroatome vorzugsweise gewählt sind aus der Gruppe bestehend aus N und O.

Weiter bevorzugt sind Verbindungen, in denen jedes Strukturelement A unabhängig von etwaigen weiteren Strukturelementen A ausgewählt ist aus der Gruppe bestehend aus C₁-C₂₀-Alkylen, C₁-C₂₀-Heteroalkylen, C₃-C₂₀-Cycloalkylen, C₄-C₂₀-Cycloalkylalkylen, C₂-C₂₀-Alkenylen, C₃-C₂₀-Cycloalkenylen, C₄-C₂₀-Cycloalkenylalkylen, C₄-C₂₀-Cycloalkenylenalkylen, C₃-C₂₅-Arylen, C₂-C₂₅-Heteroarylen, C₄-C₂₅-Arylalkylen, C₄-C₂₅-Arylenalkylen, C₄-C₂₅-Arylheteroalkylen, C₄-C₂₅-Arylenheteroalkylen.

In bevorzugten Ausgestaltungen umfasst Strukturelement A eine oder mehrere der folgenden Atome oder Atomgruppen:
-O-, -O-Ar¹-CR⁶R⁷-Ar²-O-, -NR⁸-, -N-(C=O)-, -NH-(C=O)-O-, -NH-C(=O)-NH-
wobei gilt:
Ar¹ und Ar² bedeuten unabhängig voneinander einen gegebenenfalls substituierten, dabei vorzugsweise einen ein oder mehrfach mit C1-C4-Alkylresten substituierten, aromatischen Ring, dabei wiederum bevorzugt einen Phenylring,
R⁶, R⁷ und R⁸ bedeuten unabhängig voneinander Wasserstoff oder einen C1-C8-Rest, dabei vorzugsweise einen C1-C4-Alkylrest, dabei wiederum bevorzugt Methyl oder Ethyl.

Die vorliegende Erfindung betrifft ferner ein Verfahren zur Herstellung einer erfindungsgemäßen Verbindung Q(YZₑ)_{b} (wobei jedes Y unabhängig von etwaigen weiteren Strukturelementen Y gewählt ist) oder einer Mischung, umfassend zumindest eine solche erfindungsgemäße Verbindung Q(YZₑ)_{b}, mit folgenden Schritten:
In einer ersten Reaktion Umsetzen von
   A) einer Verbindung der Struktur QG_{b}, worin jedes G eine reaktive Gruppe bedeutet, die unabhängig von weiteren Gruppen G ausgewählt ist aus der Gruppe bestehend aus (-CH₂)ₙ-NH₂, (-CH₂)ₙ-(OCH₂-CHR)ₘ-OH, (-CH₂)ₙ-NCO und (-CH₂)ₙ-COOH, vorzugsweise einer Verbindung der Struktur QG_{b}, worin jedes G eine reaktive Gruppe bedeutet, die unabhängig von weiteren Gruppen G ausgewählt ist aus der Gruppe bestehend aus -NH₂, -CH₂NH₂, -OH, -CH₂OH, -NCO, -CH₂NCO, und -COOH,
      mit
   B) zwei oder mehr gleichen oder verschiedenen Verbindungen MZₑ, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, -NH₂, -OH, -NCO und -COOH
      zu einem ersten Umsetzungsprodukt,
      gegebenenfalls in einer zweiten Reaktion Umsetzen des ersten Umsetzungsproduktes mit
   C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
      zu einem zweiten Umsetzungsprodukt
      und gegebenenfalls in einer dritten Reaktion Umsetzen des zweiten Umsetzungsproduktes mit
   D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.
wobei Q, b, Y, Z, und e jeweils die oben genannte Bedeutung haben, und wobei gilt:
- R bedeutet jeweils unabhängig von etwaigen weiteren R ein Wasserstoffatom oder einen Alkylrest; bevorzugt bedeutet R ein Wasserstoffatom oder einen linearen oder verzweigten Alkylrest mit 1 bis 6 C-Atomen; weiter bevorzugt bedeutet R ein Wasserstoffatom oder einen Methylrest;
- m ist eine natürliche Zahl ausgewählt aus der Gruppe der natürlichen Zahlen von 0 bis 10,
- jeder Index n ist eine natürliche Zahl, die unabhängig von etwaigen weiteren Indizes n ausgewählt ist aus der Gruppe bestehend aus 0 und 1,
wobei das Verhältnis der Gesamtzahl von NCO-Gruppen zu der Gesamtzahl von-NH₂, -OH und -COOH in der Gesamtzahl von Verbindungen gemäß A) und B) in der ersten, gegebenenfalls zweiten und gegebenenfalls dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.

Ein erfindungsgemäß bevorzugtes Verfahren zur Herstellung einer erfindungsgemäßen Verbindung Q(YₓZₑ)_{b} mit x = 1, vorzugsweise einer erfindungsgemäßen Verbindung Q(YₓZₑ)_{b} mit x = 1 wie sie vorstehend oder nachfolgend als bevorzugt bezeichnet ist, oder einer Mischung umfassend zumindest eine solche Verbindung Q(YₓZₑ)_{b}, ist ein Verfahren mit folgenden Schritten:
In einer ersten Reaktion Umsetzen von
   A) einer Verbindung der Struktur QG_{b}, worin jedes G eine reaktive Gruppe bedeutet, die unabhängig von weiteren Gruppen G ausgewählt ist aus der Gruppe bestehend aus (-CH₂)ₙ-NH₂, (-CH₂)ₙ-(OCH₂-CHR)ₘ-OH, (-CH₂)ₙ-NCO und (-CH₂)ₙ-COOH
      mit
   B) zwei oder mehr gleichen oder verschiedenen Verbindungen MZₑ, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, -NH₂, -OH, -NCO und -COOH
      zu einem ersten Umsetzungsprodukt,
      in einer zweiten Reaktion Umsetzen des ersten Umsetzungsproduktes mit
   C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
      zu einem zweiten Umsetzungsprodukt
      und gegebenenfalls in einer dritten Reaktion Umsetzen des zweiten Umsetzungsproduktes mit
   D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.
   wobei Q, b, Y, Z, e, M, R, m und n jeweils die oben genannte Bedeutung haben, und
   wobei das Verhältnis der Gesamtzahl von NCO-Gruppen zu der Gesamtzahl von -NH₂,-OH und -COOH in der Gesamtzahl von Verbindungen gemäß A) und B) in der ersten, der zweiten und gegebenenfalls der dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.

In einem erfindungsgemäßen Verfahren zur Herstellung einer erfindungsgemäßen Verbindung Q(YₓZₑ)_{b} mit x = 1, vorzugsweise einer erfindungsgemäßen Verbindung Q(YₓZₑ)_{b} mit x = 1 wie sie vorstehend oder nachfolgend als bevorzugt bezeichnet ist, oder einer Mischung umfassend zumindest eine solche Verbindung Q(YₓZₑ)_{b}, liegt das Verhältnis der Gesamtzahl von umgesetzten NCO-Gruppen zu der Gesamtzahl von umgesetzten-NH₂, -OH und -COOH in der Gesamtzahl von Verbindungen gemäß A) und B) in der ersten, gegebenenfalls zweiten und gegebenenfalls dritten Reaktion bevorzugt im Bereich von 1,1 : 1 bis 5 : 1, weiter bevorzugt im Bereich von 1,25 : 1 bis 4 : 1, besonders bevorzugt im Bereich von 1,5 : 1 bis 3 : 1, und am meisten bevorzugt im Bereich von 2 : 1 bis 2,5 : 1.

Vorzugsweise erfolgt die Reaktion zu dem ersten Umsetzungsprodukt, zu dem zweiten Umsetzungsprodukt und/oder zu dem dritten Umsetzungsprodukt in Gegenwart eines Katalysators.

Bevorzugte Katalysatoren sind dabei tertiäre Amine oder Lewis Säuren, dabei wiederum bevorzugt Metallsalze höherer Fettsäuren, insbesondere Dibutylzinndilaurat oder Zinn(II)octoat.

Die Menge des Katalysators liegt dabei vorzugsweise im Bereich von 0,01 bis 2 Gew.-%, bevorzugt von 0,08 bis 1 Gew.-%, bezogen auf die Gesamtmenge der Reaktanden gemäß A) und B) sowie gegebenenfalls C) und gegebenenfalls D).

Die Reaktion zu dem ersten Umsetzungsprodukt, zu dem zweiten Umsetzungsprodukt und/oder zu dem dritten Umsetzungsprodukt erfolgt vorzugsweise in einem Temperaturbereich von 0 bis 160°C, bevorzugt im Bereich von 30 bis 140°C und besonders bevor-zugt im Bereich von 60 bis 120°C. Die Umsetzung wird vorzugsweise bei Normaldruck (1013 mbar) ausgeführt.

In einem weiteren Aspekt betrifft die vorliegende Erfindung eine Mischung umfassend ein, zwei oder mehr unterschiedliche erfindungsgemäße Verbindungen, herstellbar nach einem erfindungsgemäßen Verfahren.

Die vorliegende Erfindung betrifft ferner die Verwendung einer erfindungsgemäßen Verbindung, vorzugsweise in einer der als bevorzugt bzw. besonders bevorzugt gekennzeichneten Ausgestaltungen, in einem Kompositmaterial, vorzugsweise in einem dentalen Kompositmaterial.

Die vorliegende Erfindung betrifft auch die Verwendung einer erfindungsgemäßen Verbindung, vorzugsweise in einer der als bevorzugt bzw. besonders bevorzugt gekennzeichneten Ausgestaltungen, zur Herstellung eines Kompositmaterials, vorzugsweise eines dentalen Kompositmaterials, insbesondere eines restaurativen Kompositmaterials, dabei bevorzugt als Füllungs-, Unterfüllungs-, Befestigungs- und/oder Stumpfaufbaumaterial, als provisorisches Kronen- und/oder Brückenmaterial, als Prothesen- und/oder Unterfütterungsmaterial oder als Flow-Material.

Die vorliegende Erfindung betrifft zudem eine erfindungsgemäße Verbindung, vorzugsweise in einer der als bevorzugt bzw. besonders bevorzugt gekennzeichneten Ausgestaltungen, als bzw. zur Verwendung als Dentalmaterial, insbesondere als Füllungs-, Unterfüllungs-, Befestigungs- und/oder Stumpfaufbaumaterial, als provisorisches Kronen- und/oder Brückenmaterial, als Prothesen- und/oder Unterfütterungsmaterial oder als Flow-Material.

Zur Herstellung der erfindungsgemäßen Verbindungen können vorzugsweise Hydroxylverbindungen von (Meth)acrylaten eingesetzt werden, wobei auch Gemische von Acrylaten und Methacrylaten verwendet werden können. Bevorzugt sind (zum Einsatz als Reaktionspartner gemäß Komponente B), C) und/oder D)):
Alkylenoxidmono(meth)acrylate wie beispielsweise Ethylenglykolmono(meth)acrylat, Diethylenglykolmono(meth)acrylat, Triethylenglykolmono(meth)acrylat, Tetraethylenglykolmono(meth)acrylat, etc., Polyalkylenoxidmono(meth)acrylate wie beispielsweise Polyethylenglykolmono(meth)acrylat, Polypropylenglykolmono(meth)acrylat, Polybutylenglykolmono(meth)acrylat, etc., Hydroxyalkylmono(meth)acrylate wie beispielsweise Hydroxyethyl(meth)acrylat, Hydroxypropyl(meth)acrylat, 2-Hydroxypropyl(meth)acrylat, Hydroxybutyl(meth)acrylat, 4-Hydroxybutyl(meth)acrylat, Hydroxypentyl(meth)acrylat, 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat, Hydroxydodecyl(meth)acrylat, etc., Poly(ε-caprolacton)mono(meth)acrylat, Poly(γ-caprolacton)mono(meth)acrylat, etc., die Mono-, Di-, Tetra-, oder Penta(meth)acrylate mehrwertiger Alkohole, wie Glycerin, wie beispielsweise Glycerindi(meth)acrylat (2-Hydroxypropyl-1,3-di(meth)acrylat, 3-Hydroxypropyl-1,2-di(meth)acrylat), wie Trimethylolpropan, wie beispielsweise Trimethylolpropandi(meth)acrylat, wie Pentaerythrit, wie beispielsweise Pentaerythritol-tri(meth)acrylat, wie Dipentaerythrit, wie beispielsweise Dipentaerythritol-penta(meth)acrylat, wie Ditrimethylolpropantri(meth)acrylat, wie Neopentylglykol(meth)acrylat, die (Meth)acrylate von alkoxyliertem oder phenoxyliertem Glycerin, dabei vorzugsweise die (Meth)acrylate von ethoxyliertem, propoxyliertem, etc. Glycerin, Trimethylolpropan, Pentaerythrit, Dipentaerythrit, Ditrimethylolpropan, etc. sowie deren technische Gemische, Bisphenol-A-Glycidyl-(Meth)acrylat (Bis-GMA), Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat (z.B. ethoxyliertes Bisphenol-A-Glycidyl-(Meth)acrylat), etc..

Zur Herstellung der erfindungsgemäßen Verbindungen können als Komponente B) auch Isocyanate eingesetzt werden. Bevorzugt sind dabei Mono- und Diisocyanate.

Bevorzugte Diisocyanate sind gewählt aus der Gruppe bestehend aus Cyclohexandiisocyanat, Methylcyclohexandiisocyanat, Ethylcyclohexandiisocyanat, Propylcyclohexandiisocyanat, Methyldiethylcyclohexandiisocyanat, Phenylendiisocyanat, Toluylendiisocyanat, Bis(isocyanatophenyl)methan, Propandiisocyanat, Butandiisocyanat, Pentandiisocyanat, Hexandiisocyanat, wie Hexamethylendiisocyanat oder 1,5-Diisocyanato-2-methylpentan, Heptandiisocyanat, Octandiisocyanat, Nonandiisocyanat, wie 1,6-Diisocyanato-2,4,4-trimethylhexan oder 1,6-Diisocyanato-2,2,4-trimethylhexan, Nonantriisocyanat, wie 4-Isocyanatomethyl-1,8-octandiisocyanat, Decandi- und triisocyanat, Undekandi- und -triisocyanat, Dodecandi- und -triisocyanate, Isophorondiisocyanat, Dicyclohexylmethan-4,4'-diisocyanat, Isocyanatomethylmethylcyclohexylisocyanat, 1,3-Bis(isocyanatomethyl)cyclohexan oder 1,4-Bis(isocyanatomethyl)cyclohexan.

Bevorzugte Monoisocyanate sind (Meth)acryloylisocyanat und (Meth)acryl-C2-C8-alkylisocyanate (d.h. (Meth)acrylalkylisocyanate mit Alkylspacern, die über 2 bis 8, besonders bevorzugt 2 bis 6 Kohlenstoffatome verfügen), dabei wiederum bevorzugt ist (Meth)acrylethylisocyanat (2-Isocyanatoethyl(meth)acrylat).

Außerdem haben sich als Komponente B) Monoisocyanate vorteilhaft erwiesen, die Umsetzungsprodukte sind aus Amino- bzw. Hydroxyalkyl(meth)acrylaten, deren Alkylspacer über 1 bis 12, bevorzugt 2 bis 8, besonders bevorzugt 2 bis 6 Kohlenstoffatome verfügen, und Diisocyanaten.

Vorzugsweise wird dazu ein vorstehend genanntes Diisocyanat äquimolar mit einer (oben als bevorzugt gekennzeichneten) Amino- oder Hydroxylalkylverbindung eines (Meth)acrylats umgesetzt, wobei die Hydroxylalkylverbindungen wiederum vorzugsweise gewählt sind aus der Gruppe bestehend aus Hydroxyethyl(meth)acrylat, Hydroxypropyl(meth)acrylat, Hydroxybutyl(meth)acrylat, Hydroxypentyl(meth)acrylat, Hydroxyhexyl(meth)acrylat.

Als Beispiele seien die Umsetzungsprodukte im molaren Verhältnis von 1 : 1 von Hydroxyethylmethacrylat mit Isophorondiisocyanat, Dicyclohexylmethan-4,4'-diisocyanat oder Hexamethylendiisocyanat genannt.

Im Folgenden wird die Erfindung zunächst für Monomere umfassend tricyclische Strukturelemente Q am Beispiel von Tricyclo[5.2.1.0^{2,6}]decan (TCD) - Derivaten im Detail erläutert.

### 1.) Ausgehend vom Bis(hydroxymethyl)tricyclo[5.2.1.0^{2,6}]decan (TCD-Diol)

Bis(hydroxymethyl)tricyclo[5.2.1.0^{2,6}]decan ist kommerziell erhältlich, beispielsweise als Dicidolgemisch der isomeren Verbindungen 3,8-Bis(hydroxymethyl)tricyclo[5.2.1.0^{2,6}]decan und 4,8-Bis(hydroxymethyl)tricyclo[5.2.1.0^{2,6}]decan sowie 3,9-Bis(hydroxymethyl)tricyclo[5.2.1.0^{2,6}]decan und 4,9-Bis(hydroxymethyl)tricyclo[5.2.1.0^{2,6}]decan.

Die Bis(hydroxymethyl)tricyclo[5.2.1.0^{2,6}]decane können, ausgehend von Dicyclopentadien (Tricyclo[5.2.1.0^{2,6}]deca-3,8-dien), synthetisiert werden. Dicyclopentadien ist präparativ leicht in einer Diels-Alder Reaktion durch Dimerisierung von Cyclopentadien zugänglich. Hydroformylierung von Dicyclopentadien ergibt dann das Bis(hydroxymethyl)tricyclo[5.2.1.0^{2,6}]decan. Je nach Synthesepfad können gezielt an unterschiedlichen Positionen substituierte Bis(hydroxymethyl)tricyclo[5.2.1.0^{2,6}]decane erhalten werden. So werden in den Druckschriften JP 7-206740, EP 1 112 995 B1 oder EP 0 049 631 B1 Vorschriften angegeben, wie beispielsweise das 8,9-Bis(hydroxymethyl)tricyclo[5.2.1.0^{2,6}]decan herstellbar ist. Die DE 103 52 260 B3 beschreibt dagegen Verfahren zur Herstellung von 3(4), 8(9)-Bis(hydroxymethyl)tricyclo[5.2.1.0^{2,6}]decan. Die Notation der Positionen der Hydroxymethylgruppen 3(4), 8(9) bedeutet 3 oder 4, 8 oder 9.

Das im Handel erhältliche und als Ausgangsverbindung zur Herstellung erfindungsgemäßer Monomere einsetzbare Bis(hydroxymethyl)tricyclo[5.2.1.0^{2,6}]decan enthält somit Hydroxymethylgruppen sowohl an den Positionen 3 oder 4 als auch in den Stellungen 8 oder 9. Es ist nun möglich, durch Anlagerung von Alkylenoxiden, im Allgemeinen in Mengen von 1 bis 10 mol, insbesondere von Ethylenoxid, Propylenoxid, Butylenoxid, etc. in Gegenwart basischer Katalysatoren nach bekannten Verfahren die entsprechenden Polyetherpolyole zu synthetisieren. Die EP 0 023 686 B1 enthält hierzu genaue Herstellvorschriften.

Die Umsetzung der 3(4), 8(9)-Bis(hydroxymethyl)tricyclo[5.2.1.0^{2,6}]decane mit Isocyanaten zu den entsprechenden Urethanen ist ebenfalls bekannt. So beschreibt die DE 35 22 006 A1 die Reaktion des 3(4), 8(9)-Bis(hydroxymethyl)tricyclo[5.2.1.0^{2,6}]decans mit 2-Isocyanatoethylmethacrylat. 2-Isocyanatoethylmethacrylat ist kommerziell erhältlich oder kann gemäß der Herstellvorschrift aus der DE 33 38 077 A1 durch Phosgenierung von Dihydrooxazinen synthetisiert werden.

Das erhaltene Reaktionsprodukt (Formel (1)) von 2-Isocyanatoethylmethacrylat mit 3(4), 8(9)-Bis(hydroxymethyl)tricyclo[5.2.1.0^{2,6}]decan weist in einer Formulierung nach Aushärtung einen niedrigen Reaktionsschrumpf und eine hohe mechanische Festigkeit auf.

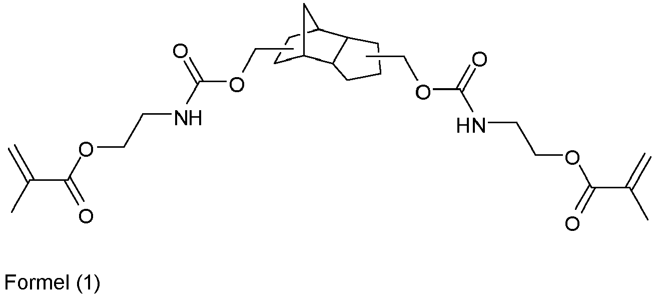

Das Urethan der Formel (1) besitzt noch zwei reaktionsfähige Wasserstoffatome am Stickstoff, die nun in einer zweiten Umsetzungsstufe mit überschüssigem Isocyanat weiter abreagiert werden unter Bildung einer erfindungsgemäßen Verbindung. Es bildet sich hierbei zunächst das Allophanat der Formel (2) als tetrafunktionalisierte, radikalisch vernetzbare Verbindung. Auch dieses Monomer weist wiederum noch umsetzungsfähige Wasserstoffatome am Stickstoff auf, die erfindungsgemäß bei Reaktion mit weiterem Isocyanat das hexafunktionalisierte, radikalisch härtbare Allophanat der Formel (3) bilden.

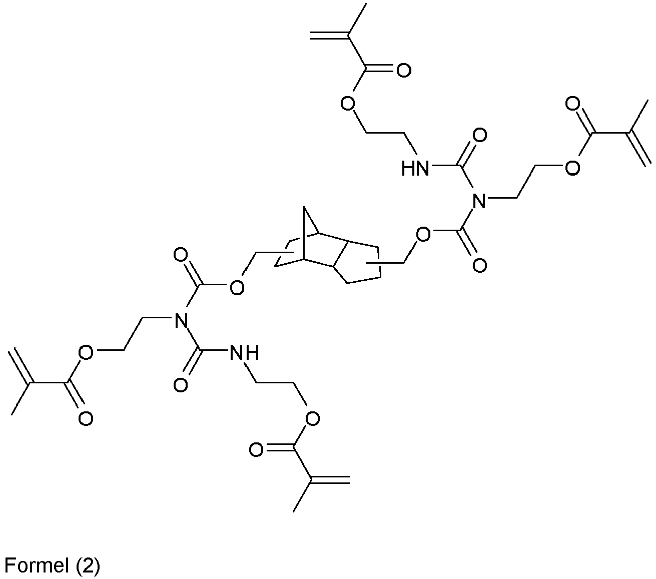

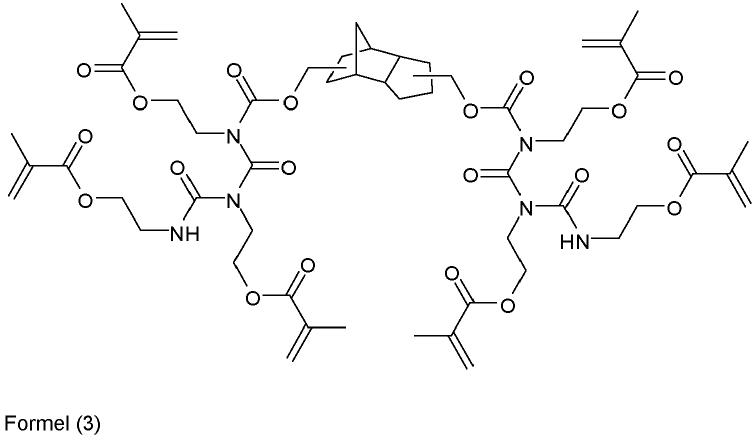

Alternativ kann das 3(4), 8(9)-Bis(hydroxymethyl)tricyclo[5.2.1.0^{2,6}]decan auch mit Methacryloylisocyanat zur Reaktion gebracht werden. Methacryloylisocyanat ist kommerziell oder durch Umsetzung von Methacrylamid mit Oxalylchlorid erhältlich, wie in der EP 0 143 613 B1 beschrieben. Durch Umsetzung von 3(4), 8(9)-Bis(hydroxymethyl)tricyclo[5.2.1.0^{2,6}]decan mit Methacryloylisocyanat wird eine Verbindung der Formel (4) erhalten:

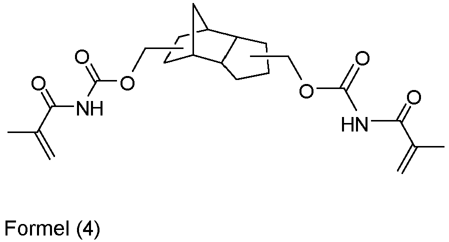

Die verbleibenden reaktiven Wasserstoffatome am Stickstoff der Verbindung der Formel (4) können anschließend wiederum in Isocyanatreaktionen zu Allophanaten umgesetzt werden. Beispielhaft sei hier das Reaktionsprodukt mit 2-Isocyanatoethylmethacrylat (Formel (5)) gezeigt.

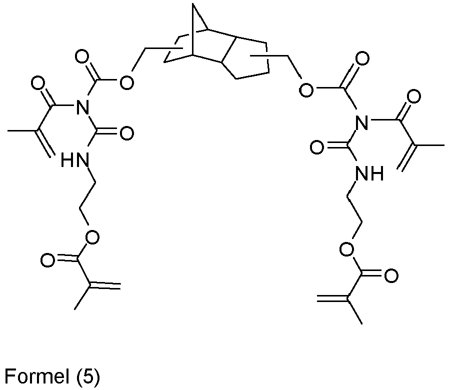

### 2.) Ausgehend von 3(4), 8(9)-Bis(Carbonsäure)tricyclo[5.2.1.0^{2,6}]decan

Das 3(4), 8(9)-Bis(Carbonsäure)tricyclo[5.2.1.0^{2,6}]decan ist durch einfache Oxidation des kommerziell erhältlichen 3(4), 8(9)-Bis(formyl)tricyclo[5.2.1.0^{2,6}]decans herstellbar. Umsetzung der Dicarbonsäure mit 2-Isocyanatoethylmethacrylat ergibt das Amid der Formel (8):

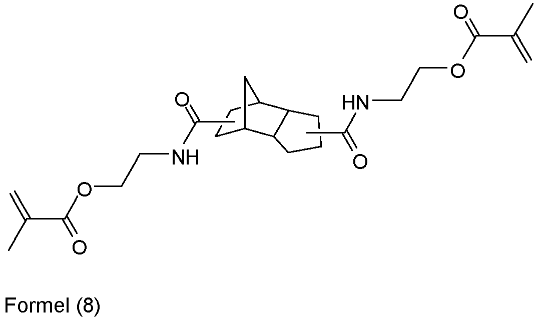

Weitere Umsetzung der beiden reaktionsfähigen Amid-Wasserstoffatome des Amids der Formel (8) mit 2-Isocyanatoethylmethacrylat ergibt den Acylharnstoff der Formel (9).

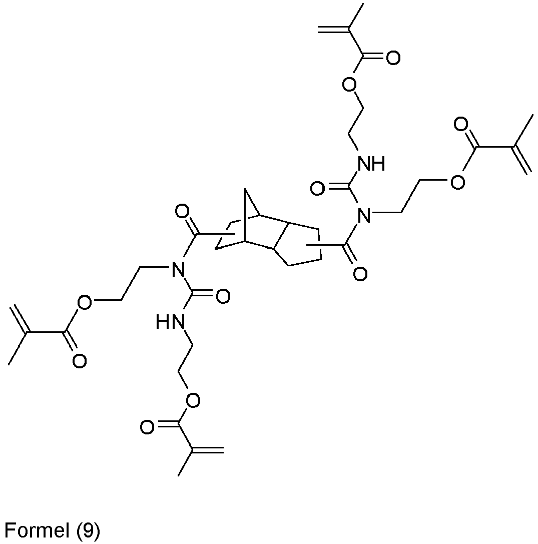

Wird 3(4), 8(9)-Bis(Carbonsäure)tricyclo[5.2.1.0^{2,6}]decan mit Methacryloylisocyanat umgesetzt, ergibt sich das Imid der Formel (10). Die reaktiven Wasserstoffatome am Stickstoff können auch hier weiter in Isocyanatreaktionen umgesetzt werden.

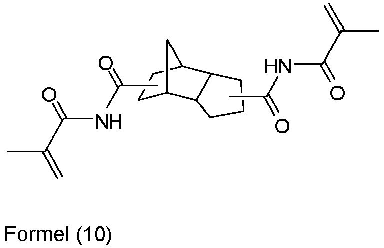

### 3.) Ausgehend von 3(4), 8(9)-Bis(isocyanatomethyl)tricyclo[5.2.1.0^{2,6}]decan

Das 3(4), 8(9)-Bis(isocyanatomethyl)tricyclo[5.2.1.0^{2,6}]decan ist an sich bekannt und zählt zu den herkömmlichen, in industriellen Anwendungen eingesetzten Diisocyanatverbindungen (siehe hierzu die DE 37 03 120 A1 sowie die WO 2009/065873 A2). Die erfindungsgemäße Durchführung der zweiten Umsetzungsstufe der Isocyanat-Alkoholreaktion kann nicht nur ausgehend vom Tricyclodecandiol mit dem Isocyanatoethylmethacrylat, sondern auch ausgehend vom Tricyclodecandiisocyanat und Hydroxyethylmethacrylat eingeleitet werden. Durch stöchiometrische Umsetzung der beiden Reaktanden erhält man das Urethan der Formel (11).

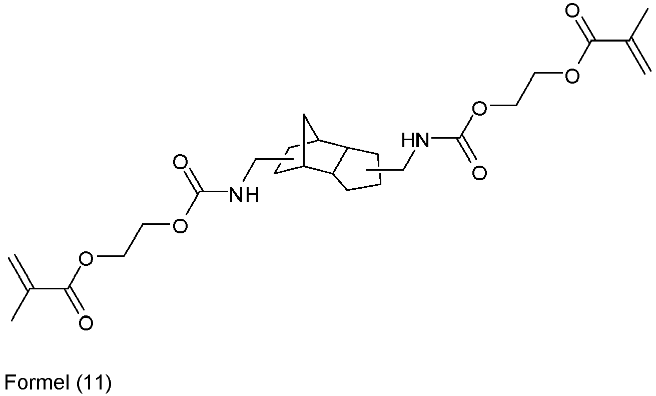

Auch dieses Carbamat (Formel (11)) weist zwei reaktionsfähige Wasserstoffatome am Stickstoff auf, die mit einem Überschuss an Bis(isocyanatomethyl)tricyclo[5.2.1.0^{2,6}]decan zu dem Diisocyanat der Formel (12) weiter umgesetzt werden kann.

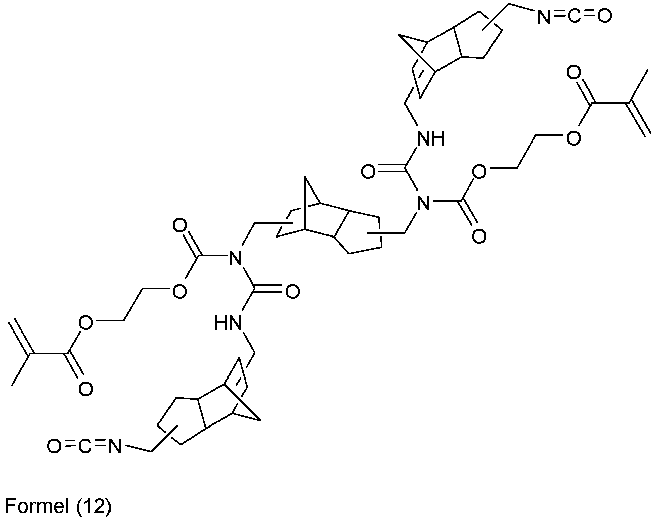

Umsetzung des Allophanat-Diisocyanats (Formel (12)) mit Methacrylsäure liefert die Verbindung der Formel (13).

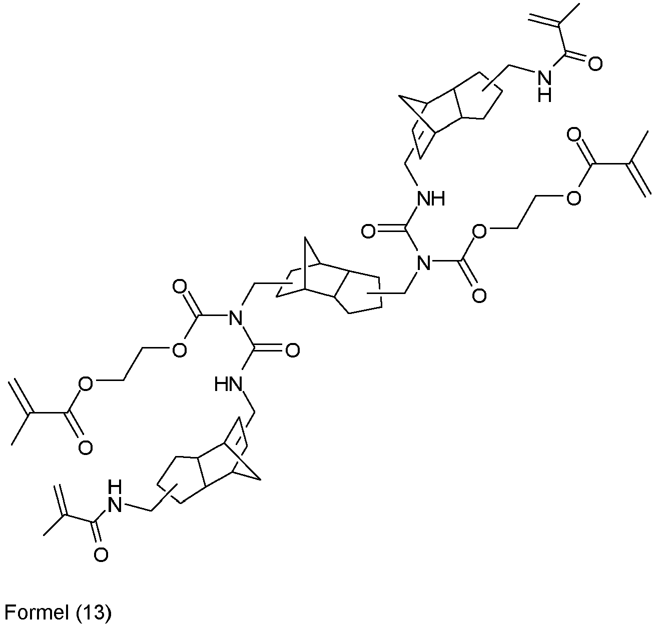

Anstelle von Hydroxyethylmethacrylat können in den oben exemplarisch dargelegten Umsetzungen auch andere Hydroxylverbindungen von (Meth)acrylaten eingesetzt werden, wobei auch Gemische von Acrylaten und Methacrylaten verwendet werden können. So kann - analog dem obigen Beispiel - 3(4), 8(9)-Bis(isocyanatomethyl)-tricyclo[5.2.1.0^{2,6}]decan umgesetzt werden. Dabei bevorzugte Hydroxylverbindungen von (Meth)acrylaten sind die oben explizit genannten.

Diese Verbindungen weisen sowohl (Meth)acrylatgruppen als auch Hydroxygruppen auf. Letztere können mit Isocyanatgruppen in der oben für die Reaktion zwischen Hydroxyethylmethacrylat und 3(4), 8(9)-Bis(isocyanatomethyl)-tricyclo[5.2.1.0^{2,6}]decan beschriebenen Weise reagieren. So lässt sich in einem einzigen Reaktionsschritt ein hoher Funktionalisierungsgrad erreichen.

3(4), 8(9)-Bis(isocyanatomethyl)tricyclo[5.2.1.0^{2,6}]decan kann mit 2-Carbonsäureethylmethacrylat zum entsprechenden Amid der Formel (16) umgesetzt werden.

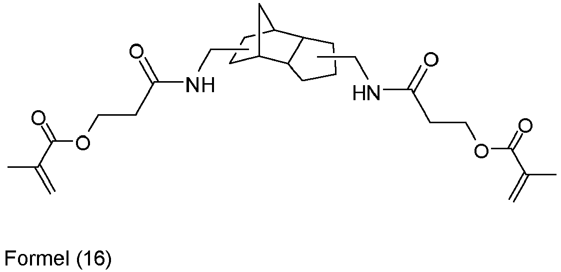

Umsetzung des Amids der Formel (16) mit 2-Isocyanatoethylmethacrylat liefert den Acylharnstoff der Formel (17).

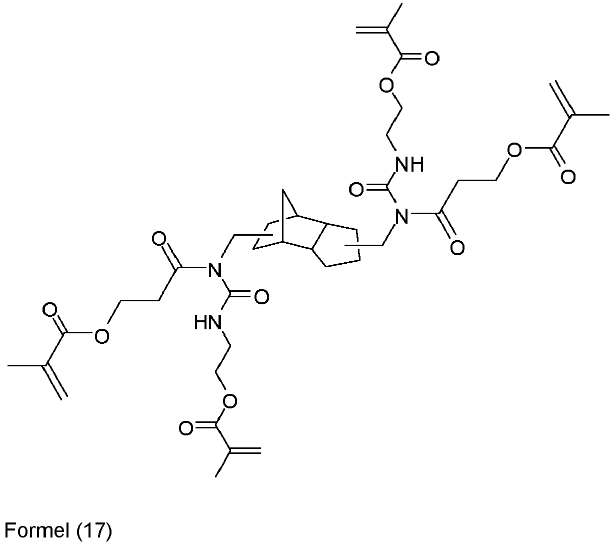

Das Amid der Formel (16) kann auch mit einem Überschuss an 3(4), 8(9)-Bis(isocyanatomethyl)tricyclo[5.2.1.0^{2,6}]decan zum entsprechenden Isocyanat umgesetzt werden, wobei das gebildete Isocyanat mit Hydroxyethylmethacrylat weiter zum vernetzbaren Monomer der Formel (18) weiter umgesetzt wird.

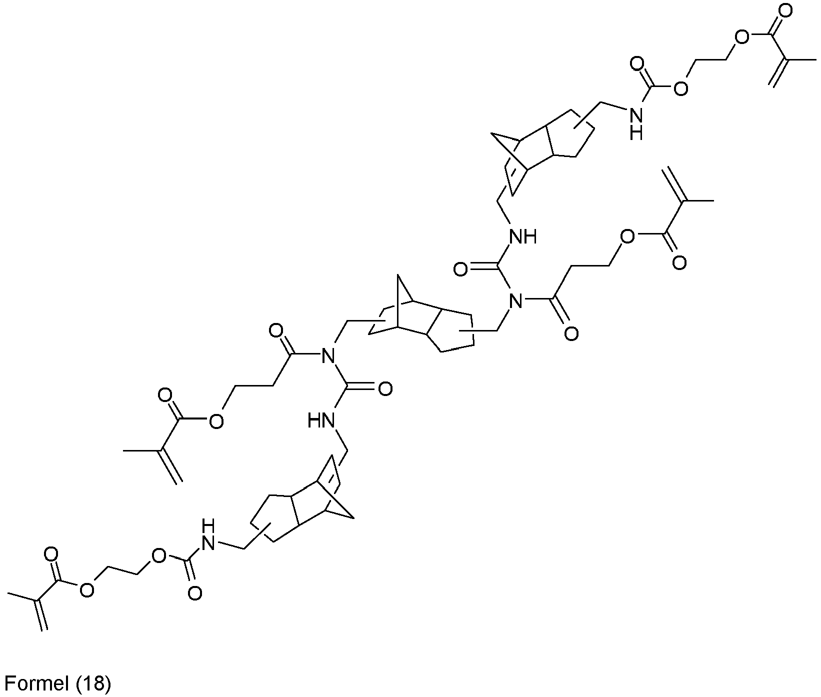

Wird 3(4), 8(9)-Bis(isocyanatomethyl)tricyclo[5.2.1.0^{2,6}]decan mit 2-Methacryloyloxyethylhydrogensuccinat umgesetzt, so erhält man das Amid der Formel (19), das weiter mit 2-Isocyanatoethylmethacrylat zum Acylharnstoff der Formel (20) abreagiert wird.

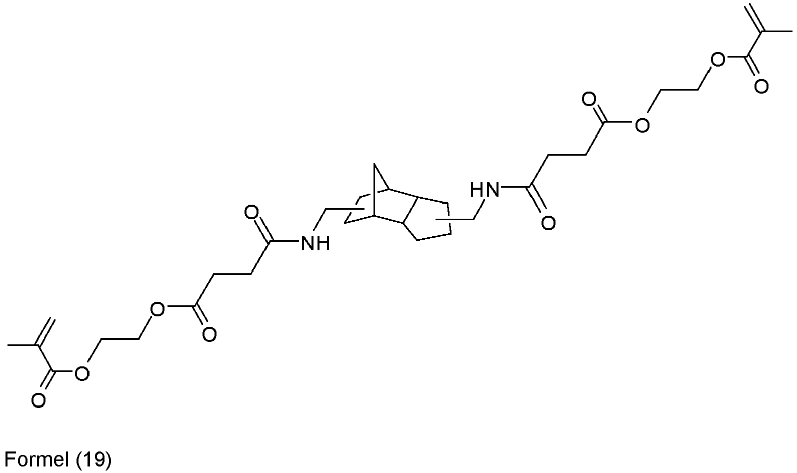

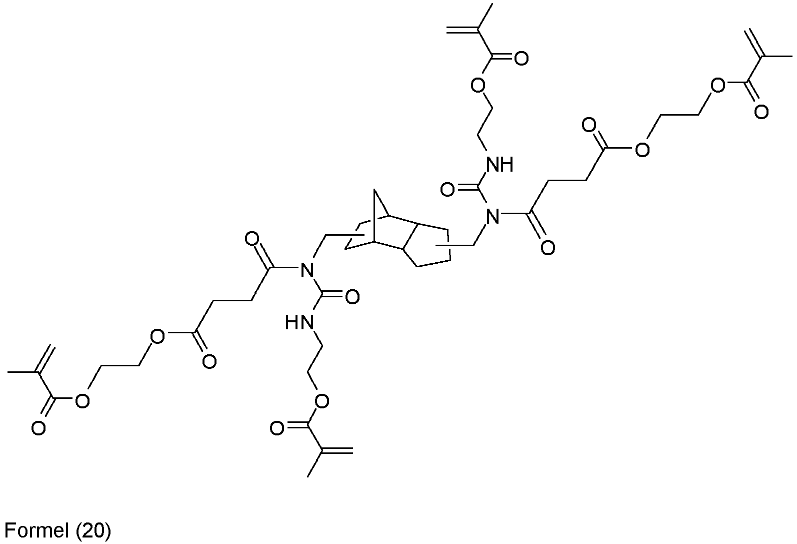

Weitere geeignete Carbonsäuremethacrylate können durch Reaktionen von Di- oder Tetracarbonsäuremono- oder dianhydrid mit geeigneten OH-funktionalisierten, härtbaren Verbindungen wie beispielsweise 2-Hydroxyethylmethacrylat erhalten werden.

### 4.) Ausgehend von 3(4), 8(9)-Bis(Aminomethyl)tricyclo[5.2.1.0^{2,6}]decan

Das 3(4), 8(9)-Bis(Aminomethyl)tricyclo[5.2.1.0^{2,6}]decan ist an sich bekannt oder kann beispielsweise durch Umsetzung der entsprechenden Tosylate mit Ammoniak hergestellt werden. Reaktion des 3(4), 8(9)-Bis(Aminomethyl)tricyclo[5.2.1.0^{2,6}]decans mit 2-Isocyanatoethylmethacrylat ergibt die aus der EP 0 209 700 A2 bekannte Harnstoffverbindung der Formel (26).

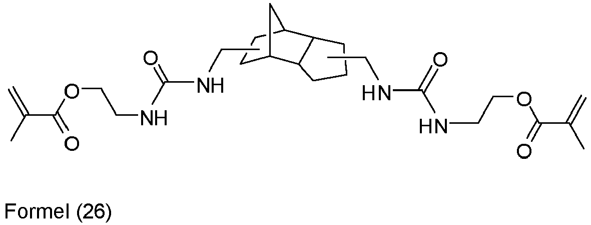

Auch hier befinden sich noch aktive, reaktionsfähige Wasserstoffatome am Stickstoff, die mit einem Überschuss an Isocyanat beispielsweise zum Biuret der Formel (27) reagieren.

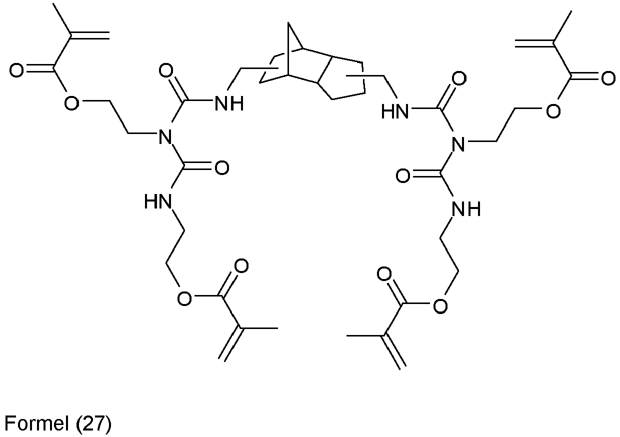

Das 3(4), 8(9)-Bis(Aminomethyl)tricyclo[5.2.1.0^{2,6}]decan kann auch mit Methacryloylisocyanat zum entsprechenden Acylharnstoff umgesetzt werden. Die weitere Reaktion der verbleibenden reaktiven am Stickstoff befindlichen Wasserstoffatome mit Methacryloylisocyanat liefert das Biuret der Formel (28).

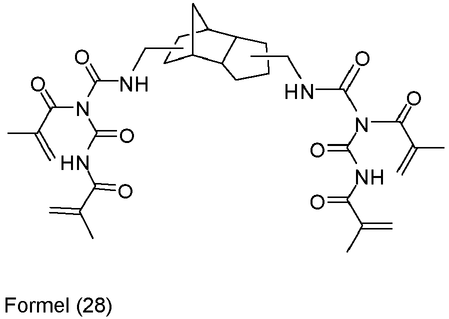

Analog zu den vorstehend beschriebenen Monomeren, welche ein vom Tricyclo[5.2.1.0^{2,6}]decan abgeleitetes polyalicyclisches Strukturelement Q umfassen, können auch Monomere hergestellt werden, welche ein vom Tricyclo[3.3.1.1^{3,7}]decan (Adamantan) abgeleitetes polyalicyclisches Strukturelement Q umfassen. Als Beispiele seien folgende Reaktionsprodukte gezeigt:

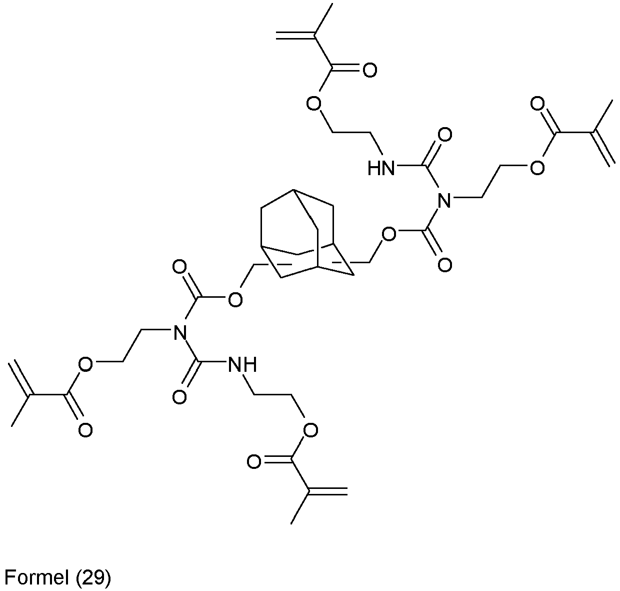

Die Umsetzung der Verbindung der Formel (11) mit Diisocyanatoadamantan [(Bis(isocyanatomethyl)tricyclo[3.3.1.1^{3,7}]decan] liefert ein erfindungsgemäßes Monomer, dessen Molekül zwei voneinander verschiedene polyalicyclische Strukturelemente Q umfasst, wie die nachfolgende Strukturformel der Verbindung der Formel (69) zeigt.

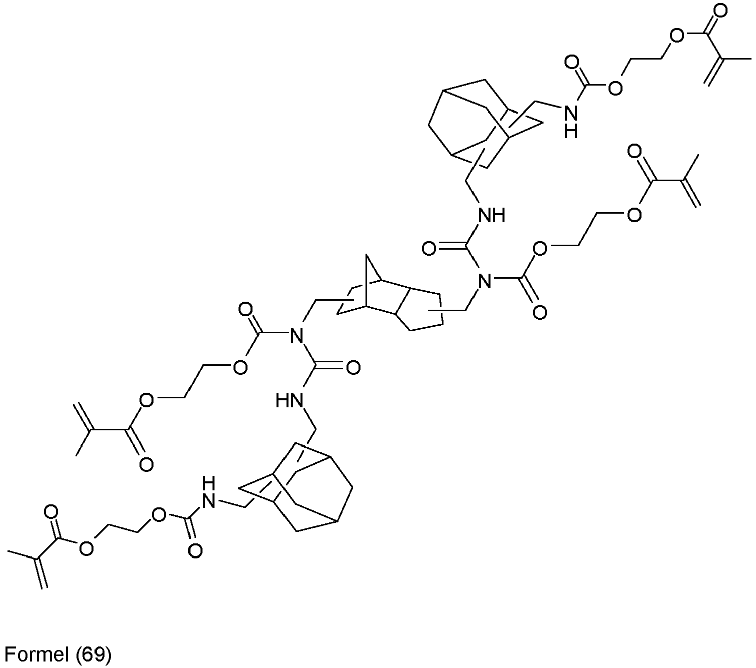

### Komponente (b2): ein, zwei oder mehr weitere radikalisch polymerisierbare Monomere aus der Gruppe bestehend aus Acrylaten und Methacrylaten, vorzugsweise der Gruppe der Methacrylate

Den zweiten, nicht zu Komponente (b1) zählbaren Bestandteil der matrixbildenden Monomerenmischung bilden radikalisch polymerisierbare Monomere ausgewählt aus der Gruppe bestehend aus Acrylaten und Methacrylaten. Ihre Funktion innerhalb des erfindungsgemäßen Kompositmaterials besteht im Wesentlichen im Einstellen der Viskosität.

Erfindungsgemäß sind die Methacrylsäureester bzw. -diester wegen ihrer höheren biologischen Verträglichkeit gegenüber den entsprechenden Acrylsäureestern bzw. -diestern bevorzugt.

Die radikalisch polymerisierbaren Monomere der Komponente (b2) weisen vorzugsweise mindestens zwei ethylenische Gruppen auf.

In der Patentliteratur ist eine Vielzahl von Diacrylat- und Dimethacrylat-Monomeren genannt (beispielsweise auch in der DE 39 41 629 A1 insbesondere die Offenbarung im Bereich von Spalte 6, Zeile 15 bis Spalte 8, Zeile 10), die sich zum Einsatz in einem erfindungsgemäßem Kompositmaterial eignen.

In einem bevorzugten erfindungsgemäßen Kompositmaterial enthält Komponente (b2) ein oder mehrere Dimethacrylat-Monomere gewählt aus der Gruppe bestehend aus Ethylenglykoldimethacrylat (EGDMA), 1,6-Hexandioldimethacrylat (HEDMA), Triethylenglykoldimethacrylat (TEDMA), 1,12-Dodecandioldimethacrylat (DODMA), ethoxyliertes Bisphenol-A-dimethacrylat, Polyethylenglykoldimethacrylat (PEGDMA), 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat und Glycerindimethacrylat.

Bisphenol-A-Glycidyl-Methacrylat (Bis-GMA) kann zwar eingesetzt werden.Vorzugsweise enthält ein erfindungsgemäßes Kompositmaterial, insbesondere ein erfindungsgemäßes dentales Kompositmaterial, jedoch nicht die Verbindung Bis-GMA. Vorzugsweise ist ein erfindungsgemäßes Kompositmaterial, insbesondere ein erfindungsgemäßes dentales Kompositmaterial, frei von sämtlichen Verbindungen mit einem Bisphenol-A-Strukturelement.

Die radikalisch polymerisierbaren Monomere der Komponente (b2), die somit nicht zu Komponente (b1) zählen, können auch Hydroxylverbindungen sein. Dabei können alle in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-bis[4-[3-methacryloyloxy-2-hydroxypropoxy]phenyl]propan. Als weiterer Bestandteil können auch lichthärtbare Acrylat- oder Methacrylat-Monomere auf Basis von Polysiloxanen verwendet werden, wie sie beispielsweise in der DE 199 03 177 oder in der DE 44 16 857 beschrieben sind.

Ein erfindungsgemäßes Kompositmaterial, insbesondere ein erfindungsgemäßes dentales Kompositmaterial, kann ferner in Komponente (b2) ein oder mehrere säuregruppenhaltige Acrylate- und/oder Methacrylat-Monomere enthalten. Solche säuregruppenhaltigen Monomere können vorzugsweise eine Carbonsäure-, eine Phosphorsäure-, eine Phosphonsäure-, eine Sulfonsäure- und/oder eine Thiophosphorsäurefunktion aufweisen. Das Monomer kann, eine oder eine Vielzahl von Säurefunktionen in einem Molekül enthalten.

Geeignete eine Phosphorsäuregruppe enthaltende Monomere sind beispielsweise 2-(Meth)acryloyloxyethyldihydrogenphosphat, Bis[2-(meth)acryloyloxyethyl]hydrogen-phosphat, 2-(Meth)acryloyloxyethylphenylhydrogenphosphat, 6-(Meth)-acryloyloxyhexyl-dihydrogenphosphat, 10-(Meth)acryloyloxydecyldihydrogenphosphat (MDP), 6-(Meth)-acryloyloxyhexylphenylhydrogenphosphat, 10-(Meth)acryloyloxydecyldihydrogenphosphat, 1,3-Di-(meth)acryloyloxypropan-2-dihydrogenphosphat, 1,3-Di-(meth)acryloyl-oxypropan-2-phenylhydrogenphosphat und Bis[5-(2-(meth)acryloyloxyethoxycarbonyl)-heptyl]hydrogenphosphat.

Geeignete eine Carbonsäuregruppe enthaltende Monomere sind beispielsweise 4-(Meth)acryloxyethyltrimellithsäure (4-MET), 4-(Meth)acryloxyethyltrimellithsäureanhydrid (4-META), 4-(Meth)acryloxydecyltrimellithsäure, 4-(Meth)acryloxydecyltrimellithsäure-anhydrid, 11-(Meth)acryloyloxy-1,1-undecandicarbonsäure, 1,4-Di(meth)acryloyloxypyromellithsäure, 2-(Meth)acryloyloxyethylmaleinsäure, 2-(Meth)acryloyloxyethylphthalsäure und 2-(Meth)acryloyloxyethylhexahydrophthalsäure.

Weitere geeignete säuregruppentragende Monomere sind beispielsweise in der EP 0 980 682 A1 (insbesondere Absätze [0059] bis [0065]) oder der EP 0 948 955 A1 (insbesondere Absätze [0031] bis [0034]) genannt.

Ferner können auch die Phosphorsäureester mit Glycerindimethacrylat oder mit Hydroxyethylmethacrylat oder mit Hydroxypropylmethacrylat verwendet werden.

Die genannten Monomere können einzeln oder in Gemischen eingesetzt werden.

### Bestandteil (c): Initiatoren und/oder Katalysatoren

Ein erfindungsgemäßes Kompositmaterial ist vorzugsweise lichthärtbar und/oder chemisch härtbar. Bevorzugt ist ein erfindungsgemäßes Kompositmaterial, wobei Bestandteil (c) einen oder mehrere Lichthärtungsinitiatoren und/oder einen oder mehrere Initiatoren zur chemischen Aushärtung umfasst oder aus diesen besteht.

Bevorzugte erfindungsgemäße Kompositmaterialien sind lichthärtbar (photohärtbar) und umfassen Lichthärtungsinitiatoren. Beispiele für einen Lichthärtungsinitiator schließen Substanzen ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Initiatoren sowie Initiatorkombinationen sind in der DE 601 16 142 beschrieben.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung eines erfindungsgemäßen Kompositmaterials, insbesondere ein erfindungsgemäßes dentalen Kompositmaterials, bewirken können.

Das Absorptionsmaximum von Campherchinon (CC) liegt bei ca. 470 nm und somit im Bereich des blauen Lichts. Campherchinon (CC) zählt zu den PI₂-Initiatoren und wird regelmäßig zusammen mit einem Coinitiator eingesetzt.

Vorzugsweise enthält ein erfindungsgemäßes Kompositmaterial die Kombination eines alpha-Diketons und eines aromatischen tertiären Amins, bevorzugt ist die Kombination von Campherchinon (CC) und Ethyl-*p*-*N*,*N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in einem erfindungsgemäßen Kompositmaterial wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha-Diketon/Amin" als Photopolymerisationsinitiatorsystem in einem erfindungsgemäßen Kompositmaterial.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

Dem Fachmann sind diverse Initiatoren für eine chemische Aushärtung bekannt. Es sei insoweit exemplarisch auf die EP 1 720 506 verwiesen.

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht- und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Geeignete Malonylsulfamide sind in der EP 0 059 451 beschrieben welche im Wege der Verweisung Bestandteil der vorliegenden Anmeldung ist. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

### Bestandteil (d): optionale weitere Additive

Ein erfindungsgemäßes Kompositmaterial umfasst in manchen Fällen ein oder mehrere weitere Additive.

Diese Additive können verschiedene Funktionen haben. Übliche Additive für den Einsatz in dentalen Kompositmaterialien sind dem Fachmann bekannt, je nach gewünschter Funktion wird er das oder die geeigneten Additive auswählen. Beispielhaft sind im folgenden typische Additive und ihre Funktionen beschrieben.

Lichthärtbare dentale Kompositmaterialien, wie sie erfindungsgemäß bevorzugt sind, enthalten vorzugsweise einen oder mehrere Inhibitoren, auch Stabilisatoren genannt. Diese werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren dentalen Zusammensetzung. Gängige Inhibitoren sind Phenolderivate wie Hydrochinonmonomethylether (HQME) oder 2,6-Di-tert.butyl-4-methylphenol (BHT). Weitere Inhibitoren wie und tert.-Butylhydroxyanisol (BHA), 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben welche im Wege der Verweisung Bestandteil der vorliegenden Anmeldung sind. Alternative Inhibitoren sind in der DE 101 19 831 A1 oder in der EP 1 563 821 A1 angegeben.

Ein erfindungsgemäß bevorzugtes dentales Kompositmaterial umfasst somit als Additiv ein oder mehrere Polymerisationsinhibitoren zur Erhöhung der Lagerstabilität des Kompositmaterials, vorzugsweise ausgewählt aus der Gruppe bestehend aus Hydrochinonmonomethylether (HQME), Phenolen, dabei vorzugsweise 2,6-Di-tert.butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.

Ein erfindungsgemäßes dentales Kompositmaterial kann als Additiv eine oder mehrere fluoridabgebende Substanzen, dabei vorzugsweise Natriumfluorid und/oder Aminfluoride umfassen.

UV-Absorber, die beispielsweise durch ihre konjugierten Doppelbindungssytemen und aromatischen Ringe befähigt sind, UV Strahlung zu absorbieren, sind in manchen Fällen Bestandteil eines erfindungsgemäßen (vorzugsweise dentalen) Kompositmaterials. Beispiele an UV-Absorbern sind 2-Hydroxy-4-methoxybenzophenon, Salicylsäurephenylester 3-(2'-Hydroxy-5'-methylphenyl)-benzotriazol oder Diethyl-2,5-dihydroxy-terephthalat.

Da die Zähne möglichst naturgetreu wiederherzustellen sind, ist es notwendig, erfindungsgemäße dentale Kompositmaterialien in den unterschiedlichsten Farbtönen bereitzustellen. Man benutzt zu diesem Zweck in der Regel anorganische Farbstoffe sowie organische Pigmente in sehr geringen Mengen, die somit in bevorzugten Ausgestaltungen als Additiv eingesetzt werden.

Weitere optionale Additive sind Aromastoffe.

Die vorliegende Erfindung betrifft auch ein erfindungsgemäßes Kompositmaterial als Dentalmaterial bzw. zur Verwendung als Dentalmaterial, insbesondere als restauratives Kompositmaterial, insbesondere für als Füllungs-, Unterfüllungs-, Befestigungs- und/oder Stumpfaufbaumaterial, als provisorisches Kronen- und/oder Brückenmaterial, als Prothesen- und/oder Unterfütterungsmaterial oder als Flow-Material.

Die vorliegende Erfindung betrifft auch ein erfindungsgemäßes Kompositmaterial, insbesondere in einer der als bevorzugt bzw. besonders bevorzugt gekennzeichneten Ausgestaltungen, zur Anwendung in einem therapeutischen dentalen Verfahren als Dentalmaterial, insbesondere zum Füllen, zum Unterfüllen, zum Befestigen von Zähnen und/oder zum Aufbauen von Stümpfen, oder zur Herstellung provisorischer Kronen und/oder Brücken.

Die vorliegende Erfindung betrifft auch die Verwendung eines erfindungsgemäßen Kompositmaterials zur Herstellung und/oder Unterfütterung von Prothesen oder zur Herstellung eines (vorzugsweise dentalen) Flow-Materials.

Die vorliegende Erfindung betrifft auch ein Erzeugnis, erhältlich durch Härten eines erfindungsgemäßen Kompositmaterials, insbesondere in einer der als bevorzugt bzw. besonders bevorzugt gekennzeichneten Ausgestaltungen.

Die vorliegende Erfindung betrifft auch ein Verfahren zum Behandeln einer Zahnerkrankung, dadurch gekennzeichnet, dass ein erfindungsgemäßes Kompositmaterial, vorzugsweise in einer der als bevorzugt angegebenen Ausgestaltungen, als Füllungs-, Unterfüllungs-, Befestigungs- und/oder Stumpfaufbaumaterial, als provisorisches Kronen- und/oder Brückenmaterial, als Prothesen- und/oder Unterfütterungsmaterial oder als Flow-Material eingesetzt wird.

Die Erfindung betrifft zudem ein Verfahren zur Herstellung eines erfindungsgemäßen Kompositmaterials, insbesondere eines erfindungsgemäßen dentalen Kompositmaterials, mit folgendem Schritt:
- Mischen der Bestandteile (a), (b) und (c) und gegebenenfalls (d).

Anhand der folgenden Beispiele wird die Erfindung weiter erläutert.

### Beispiele

Anhand der folgenden Beispiele wird die Erfindung weiter erläutert. Sofern nicht anders angegeben, beziehen sich alle Angaben auf das Gewicht. Es werden dabei folgende branchenübliche Abkürzungen verwendet:
UDMA = Urethandimethacrylat (7,7,9-Trimethyl-4,13-dioxo-5,12-diazahexadecan-1,16-dioxydimethacrylat)
TEDMA = Triethylenglykoldimethacrylat
CC = Campherchinon
DABE = Ethyl-*p*-*N*,*N*-dimethylaminobenzoat
BHT = 2,6-Di-tert.butyl-4-methylphenol
PPD = Phenylpropandion

### Herstellung von Kompositmaterialien

Erfindungsgemäße Kompositmaterialien (Beispiele 1 bis 10) sowie ein nicht erfindungsgemäßes Vergleichsmaterial (Beispiel 11) wurden wie folgt hergestellt:
Die Monomere (b1) und (b2), sowie Initiatoren (c) und Additive (d) werden zunächst in einem Kunststoffbehälter mittels eines KPG-Rührers homogenisiert. Anschließend werden die Füllstoffe (a1), (a2) und ggf. (a3) zugegeben und durch inniges Vermischen mit einem Doppelplaneten-Mischer wird eine homogene Paste hergestellt.

Die Zusammensetzungen der erfindungsgemäßen Kompositmaterialien (Beispiele 1-10) sind in den Tabellen 1 und 2 angegeben, die Zusammensetzung des nicht erfindungsgemäßen Kompositmaterials (Vergleichsbeispiel 11) in Tabelle 3. Die Zusammensetzung der Komponenten (c) und (d), insbesondere die Auswahl des oder der UV-Absorber, falls solche eingesetzt werden, hat keinen signifikanten Einfluss auf die Eigenschaften der Kompositmaterialien.

### Messmethoden:

### Bestimmung der mittleren Partikelgröße:

Die mittleren Partikelgrößen d₅₀ der erfindungsgemäß einzusetzenden Nanopartikel der Komponente (a1) und Mikropartikel der Komponente (a2) der Füllstoffkomponente des dentalen Kompositmaterials wurden mit einem Partikelgrößenmessgerät Beckman Coulter LS 13320 bestimmt. Dazu wurden jeweils Proben mit einer Masse von 50 mg zunächst 5 min in 2 mL Isopropanol suspendiert und anschließend vermessen.

### Bestimmung der Polymerisationsschrumpfung:

Die Polymerisations-Volumenschrumpfung (Polymerisationsschrumpf) wurde gemäß der Bonded-Disc-Methode (Dental Materials 2004, 20, 88-95) bestimmt. 100 mg Material wurden für einen Zeitraum von 40 Sekunden (Softstart) belichtet (Celalux2, VOCO GmbH Cuxhaven) und die Polymerisationsschrumpfung über einen Zeitraum von 1800 Sekunden gemessen.

### Bestimmung der Abrasion (ACTA)

Die Drei-Medien Acta Abrasion wurde gemäß J. Dent. Suppl. 1, 1994, 22, 21-27 nach 200000 Zyklen ermittelt.

### Bestimmung der Vickers-Mikrohärte

Die Mikrohärte nach Vickers wurde mit einem Messgerät MTH 4 der Firma Anton Paar (Graz, Österreich) an 2x2 mm großen Prüfkörpern gemessen, die zuvor mit Schleifpapier der Körnung 4000 beschliffen wurden. Dabei betrug die Kraft 1 N, die Eindringgeschwindigkeit 0,2 N/s und die Haltezeit 5 s.

### Ergebnisse

Die Ergebnisse der Messungen der Polymerisationsschrumpfung, der Vickers-Mikrohärte und der Abrasion sind für die erfindungsgemäßen Kompositmaterialien der Beispiele 1-10 in den Tabellen 1 und 2 und für das nicht erfindungsgemäßes Kompositmaterial des Vergleichsbeispiels 11 in der Tabelle 3 angegeben.

Alle erfindungsgemäßen Kompositmaterialien (Beispiele 1-10) zeichnen sich im ausgehärteten Zustand durch eine geringe Polymerisationsschrumpfung (weniger als 1,7 Vol-%, besonders bevorzugt weniger als 1,6 Vol.-%), eine geringe Abrasion (weniger als 35 µm, besonders bevorzugt weniger als 30 µm, bestimmt nach der ACTA-Methode) und eine hohe Vickers-Mikrohärte (vorzugsweise 140 oder mehr) aus.

In dem nicht erfindungsgemäßen Kompositmaterial des Vergleichsbeispiels 11 beträgt der Anteil der nicht agglomerierten, organisch oberflächenmodifizierten Nanopartikel mit einer mittleren Partikelgröße kleiner 200 nm nur 4,32 Gew.-%. Dies führt zu einer Erhöhung der Abrasion (37,2 µm), einer Verminderung der Vickers-Mikrohärte (123,7) und einer Vergrößerung der Polymerisationsschrumpfung (2,13 %), so dass dieses Kompositmaterial im Gegensatz zu den erfindungsgemäßen Kompositmaterialien für den Einsatz im dentalen Bereich weniger geeignet ist.

Die Zusammensetzungen der Mischungen (in Gewichtsteilen) sowie die Ergebnisse der Messungen sind in den nachfolgenden Tabellen aufgelistet.

### Eingesetzte TCD-Monomere:

Bei dem eingesetzten TCD-Monomer der Komponente (b1) handelte es sich in den erfindungsgemäßen Beispielen 1 - 4 und 6 - 9 jeweils um Bis(methacryloyloxymethyl)tricyclo[5.2.1.0^{2,6}]decan.

In dem erfindungsgemäßen Beispiel 5 wurde als TCD-Monomer, ebenso wie in Vergleichsbeispiel 11, Bis(acryloyloxymethyl)tricyclo [5.2.1.0^{2,6}]decan eingesetzt.

In dem erfindungsgemäßen Beispiel 10 wurde als TCD-Monomer die Verbindung der Formel (2) eingesetzt.

**Tabelle 1**

| Beispiel Nr. | 1 | 2 | 3 | 4 | 5 |
|---|---|---|---|---|---|
| (a) Füllstoffe | | | | | |
| (a1) SiO₂-Nanopartikel (d₅₀ = 50 nm, silanisiert) | 22,82 | 22,81 | 22,81 | 23,44 | 22,62 |
| (a2) Mikropartikel, erste Fraktion (d₅₀ = 1,5 µm, silanisiertes Dentalglas) | 52,94 | 54,99 | 41,88 | 52,16 | 52,44 |
| (a2) Mikropartikel, zweite Fraktion (d₅₀ = 0,7 µm, silanisiertes Dentalglas) | 14,07 | 12,04 | 25,15 | 13,95 | 14,86 |
| (b) Monomere | | | | | |
| (b1) TCD-Monomer | 7,67 | 5,75 | 4,79 | 1,97 | 7,60 |
| (b2) UDMA | 1,92 | 3,83 | 4,79 | 7,88 | 1,90 |
| (b2) TEDMA | 0,43 | 0,43 | 0,43 | 0,45 | 0,43 |
| (c) CC | 0,05 | 0,05 | 0,05 | 0,05 | 0,05 |
| (c) DABE | 0,075 | 0,075 | 0,075 | 0,075 | 0,075 |
| (d) BHT | 0,015 | 0,015 | 0,015 | 0,015 | 0,014 |
| (d) UV-Absorber | 0,072 | 0,072 | 0,072 | 0,074 | 0,071 |
| Massenverhältnis (b1)/(b2) | 3,26 | 1,35 | 0,92 | 0,24 | 3,26 |
| Vickers-Mikrohärte [MHV] | 157,5 | 147,2 | 156,3 | 168,7 | 160,3 |
| ACTA [µm] | 34,6 | 24,0 | 23,2 | 34,0 | 18,4 |
| Polymerisationsschrumpfung [%] | 1,48 | 1,56 | 1,58 | 1,51 | 1,54 |
| Quotient (MHV/(ACTA*Polymerisationsschrumpfung)) [100/µm] | 3,1 | 3,9 | 4,3 | 3,3 | 5,7 |

**Tabelle 2**

| Beispiel Nr. | 6 | 7 | 8 | 9 | 10 |
|---|---|---|---|---|---|
| (a) Füllstoffe | | | | | |
| (a1) SiO₂-Nanopartikel (d₅₀ = 50 nm, silanisiert) | 22,79 | 22,51 | 22,52 | 24,19 | 22,62 |
| (a2) Mikropartikel, erste Fraktion (d₅₀ = 1,5 µm, silanisiertes Dentalglas) | 55,02 | 55,05 | 49,44 | 50,60 | 52,44 |
| (a2) Mikropartikel, zweite Fraktion (d₅₀ = 0,7 µm, silanisiertes Dentalglas) | 12,03 | 12,52 | 17,97 | 14,41 | 14,86 |
| (b) Monomere | | | | | |
| (b1) TCD-Monomer | 3,59 | 6,31 | 4,73 | 0,51 | 7,60 |
| (b2) UDMA | 5,99 | 3,15 | 4,76 | 9,66 | 1,90 |
| (b2) TEDMA | 0,43 | 0,33 | 0,43 | 0,46 | 0,43 |
| (c) CC | 0,05 | 0,04 | 0,05 | 0,06 | 0,05 |
| (c) DABE | 0,075 | 0,075 | 0,075 | 0,09 | 0,075 |
| (d) BHT | 0,015 | 0,014 | 0,014 | 0,016 | 0,014 |
| (d) UV-Absorber | 0,072 | 0,07 | 0,071 | 0,076 | 0,071 |
| Massenverhältnis (b1)/(b2) | 0,56 | 1,81 | 0,91 | 0,05 | 3,26 |
| Vickers-Mikrohärte [MHV] | 153,8 | 142,2 | 140,4 | 139,8 | 162,5 |
| ACTA [µm] | 25,0 | 29,8 | 30,2 | 34,6 | 22,6 |
| Polymerisationsschrumpfung [%] | 1,46 | 1,57 | 1,52 | 1,55 | 1,49 |
| Quotient (MHV/(ACTA*Polymerisationsschrumpfung)) [100/µm] | 4,2 | 3,0 | 3,1 | 2,6 | 4,83 |

Das nachfolgende Vergleichsbeispiel (Beispiel 11) entspricht stellvertretend Kompositmaterialien des Stands der Technik. Dieses Vergleichsbeispiel basiert auf Rezeptur 349 der EP 2 016 931 A2.

**Tabelle 3**

| Beispiel Nr. | 11 (Vergleichsbeispiel) |
|---|---|
| (a) Füllstoffe | |
| (a1) SiO₂-Nanopartikel (d₅₀ = 50 nm, silanisiert) | 4,32 |
| (a2) Dentalglas-Mikropartikel, erste Fraktion (d₅₀ = 5,0 µm, silanisiert) | 39,38 |
| (a2) Dentalglas-Mikropartikel, zweite Fraktion (d₅₀ = 0,85 µm, silanisiert) | 39,38 |
| (b) Monomere | |
| (b1) TCD-di-HEA | 11,34 |
| (b2) UDMA | 2,7 |
| (b2) TEDMA | 2,88 |
| (c) Initiatoren (CC, DABE, PPD) | 0,1 |
| (d) Additive (UV-Absorber, TEMPO, BHT, Pigmente) | 0,26 |
| Massenverhältnis (b1)/(b2) | 2,03 |
| Vickers-Mikrohärte [MHV] | 123,7 |
| ACTA [µm] | 37,2 |
| Polymerisationsschrumpfung [%] | 2,13 |
| Verhältnis (MHV/(ACTA*Polymerisationsschrumpfung)) [100/µm] | 1,6 |

### Synthese der Verbindung der Formel (2)

0,95 g (4,84 mmol) 3(4), 8(9)-Bis(hydroxymethyl)tricyclo[5.2.1.0^{2,6}]decan wurden in 10 mL Toluol gelöst und mit 0,04 g BHT und 0,103 g der Katalysatorlösung (0,50 g Dibutylzinn(ll)dilaurat gelöst in 9,50 g Toluol) versetzt. Unter Rühren wurden 3,00 g (19,34 mmol, 4 Äquivalente) Isocyanatoethylmethacrylat, gelöst in 10 mL Toluol, zugetropft. Nach beendeter Zugabe wurde der Tropftrichter durch einen Rückflusskühler ausgetauscht und das Reaktionsgemisch auf 120 °C erwärmt und der Fortschritt der Reaktion mittels IR-Spektroskopie verfolgt. Nach 72 Stunden wurden weitere 0,102 g Katalysatorlösung zugesetzt und weiter erhitzt, bis keine Isocyanatbande mehr detektiert wurde. Das Lösungsmittel wurde am Rotationsverdampfer entfernt. Das Allophanat der Formel (2) wurde in einer Ausbeute von 3,83 g (4,69 mmol, 97%) als leicht gelbliches Öl erhalten.

Die Beispiele 1 bis 9 wurden unter Austausch des dort jeweils eingesetzten TCD-Monomeren gegen die Verbindung der Formel (2) wiederholt; diese weiteren Beispiele werden als Beispiele S1 bis S9 bezeichnet. Es wurden sämtliche in den Beispielen 1 bis 9 bestimmten Parameter auch für die Beispiele S1 bis S9 bestimmt. Die bestimmten Parameterwerte für die Beispiele S1 bis S9 sind ähnlich zu denen aus den Beispielen 1 bis 9 und übertreffen diese teilweise. Dies zeigt neben Beispiel 10, dass die Verbindung der Formel (2), welche repräsentativ für die erfindungsgemäßen Verbindungen steht, hervorragend für den Einsatz in erfindungsgemäßen Kompositmaterialien geeignet ist.

## Patentansprüche

1. Kompositmaterial bestehend aus oder umfassend:
(a) eine Gesamtmenge an Füllstoffen im Bereich von> 75 bis 95 Gew.-%, bezogen auf die Gesamtmasse des Kompositmaterials, wobei die Gesamtmenge an Füllstoffen eine Mischung von Füllstoffen ist umfassend
(a1) eine Gesamtmenge im Bereich von > 12 bis 30 Gew.-% an nicht agglomerierten, organisch oberflächenmodifizierten Nanopartikeln mit einer mittleren Partikelgröße kleiner 200 nm
und
(a2) eine Gesamtmenge im Bereich von 45 bis < 83 Gew.-% an Mikropartikeln mit einer mittleren Partikelgröße im Bereich von 0,4 µm bis 10 µm sowie
(a3) gegebenenfalls weitere Füllstoffe,
wobei die Gewichtsprozentangaben für die Komponenten (a1) und (a2) auf die Gesamtmasse des Kompositmaterials bezogen sind,
(b) eine Gesamtmenge an polymerisierbaren Monomeren im Bereich von 3 bis < 25 Gew.-%, bezogen auf die Gesamtmasse des Kompositmaterials, wobei die Gesamtmenge an polymerisierbaren Monomeren eine Monomerenmischung ist umfassend
(b1) ein, zwei oder mehr Monomere ausgewählt aus der Gruppe bestehend aus Verbindungen (Monomere) der Struktur Q(YₓZₑ)_{b}, wobei gilt:
- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten YₓZₑ substituierten Wasserstoffatome dieses polyalicyclischen Strukturelements Q durch Alkylgruppen, Alkoxygruppen, Halogenatome oder Trifluormethylgruppen substituiert sind,
- b ist eine natürliche Zahl ausgewählt aus der Gruppe der natürlichen Zahlen 1, 2, 3 und 4,
- jedes Z bedeutet ein Strukturelement, das unabhängig von etwaigen weiteren Strukturelementen Z ausgewählt ist aus der Gruppe bestehend aus
-O-(C=O)-CH=CH₂, -O-(C=O)-C(CH₃)=CH₂,
-(C=O)-CH=CH₂, -(C=O)-C(CH₃)=CH₂,
-CH=CH₂, -C(CH₃)=CH₂ und -O-CH=CH₂,
- jeder Index e ist eine natürliche Zahl, die unabhängig von etwaigen weiteren Indizes e ausgewählt ist aus der Gruppe der natürlichen Zahlen 1, 2, 3 und 4,
- jeder Index x bedeutet unabhängig von etwaigen weiteren Indizes x 0 oder 1,
- jedes Y bedeutet in der Struktur Q(YₓZₑ)_{b} bei x = 1 ein Strukturelement, welches das polyalicyclische Strukturelement Q mit e Strukturelementen Z verbindet, wobei jedes Y unabhängig von etwaigen weiteren Strukturelementen Y gewählt ist,
(b2) ein, zwei oder mehr weitere radikalisch polymerisierbare Monomere aus der Gruppe bestehend aus Acrylaten und Methacrylaten, vorzugsweise der Gruppe der Methacrylate,
wobei das oder die weiteren radikalisch polymerisierbaren Monomere keine Verbindungen (Monomere) der vorstehend definierten Struktur Q(YₓZₑ)_{b} sind und
wobei das Verhältnis der Masse der Komponente (b1) zur Masse der Komponente (b2) im Bereich von 1 : 20 bis 4 : 1 liegt,
(c) ein oder mehrere Initiatoren und/oder Katalysatoren, sowie
(d) gegebenenfalls ein oder mehrere sonstige Additive.

2. Kompositmaterial nach Anspruch 1, wobei Komponente (a2) zwei oder mehr Mikropartikelfraktionen enthält, wobei eine oder mehrere erste Mikropartikelfraktionen jeweils eine mittlere Partikelgröße im Bereich von 1 µm bis 10 µm, vorzugsweise 1 µm bis 5 µm, besitzen und wobei eine oder mehrere zweite Mikropartikelfraktionen jeweils eine mittlere Partikelgröße im Bereich von > 0,4 µm bis < 1 µm, vorzugsweise 0,5 µm bis 0,8 µm, besitzen.

3. Kompositmaterial nach Anspruch 2, wobei
das Verhältnis der Gesamtmasse der ersten Mikropartikelfraktionen zur Gesamtmasse der zweiten Mikropartikelfraktionen im Bereich von 1 : 1 bis 10 : 1, vorzugsweise im Bereich von 1,5 : 1 bis 5 : 1 liegt,
und/oder
das Verhältnis der mittleren Korngröße der oder einer ersten Mikropartikelfraktion zur mittleren Korngröße der oder einer zweiten Mikropartikelfraktion der Komponente (a2) im Bereich von 1,5 : 1 bis 10 : 1, vorzugsweise im Bereich von 2 : 1 bis 5 : 1, liegt.

4. Kompositmaterial nach einem der vorangehenden Ansprüche, wobei zumindest ein Teil der Mikropartikel der Komponente (a2) organisch oberflächenmodifizierte Partikel, vorzugsweise silanisierte Partikel, sind
und/oder
zumindest ein Teil der Mikropartikel der Komponente (a2) Dentalglas-Partikel sind, wobei vorzugsweise zumindest ein Teil der Mikropartikel der Komponente (a2) organisch oberflächenmodifizierte Dentalglas-Partikel, vorzugsweise silanisierte Dentalglas-Partikel, sind.

5. Kompositmaterial nach einem der vorangehenden Ansprüche, umfassend:
(a) eine Gesamtmenge an Füllstoffen im Bereich von > 75 bis 95 Gew.-%, bezogen auf die Gesamtmasse des Kompositmaterials, wobei die Gesamtmenge an Füllstoffen eine Mischung von Füllstoffen ist bestehend aus
(a1) einer Gesamtmenge im Bereich von > 10 bis 30 Gew.-% an nicht agglomerierten, organisch oberflächenmodifizierten Nanopartikeln mit einer mittleren Partikelgröße kleiner 200 nm, wobei diese Nanopartikel keine Dentalglas-Partikel sind
und
(a2) einer Gesamtmenge im Bereich von 45 bis < 85 Gew.-% an Mikropartikeln mit einer mittleren Partikelgröße im Bereich von 0,4 µm bis 10 µm, wobei diese Mikropartikel Dentalglas-Partikel sind
sowie
(a3) gegebenenfalls weiteren Füllstoffen, wobei die weiteren Füllstoffe weder nicht agglomerierte, organisch oberflächenmodifizierte Partikel noch Dentalglas-Partikel sind,
wobei die Gewichtsprozentangaben für die Komponenten (a1) und (a2) auf die Gesamtmasse des Kompositmaterials bezogen sind.

6. Kompositmaterial nach einem der vorangehenden Ansprüche, **dadurch gekennzeichnet, dass** das Kompositmaterial kein Bis-GMA enthält, vorzugsweise keine Verbindung mit einem Bisphenol-A-Strukturelement enthält.

7. Kompositmaterial nach einem der vorangehenden Ansprüche, **dadurch gekennzeichnet, dass**
das Kompositmaterial im ausgehärteten Zustand eine Polymerisationsschrumpfung von weniger als 1,7 Vol.-%, vorzugsweise von weniger als 1,6 Vol.-% besitzt, gemessen nach der Bonded-Disc-Methode
und/oder
das Verhältnis der Vickers-Mikrohärte des Kompositmaterials zum Produkt aus der Mikroabrasion des Kompositmaterials (gemessen mit der ACTA-Methode) und der Polymerisationsschrumpfung des Kompositmaterials größer als 2, vorzugsweise größer als 2,5 [100/µm] ist.

8. Kompositmaterial nach einem der vorangehenden Ansprüche, wobei Komponente (a1) nicht agglomerierte Partikel, organisch oberflächenmodifizierte Nanopartikel ausgewählt aus der Gruppe bestehend aus Oxiden und Mischoxiden, vorzugsweise ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Wismut, Molybdän, Zinn, Zink, Ytterbium, Lanthan, Cer, Aluminium und deren Mischungen umfasst.

9. Kompositmaterial nach einem der vorangehenden Ansprüche, **dadurch gekennzeichnet, dass** das Strukturelement Q der Verbindungen der Struktur Q(YₓZₑ)_{b} der Komponente (b1) einen Tricyclo[5.2.1.0^{2,6}]decan-Rest, einen Tricyclo[5.2.1.0^{2,6}]dec-3-en-Rest, einen Tricyclo[3.3.1.1^{3,7}]decan-Rest oder einen Bicyclo[2.2.1]heptan-Rest bedeutet.

10. Kompositmaterial nach einem der vorangehenden Ansprüche, **dadurch gekennzeichnet, dass** ein, zwei oder mehr Verbindungen der Struktur Q(YₓZₑ)_{b} ein Tricyclo[5.2.1.0^{2,6}]-decan- oder Tricyclo[5.2.1.0^{2,6}]-decen-Strukturelement aufweisen und Z vorzugsweise ausgewählt ist aus der Gruppe bestehend aus -O-(C=O)-CH=CH₂ und -O-(C=O)-C(CH₃)=CH₂, besonders bevorzugt bedeutet Z die Gruppe -O-(C=O)-C(CH₃)=CH₂.

11. Kompositmaterial nach einem der vorangehenden Ansprüche, **dadurch gekennzeichnet, dass** Komponente (b1) umfasst oder besteht aus Bis(methacryloyloxymethyl)tricyclo[5.2.1.0^{2,6}]decan und/oder Bis(acryloyloxymethyl)tricyclo [5.2.1.0^{2,6}]decan.

12. Kompositmaterial nach einem der vorangehenden Ansprüche, **dadurch gekennzeichnet, dass** es lichthärtbar und/oder chemisch härtbar ist.

13. Kompositmaterial nach einem der vorangehenden Ansprüche, **dadurch gekennzeichnet, dass** Bestandteil (c) einen oder mehrere Lichthärtungsinitiatoren und/oder einen oder mehrere Initiatoren zur chemischen Aushärtung umfasst oder aus diesen Initiatoren besteht.

14. Erzeugnis, erhältlich durch Härten eines Kompositmaterials gemäß einem der Ansprüche 1 - 13.

15. Kompositmaterial gemäß einem der Ansprüche 1 - 13 zur Anwendung in einem therapeutischen dentalen Verfahren als Dentalmaterial, insbesondere zum Füllen, zum Unterfüllen, zum Befestigen von Zähnen und/oder zum Aufbauen von Stümpfen, oder zur Herstellung provisorischer Kronen und/oder Brücken.

## Claims

1. A composite material consisting of or comprising:
(a) a total quantity of fillers in the range from >75 to 95 wt.%, based on the total mass of the composite material, wherein the total quantity of fillers is a mixture of fillers comprising
(a1) a total quantity in the range from >12 to 30 wt.% of non-agglomerated, organically surface-modified nanoparticles with an average particle size of less than 200 nm
and
(a2) a total quantity in the range from 45 to < 83 wt.% of microparticles with an average particle size in the range from 0.4 µm to 10 µm
and
(a3) optionally further fillers,
wherein the weight percentages for components (a1) and (a2) are based on the total mass of the composite material,
(b) a total quantity of polymerisable monomers in the range from 3 to < 25 wt.%, based on the total mass of the composite material, wherein the total quantity of polymerisable monomers is a mixture of monomers comprising
(b1) one, two or more monomers selected from the group consisting of compounds (monomers) with the structure Q(YₓZₑ)_{b}, wherein the following applies:
- Q represents a saturated or olefinically unsaturated polyalicyclic structural element selected from the group consisting of bicyclic, tricyclic, tetracyclic, pentacyclic and hexacyclic hydrocarbon residues, wherein optionally one, two or more of the hydrogen atoms of said polyalicyclic structural element Q not substituted by substituents YₓZₑ are substituted by alkyl groups, alkoxy groups, halogen atoms or trifluoromethyl groups;
- b is a natural number selected from the group of natural numbers 1, 2, 3 and 4,
- each Z represents a structural element, which independently of any further structural elements Z is selected from the group consisting of
-O-(C=O)-CH=CH₂, -O-(C=O)-C(CH₃)=CH₂,
-(C=O)-CH=CH₂, -(C=O)-C(CH₃)=CH₂,
-CH=CH₂, -C(CH₃)=CH₂ and -O-CH=CH₂,
- each index e is a natural number, which independently of any further indices e is selected from the group of natural numbers 1, 2, 3 and 4,
- each index x independently of any further indices x represents 0 or 1,
- each Y in the structure Q(YₓZₑ)_{b} where x = 1 represents a structural element which links the polyalicyclic structural element Q with e structural elements Z, wherein each Y is selected independently of any further structural elements Y,
(b2) one, two or more further free-radically polymerisable monomers from the group consisting of acrylates and methacrylates, preferably from the group of the methacrylates,
wherein the further free-radically polymerisable monomer(s) is/are not compounds (monomers) of the structure Q(YₓZₑ)_{b} defined above and wherein the ratio of the mass of component (b1) to the mass of component (b2) is in the range from 1:20 to 4:1,
(c) one or more initiators and/or catalysts, and
(d) optionally one or more other additives.

2. The composite material according to claim 1, wherein component (a2) contains two or more microparticle fractions, wherein one or more first microparticle fractions in each case have an average particle size in the range from 1 µm to 10 µm, preferably 1 to 5 µm, and wherein one or more second microparticle fractions in each case have an average particle size in the range from > 0.4 µm to < 1 µm, preferably 0.5 µm to 0.8 µm.

3. The composite material according to claim 2, wherein
the ratio of the total mass of the first microparticle fractions to the total mass of the second microparticle fractions is in the range from 1:1 to 10:1, preferably in the range from 1.5:1 to 5:1,
and/or
the ratio of the average grain size of the or of a first microparticle fraction to the average grain size of the or of a second microparticle fraction of component (a2) is in the range from 1.5:1 to 10:1, preferably in the range from 2:1 to 5:1.

4. The composite material according to one of the preceding claims, wherein at least a part of the microparticles of component (a2) are organically surface-modified particles, preferably silanised particles,
and/or
at least a part of the microparticles of component (a2) are dental glass particles,
wherein preferably at least a part of the microparticles of component (a2) are organically surface-modified dental glass particles, preferably silanised dental glass particles.

5. The composite material according to one of the preceding claims, comprising:
(a) a total quantity of fillers in the range from >75 to 95 wt.%, based on the total mass of the composite material, wherein the total quantity of fillers is a mixture of fillers consisting of
(a1) a total quantity in the range from >10 to 30 wt.% of non-agglomerated, organically surface-modified nanoparticles with an average particle size of less than 200 nm, wherein these nanoparticles are not dental glass particles
and
(a2) a total quantity in the range from 45 to < 85 wt.% of microparticles with an average particle size in the range from 0.4 µm to 10 µm, wherein these microparticles are dental glass particles
and
(a3) optionally further fillers, wherein the further fillers are neither non-agglomerated, organically surface-modified particles nor dental glass particles,
wherein the mass percentages given for components (a1) and (a2) are based on the total mass of the composite material.

6. The composite material according to one of the preceding claims, **characterised in that** the composite material does not contain any bis-GMA and preferably contains no compound having a bisphenol A structural element.

7. The composite material according to one of the preceding claims, **characterised in that**
the composite material in the cured state has a polymerisation shrinkage of less than 1.7 vol.%, preferably less than 1.6 vol.%, measured according to the bonded disc method
and/or
the ratio of the Vickers microhardness of the composite material to the product of the microabrasion of the composite material (measured using the ACTA method) and the polymerisation shrinkage of the composite material is more than 2, preferably more than 2.5 [100/µm].

8. The composite material according to one of the preceding claims, wherein component (a1) comprises non-agglomerated particles, organically surface-modified nanoparticles selected from the group consisting of oxides and mixed oxides, preferably selected from the group consisting of oxides and mixed oxides of the elements silicon, titanium, yttrium, strontium, barium, zirconium, hafnium, niobium, tantalum, tungsten, bismuth, molybdenum, tin, zinc, ytterbium, lanthanum, cerium, aluminium and mixtures thereof.

9. The composite material according to one of the preceding claims, **characterised in that** the structural element Q of the compounds of structure Q(YₓZₑ)_{b} of component (b1) represents a tricyclo[5.2.1.0^{2,6}]decane residue, a tricyclo[5.2.1.0^{2,6}]dec-3-ene residue, a tricyclo[3.3.1.1^{3,7}]decane residue or a bicyclo[2.2.1]heptane residue.

10. The composite material according to one of the preceding claims, **characterised in that** one, two or more compounds of structure Q(YₓZₑ)_{b} have a tricyclo[5.2.1.0^{2,6}]decane or tricyclo[5.2.1.0^{2,6}]decene structural element and Z is preferably selected from the group consisting of -O-(C=O)-CH=CH₂ and -O-(C=O)-C(CH₃)=CH₂, with Z particularly preferably representing the group -O-(C=O)-C(CH₃)=CH₂.

11. The composite material according to one of the preceding claims, **characterised in that** component (b1) comprises or consists of bis(methacryloyloxymethyl)tricyclo[5.2.1.0^{2,6}]decane and/or bis(acryloyloxymethyl)tricyclo[5.2.1.0^{2,6}]decane.

12. The composite material according to one of the preceding claims, **characterised in that** it is photocurable and/or chemically curable.

13. The composite material according to one of the preceding claims, **characterised in that** component (c) comprises one or more photocuring initiators and/or one or more initiators for chemical curing or consists of these initiators.

14. A product obtainable by curing a composite material according to one of claims 1 to 13.

15. The composite material according to one of claims 1 to 13 for use in a therapeutic dental procedure as a dental material, in particular for filling, underfilling, securing teeth and/or building up stumps or producing temporary crowns and/or bridges.

## Revendications

1. Matériau composite constitué ou comprenant :
(a) une quantité totale de matières de charge dans la plage de > 75 à 95 % en poids, par rapport à la masse totale du matériau composite, dans lequel la quantité totale de matières de charge est un mélange de matières de charge, comprenant
(a1) une quantité totale dans la plage de > 12 à 30 % en poids de nanoparticules non agglomérées, modifiées en surface organiquement, avec une taille moyenne de particules inférieure à 200 nm
et
(a2) une quantité totale dans la plage de 45 à < 83 % en poids de microparticules avec une taille moyenne de particules dans la plage de 0,4 µm à 10 µm
et
(a3) optionnellement d'autres matières de charge,
dans lequel les indications de pourcentages en poids pour les composants (a1) et (a2) se rapportent à la masse totale du matériau composite,
(b) une quantité totale de monomères polymérisables dans la plage de 3 à < 25 % en poids, par rapport à la masse totale du matériau composite, dans lequel la quantité totale de monomères polymérisables est un mélange de monomères, comprenant
(b1) un, deux ou plus de deux monomères, lesquels sont choisis dans le groupe constitué des composés (monomères) de structure Q(YₓZₑ)_{b}, dans lequel :
- Q représente un élément structurel polyalicyclique saturé ou oléfiniquement insaturé choisi dans le groupe constitué des résidus hydrocarbonés bicycliques, tricycliques, tétracycliques, pentacycliques et hexacycliques, dans lequel éventuellement un, deux ou plus de deux atomes d'hydrogène, dudit élément structurel polyalicyclique Q, non substitués par des substituants YₓZₑ, sont substitués par des groupes alkyle, des groupes alcoxy, des atomes d'halogène ou des groupes trifluorométhyle,
- b est un entier naturel choisi dans le groupe des entiers naturels 1, 2, 3 et 4,
- chaque Z représente un élément structurel qui est choisi indépendamment des éventuels autres éléments structurels Z dans le groupe constitué de
- O-(C=O)-CH=CH₂, -O-(C=O)-C(CH₃)=CH₂,
- (C=O)-CH=CH₂, -(C=O)-C(CH₃)=CH₂,
- CH=CH₂, -C(CH₃)=CH₂ et -O-CH=CH₂,
- chaque indice e est un entier naturel qui est choisi indépendamment d'autres indices éventuels dans le groupe des entiers naturels 1, 2, 3 et 4,
- chaque indice x représente indépendamment d'autres indices x éventuels 0 ou 1,
- chaque Y représente dans la structure Q(YₓZₑ)_{b}, dans laquelle x = 1, un élément structurel, qui relie l'élément structurel polyalicyclique Q avec e éléments structurels Z, dans lequel chaque Y est choisi indépendamment d'éventuels autres éléments structurels Y,
(b2) un, deux ou plus de deux autres monomères polymérisables par voie radicalaire du groupe constitué des acrylates et des méthacrylates, de préférence du groupe des méthacrylates,
dans lequel le ou les autres monomères polymérisables par voie radicalaire ne sont pas des composés (monomères) de la structure préalablement définie Q(YₓZₑ)_{b}, et
dans lequel le rapport entre la masse du composant (b1) et la masse du composant (b2) est compris dans la plage de 1:20 à 4:1,
(c) un ou plusieurs initiateurs et/ou catalyseurs, et
(d) optionnellement un ou plusieurs autres additifs.

2. Matériau composite selon la revendication 1, dans lequel le composant (a2) contient deux fractions de microparticules ou plus, dans lequel une ou plusieurs premières fractions de microparticules possèdent respectivement une taille moyenne de particules dans la plage de 1 µm à 10 µm, de préférence de 1 µm à 5 µm, et dans lequel une ou plusieurs deuxièmes fractions de microparticules possèdent respectivement une taille moyenne de particules dans la plage de > 0,4 µm à < 1 µm, de préférence de 0,5 µm à 0,8 µm.

3. Matériau composite selon la revendication 2, dans lequel
le rapport entre la masse totale des premières fractions de microparticules et la masse totale des deuxièmes fractions de microparticules est compris dans la plage de 1 : 1 à 10 : 1, de préférence dans la plage de 1,5 : 1 à 5 : 1,
et/ou
le rapport entre la granulométrie moyenne de la ou d'une première fraction particules et la granulométrie moyenne de la ou d'une deuxième fraction de microparticules du composant (a2) est compris dans la plage de 1,5 : 1 à 10 : 1, de préférence dans la plage de 2 : 1 à 5 : 1.

4. Matériau composite selon l'une des revendications précédentes, dans lequel au moins une partie des microparticules du composant (a2) sont des particules modifiées en surface organiquement, de préférence des particules silanisées,
et/ou
au moins une partie des microparticules du composant (a2) sont des particules de verre dentaire, dans lequel de préférence au moins une partie des microparticules du composant (a2) sont des particules de verre dentaire modifiées en surface organiquement, de préférence des particules de verre dentaire silanisées.

5. Matériau composite selon l'une des revendications précédentes, comprenant :
(a) une quantité totale de matières de charge dans la plage de > 75 à 95 % en poids, par rapport à la masse totale du matériau composite, dans lequel la quantité totale de matières de charge est un mélange de matières de charge, constitué de
(a1) une quantité totale dans la plage de > 10 à 30 % en poids de nanoparticules non agglomérées, modifiées en surface organiquement, avec une taille moyenne de particules inférieure à 200 nm, dans lequel ces nanoparticules ne sont pas des particules de verre dentaire,
et
(a2) une quantité totale dans la plage de 45 à < 85 % en poids de microparticules avec une taille moyenne de particules dans la plage de 0,4 µm à 10 µm, dans lequel ces microparticules sont des particules de verre dentaire,
et
(a3) optionnellement d'autres matières de charge, dans lequel les autres matières de charge ne sont ni des particules non agglomérées, modifiées en surface organiquement, ni des particules de verre dentaire,
dans lequel les indications de pourcentages en poids pour les composants (a1) et (a2) se rapportent à la masse totale du matériau composite.

6. Matériau composite selon l'une des revendications précédentes, **caractérisé en ce que** le matériau composite ne comprend pas de Bis-GMA, de préférence pas de composé avec un élément structurel de bisphénol A.

7. Matériau composite selon l'une des revendications précédentes, **caractérisé en ce que**
le matériau composite possède à l'état durci un rétrécissement à la polymérisation inférieur à 1,7 % en volume, de préférence inférieur à 1,6 % en volume, mesuré d'après la méthode du disque lié,
et/ou
le rapport entre la microdureté de Vickers du matériau composite et le produit provenant de la microabrasion du matériau composite (mesurée par la méthode ACTA), et le rétrécissement à la polymérisation du matériau composite supérieur à 2, de préférence supérieur à 2,5 [100/µm].

8. Matériau composite selon l'une des revendications précédentes, dans lequel le composant (a1) comprend des particules non agglomérées, des nanoparticules modifiées en surface organiquement choisies dans le groupe constitué des oxydes et des oxydes mixtes, de préférence choisis dans le groupe constitué des oxydes et des oxydes mixtes des éléments silicium, titane, yttrium, strontium, baryum, zirconium, hafnium, niobium, tantale, tungstène, bismuth, molybdène, étain, zinc, ytterbium, lanthane, cérium, aluminium et leurs mélanges.

9. Matériau composite selon l'une des revendications précédentes, **caractérisé en ce que** l'élément structurel Q des composés de structure Q(YₓZₑ)_{b} composants (b1) représente un résidu tricyclo[5.2.1.0^{2,6}]-décane, un résidu tricyclo[5.2.1.0^{2,6}]-déc-3-ène, un résidu tricyclo[3.3.1.1^{3,7}]-décane, ou un résidu bicyclo[2.2.1]heptane.

10. Matériau composite selon l'une des revendications précédentes, **caractérisé en ce qu'**un, deux ou plus de deux composés de la structure Q(YₓZₑ)_{b} présentent un élément structurel tricyclo[5.2.1.0^{2,6}]-décane ou tricyclo[5.2.1.0^{2,6}]-décène et Z est de préférence choisi dans le groupe constitué de -O-(C=O)-CH=CH₂ et -O-(C=O)-C(CH₃)=CH₂, particulièrement préférentiellement Z représente le groupe -O-(C=O)-C(CH₃)=CH₂.

11. Matériau composite selon l'une des revendications précédentes, **caractérisé en ce que** le composant (b1) comprend ou se compose de bis(méthacryloyloxyméthyl)tricyclo[5.2.1.0^{2,6}]-décane et/ou bis(acryloyloxyméthyl)tricyclo[5.2.1.0^{2,6}]-décane.

12. Matériau composite selon l'une des revendications précédentes, **caractérisé en ce qu'**il est photodurcissable et/ou durcissable chimiquement.

13. Matériau composite selon l'une des revendications précédentes, **caractérisé en ce que** l'ingrédient (c) comprend un ou plusieurs initiateurs de photodurcissement et/ou un ou plusieurs initiateurs pour le durcissement chimique ou se compose de ces initiateurs.

14. Produit pouvant être obtenu par durcissement d'un matériau composite d'après l'une des revendications 1 à 13.

15. Matériau composite d'après l'une des revendications 1 à 13 pour l'utilisation dans un procédé thérapeutique dentaire comme matériau dentaire, en particulier pour le remplissage, pour le sous-remplissage, pour la fixation de dents et/ou pour la reconstruction des moignons, ou pour la fixation de couronnes provisoires et/ou de bridges.
